# EUROPEAN PATENT SPECIFICATION

(11) **EP 2 007 899 B1**
(45) Date of publication and mention of the grant of the patent: **07.06.2017**
(21) Application number: 07753609.2
(22) Date of filing: 20.03.2007
(51) Int. Cl.: C07K 16/30, C07K 16/22, A61K 39/00, A61K 47/68, A61K 51/10, G01N 33/574

(54) **ANTIBODIES DIRECTED TO ANGIOPOIETIN-LIKE PROTEIN 4 AND USES THEREOF**
GEGEN ANGIOPOIETIN-LIKE PROTEIN 4 GERICHTETE ANTIKÖRPER UND IHRE VERWENDUNG
ANTICORPS DIRIGÉS CONTRE LA PROTÉINE 4 DU TYPE ANGIOPOIÉTINE ET UTILISATIONS DE CEUX-CI

(30) Priority: 20.03.2006 US 784029 P
(43) Date of publication of application: 31.12.2008
(73) Proprietor: Celldex Therapeutics, Inc., Hampton, NJ 08827 (US); Amgen Fremont Inc., Thousand Oaks, CA 91320-1799 (US)
(72) Inventor: ZHONG, Haihong, Guilford, CT 06437 (US); ZHOU, Qing, Fremont, CA 94536 (US)
(74) Representative: Finnie, Isobel Lara
(86) International application number: PCT/US2007/006999
(87) International publication number: WO 2007/109307

(56) References cited:
- JAN LE S ET AL: "ANGPTL4 is a proangiogenic factor produced during ischemia and in conventional renal cell carcinoma" AMERICAN JOURNAL OF PATHOLOGY, AMERICAN SOCIETY FOR INVESTIGATIVE PATHOLOGY, US, vol. 162, no. 5, 1 January 2003 (2003-01-01), pages 1521-1528, XP002345553 ISSN: 0002-9440
- GREEN LARRY L: "Antibody engineering via genetic engineering of the mouse: XenoMouse strains are a vehicle for the facile generation of therapeutic human monoclonal antibodies" JOURNAL OF IMMUNOLOGICAL METHODS, vol. 231, no. 1-2, 10 December 1999 (1999-12-10), pages 11-23, XP004186071
- OIKE YUICHI ET AL: "Angiopoietin-related/angiopoietin-like proteins regulate angiogenesis" INTERNATIONAL JOURNAL OF HEMATOLOGY, ELSEVIER SCIENCE PUBLISHERS, NL, vol. 80, no. 1, 1 July 2004 (2004-07-01), pages 21-28, XP009075061 ISSN: 0925-5710
- DESAI U. ET AL.: 'Lipid-lowering Effects of Anti-angiopoietin-like 4 Antibody Recapitulate the Lipid Phenotype Found in Angiopoietin-like 4 Knockout Mice' PROC. NATL. ACAD. SCI. USA vol. 104, no. 28, 10 July 2007, pages 11766 - 11771, XP008130955
- HERMANN L.M. ET AL.: 'Angiopoietin-like-4 is a Potential Angiogenic Mediator in Arthritis' CLINICAL IMMUNOLOGY vol. 115, 2005, pages 93 - 101, XP004875154
- ZHONG: 'CR064, a fully human monoclonal antibody to angiopoietin related protein 4 (ARP4) inhibits ARP4-mediated angiogenesis and renal cell carcinoma survival in vitro' vol. 65, 01 May 2005, page 706, XP055181293

### SEQUENCE LISTING

<110> CuraGen Corporation Amgen Fremont Inc. Haihong Zhong Qing Zhou
<120> Antibodies directed to angiopoietin-like protein 4 and uses thereof
<130> 21402-663-061
<150> 60/784,029
   <151> 2006-03-20
<160> 106
<170> CuraSeqList version 0.1
<210> 1
<400> 1
   000
<210> 2
   <211> 112
   <212> PRT
   <213> Homo sapiens
<400> 2
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu Gln Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Leu Thr Phe Gly Gly Gly Thr Lys Val Glu Ile Lys Arg
               100                 105                 110
<210> 3
   <211> 112
   <212> PRT
   <213> Homo sapiens
<400> 3
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu Gln Ser
   20 25 30
   Asn Gly Tyr Asn Tyr Leu Asp Trp Tyr Leu Gln Lys Pro Gly Gln Ser
   35 40 45
   Pro Gln Leu Leu Ile Tyr Leu Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Ile Gly Val Tyr Tyr Cys Met Gln Ala
   85 90 95
   Leu Gln Thr Leu Thr Phe Gly Gly Gly Thr Lys Val Glu Ile Lys Arg
   100 105 110
<210> 4
   <211> 112
   <212> PRT
   <213> Homo sapiens
<400> 4
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu Gln Ser
   20 25 30
   Asn Gly Tyr Asn Tyr Leu Asp Trp Tyr Leu Gln Lys Pro Gly Gln Ser
   35 40 45
   Pro Gln Leu Leu Ile Tyr Leu Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Ala
   85 90 95
   Leu Gln Thr Leu Thr Phe Gly Gly Gly Thr Lys Val Glu Ile Lys Arg
   100 105 110
<210> 5
   <211> 112
   <212> PRT
   <213> Homo sapiens
<400> 5
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu His Arg
   20 25 30
   Asn Gly Tyr Asn Tyr Leu Asp Trp Tyr Leu Gln Lys Pro Gly Gln Ser
   35 40 45
   Pro Gln Leu Leu Ile Tyr Leu Gly Ser His Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Ala
   85 90 95
   Leu Gln Thr Leu Thr Phe Gly Gly Gly Thr Lys Val Glu Ile Lys Arg
   100 105 110
<210> 6
   <211> 112
   <212> PRT
   <213> Homo sapiens
<400> 6
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu Gln Ser
   20 25 30
   Asn Gly Tyr Asn Tyr Leu Asp Trp Tyr Leu Gln Lys Pro Gly Gln Ser
   35 40 45
   Pro Gln Leu Leu Ile Tyr Leu Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Ala
   85 90  95
   Leu Gln Thr Leu Thr Phe Gly Gly Gly Thr Lys Val Glu Ile Lys Arg
   100 105 110
<210> 7
   <211> 112
   <212> PRT
   <213> Homo sapiens
<400> 7
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu Gln Ser
   20 25 30
   Asn Gly Tyr Asn Tyr Leu Asp Trp Tyr Leu Gln Lys Pro Gly Gln Ser
   35 40 45
   Pro Gln Leu Leu Ile Tyr Leu Gly Ser Asn Arg Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Ala
   85 90 95
   Leu Gln Thr Leu Thr Phe Gly Gly Gly Thr Lys Val Glu Ile Lys Arg
   100 105 110
<210> 8
<400> 8
   000
<210> 9
   <211> 108
   <212> PRT
   <213> Homo sapiens
<400> 9
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Ser Asn Tyr
   20 25 30
   Leu Ala Trp Tyr Gln Gln Lys Pro Gly Lys Val Pro Lys Leu Leu Ile
   35 40 45
   Tyr Ala Ala Ser Thr Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Glu Phe Asn Ser Ala Pro Leu
   85 90 95
   Thr Phe Gly Gly Gly Thr Lys Val Glu Ile Lys Arg
   100 105
<210> 10
   <211> 108
   <212> PRT
   <213> Homo sapiens
<400> 10
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Ile Ser Asn Tyr
   20 25 30
   Leu Ala Trp Tyr Gln Gln Lys Pro Gly Lys Val Pro Lys Leu Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Val Ala Thr Tyr Tyr Cys Gln Glu Phe Asn Ser Ala Pro Leu
   85 90 95
   Thr Phe Gly Gly Gly Thr Lys Val Glu Ile Lys Arg
   100 105
<210> 11
<400> 11
   000
<210> 12
   <211> 113
   <212> PRT
   <213> Homo sapiens
<400> 12
   Asp Ile Val Met Thr Gln Thr Pro Leu Ser Leu Ala Val Thr Pro Gly
   1 5 10 15
   Gln Pro Ala Ser Ile Ser Cys Lys Ser Ser Gln Ser Leu Leu His Ser
   20 25 30
   Asp Arg Lys Thr Tyr Leu Tyr Trp Tyr Leu Gln Lys Pro Gly Gln Pro
   35 40 45
   Pro Gln Leu Leu Ile Tyr Glu Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Met Gln Ser
   85 90 95
   Ile Gln Leu Pro Trp Thr Phe Gly Gln Gly Thr Lys Val Glu Ile Lys
   100 105 110
   Arg
<210> 13
<400> 13
   000
<210> 14
   <211> 108
   <212> PRT
   <213> Homo sapiens
<400> 14
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Asn Asn Tyr
               20                  25                  30
   Leu Ala Trp Tyr Gln Gln Lys Pro Gly Lys Val Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Ser Ile Ser Ser Leu Gln Pro
   65                  70                  75                  80
   Glu Asp Val Ala Thr Tyr Tyr Cys Gln Lys Tyr Asn Ser Ala Pro Arg
                   85                  90                  95
   Thr Phe Gly Gln Gly Thr Lys Val Glu Ile Lys Arg
               100                 105
<210> 15
<400> 15
   000
<210> 16
   <211> 108
   <212> PRT
   <213> Homo sapiens
<400> 16
   Glu Ile Val Leu Thr Gln Ser Pro Asp Phe Gln Ser Val Thr Pro Lys
   1               5                   10                  15
   Glu Lys Val Thr Ile Thr Cys Arg Ala Ser Gln Ser Ile Gly Ser Ser
               20                  25                  30
   Leu His Trp Tyr Gln Gln Lys Pro Asp Gln Ser Pro Lys Leu Leu Ile
           35                  40                  45
   Lys His Ala Ser Gln Ser Phe Ser Gly Val Pro Ser Arg Phe Ser Gly
       50                  55                  60
   Ser Gly Ser Gly Thr Asp Phe Thr Leu Thr Ile Asn Ser Leu Glu Ala
   65                  70                  75                  80
   Glu Asp Ala Ala Thr Tyr Tyr Cys His Gln Ser Ser Ser Leu Pro His
                   85                  90                  95
   Thr Phe Gly Gly Gly Thr Lys Val Glu Ile Lys Arg
               100                 105
<210> 17
<400> 17
   000
<210> 18
   <211> 112
   <212> PRT
   <213> Homo sapiens
<400> 18
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu Gln Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Leu Thr Phe Gly Gln Gly Thr Arg Leu Glu Ile Lys Arg
               100                 105                 110
<210> 19
   <211> 112
   <212> PRT
   <213> Homo sapiens
<400> 19
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu Gln Thr
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Leu Tyr Tyr Cys Met Gln Ala
                   85                  90                  95
   Leu Gln Thr Leu Thr Phe Gly Gln Gly Thr Arg Leu Glu Ile Lys Arg
               100                 105                 110
<210> 20
<400> 20
   000
<210> 21
   <211> 108
   <212> PRT
   <213> Homo sapiens
<400> 21
   Glu Ile Val Leu Thr Gln Ser Pro Asp Phe Gln Ser Val Thr Pro Lys
   1               5                   10                  15
   Glu Lys Val Thr Ile Thr Cys Arg Ala Ser Gln Ser Ile Gly Ser Ser
               20                  25                  30
   Leu His Trp Tyr Gln Gln Lys Pro Asp Gln Ser Pro Lys Leu Leu Ile
           35                  40                  45
   Lys Tyr Ala Ser Gln Ser Phe Ser Gly Val Pro Ser Arg Phe Ser Gly
       50                  55                  60
   Ser Gly Ser Gly Thr Asp Phe Thr Leu Thr Ile Asn Ser Leu Glu Ala
   65                  70                  75                  80
   Glu Asp Ala Ala Thr Tyr Tyr Cys His Gln Ser Ser Asn Leu Pro His
                   85                  90                  95
   Thr Phe Gly Pro Gly Thr Lys Val Asp Ile Lys Arg
               100                 105
<210> 22
   <211> 108
   <212> PRT
   <213> Homo sapiens
<400> 22
   Glu Ile Val Leu Thr Gln Ser Pro Asp Phe Gln Ser Val Thr Pro Lys
   1               5                   10                  15
   Glu Lys Val Thr Ile Thr Cys Arg Ala Ser Gln Ser Ile Gly Ser Ser
               20                  25                  30
   Leu His Trp Tyr Gln Leu Lys Pro Asp Gln Ser Pro Lys Leu Leu Ile
           35                  40                  45
   Lys Tyr Val Ser Gln Ser Phe Ser Gly Val Pro Ser Arg Phe Ser Gly
       50                  55                  60
   Ser Gly Ser Gly Thr Asp Phe Thr Leu Thr Ile Ser Ser Leu Glu Ala
   65                  70                  75                  80
   Glu Asp Ala Ala Thr Tyr Cys Cys His Gln Ser Ser Ser Leu Pro His
                   85                  90                  95
   Thr Phe Gly Pro Gly Thr Lys Val Asp Ile Asn Arg
               100                 105
<210> 23
<400> 23
   000
<210> 24
   <211> 124
   <212> PRT
   <213> Homo sapiens
<400> 24
   Glu Val Gln Leu Val Glu Ser Gly Gly Gly Leu Ile Gln Pro Gly Gly
   1               5                   10                  15
   Ser Leu Arg Leu Ser Cys Ala Ala Ser Gly Phe Thr Val Ser Ser Asn
               20                  25                  30
   Tyr Met Ser Trp Val Arg Gln Ala Pro Gly Lys Gly Leu Glu Trp Val
           35                  40                  45
   Ser Ile Ile Tyr Ser Asp Gly Asn Thr Tyr Tyr Ala Asp Ser Val Lys
       50                  55                  60
   Gly Arg Phe Thr Ile Ser Arg Asp Asn Ser Lys Asn Thr Leu Phe Leu
   65                  70                  75                  80
   Gln Met Asp Ser Leu Arg Ala Glu Asp Thr Ala Val Tyr Tyr Cys Ala
                   85                  90                  95
   Gly Arg Gly Tyr Asn Tyr Gly Tyr Asp Tyr Tyr Tyr Gly Leu Asp Val
               100                 105                 110
   Trp Gly Gln Gly Thr Thr Val Thr Val Ser Ser Ala
           115                 120
<210> 25
   <211> 124
   <212> PRT
   <213> Homo sapiens
<400> 25
   Glu Val Gln Leu Val Glu Ser Gly Gly Gly Leu Ile Gln Pro Gly Gly
   1 5 10 15
   Ser Leu Arg Leu Ser Cys Ala Ala Ser Gly Phe Thr Val Ser Ser Asn
   20 25 30
   Tyr Met Ser Trp Val Arg Gln Ala Pro Gly Arg Gly Leu Glu Trp Val
   35 40 45
   Ser Val Ile Tyr Ser Asp Gly Asn Thr Tyr Tyr Ala Asp Ser Val Lys
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Gly Arg Gly Tyr Ser Tyr Gly Tyr Asp Tyr Tyr Tyr Gly Leu Asp Val
   100 105 110
   Trp Gly Gln Gly Thr Thr Val Thr Val Ser Ser Ala
   115 120
<210> 26
   <211> 124
   <212> PRT
   <213> Homo sapiens
<400> 26
   Glu Val Gln Leu Val Glu Ser Gly Gly Gly Leu Ile Gln Pro Gly Gly
   1 5 10 15
   Ser Leu Arg Leu Ser Cys Ala Ala Ser Gly Phe Thr Val Ser Ser Thr
   20 25 30
   Tyr Met Thr Trp Val Arg Gln Ala Pro Gly Lys Gly Leu Glu Trp Val
   35 40 45
   Ser Val Ile Tyr Ser Asp Gly Asn Thr Tyr Tyr Ala Asp Ser Val Lys
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Ser Ala Val Tyr Tyr Cys Ala
   85 90 95
   Gly Arg Gly Tyr Asn Asn Gly Asn Asp Tyr Tyr Tyr Gly Leu Asp Val
   100 105 110
   Trp Gly Gln Gly Thr Thr Val Thr Val Ser Ser Ala
   115 120
<210> 27
   <211> 124
   <212> PRT
   <213> Homo sapiens
<400> 27
   Glu Val Gln Leu Val Glu Ser Gly Gly Gly Leu Ile Gln Pro Gly Gly
    1               5                  10                  15
   Ser Leu Arg Leu Ser Cys Ala Ala Ser Gly Phe Thr Val Ser Ser Thr
               20                  25                  30
   Tyr Met Ser Trp Val Arg Gln Ala Pro Gly Lys Gly Leu Glu Trp Val
           35                  40                  45
   Ser Val Ile Tyr Ser Asp Gly Asn Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Ser Ala Val Tyr Tyr Cys Ala
                   85                  90                  95
   Gly Arg Gly Tyr Ser Tyr Gly Asn Asp Tyr Tyr Tyr Gly Leu Asp Val
               100                 105                 110
   Trp Gly Gln Gly Thr Thr Val Thr Val Ser Ser Ala
           115                 120
<210> 28
<400> 28
   000
<210> 29
   <211> 128
   <212> PRT
   <213> Homo sapiens
<400> 29
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Val Trp Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Asn
   65                  70                  75                  80
   Leu Gln Met Asn Ser Leu Arg Ala Glu Asp Thr Ala Val Tyr Tyr Cys
                   85                  90                  95
   Ala Arg Asp Gly Ile Thr Leu Val Arg Gly Val Ile Tyr Tyr Tyr Tyr
               100                 105                 110
   Gly Met Asp Val Trp Gly Gln Gly Thr Thr Val Thr Val Ser Ser Ala
           115                 120                 125
<210> 30
   <211> 128
   <212> PRT
   <213> Homo sapiens
<400> 30
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Gly Ile His Trp Ile Arg Gln Ala Pro Gly Lys Gly Leu Glu Trp Val
           35                  40                  45
   Ala Val Ile Trp Tyr Asp Gly Ser His Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Gly Ile Thr Met Val Arg Gly Val Leu Tyr Tyr Phe Tyr
               100                 105                 110
   Gly Met Asp Val Trp Gly Gln Gly Thr Thr Val Thr Val Ser Ser Ala
           115                 120                 125
<210> 31
<400> 31
   000
<210> 32
   <211> 123
   <212> PRT
   <213> Homo sapiens
<400> 32
<210> 33
<400> 33
   000
<210> 34
   <211> 122
   <212> PRT
   <213> Homo sapiens
<400> 34
   Glu Val Gln Leu Val Glu Ser Gly Gly Gly Leu Ile Gln Pro Gly Gly
   1 5 10 15
   Ser Leu Arg Leu Ser Cys Ala Ala Ser Gly Phe Thr Val Ser Ser Asn
   20 25 30
   Tyr Met Ser Trp Val Arg Gln Ala Pro Gly Lys Gly Leu Glu Trp Val
   35 40 45
   Ser Val Ile Tyr Ser Gly Gly Ser Thr Tyr Tyr Ala Asp Ser Val Lys
   50 55 60
   Gly Arg Phe Thr Ile Ser Arg Asp Ser Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Gly Tyr Ser Gly Tyr Asp Tyr Tyr Tyr Tyr Gly Leu Asp Val Trp Gly
   100 105 110
   Gln Gly Thr Thr Val Thr Val Ser Ser Ala
   115 120
<210> 35
<400> 35
   000
<210> 36
   <211> 120
   <212> PRT
   <213> Homo sapiens
<400> 36
   Glu Val Gln Leu Val Glu Ser Gly Gly Asn Leu Ile Gln Pro Gly Gly
    1               5                  10                  15
   Ser Leu Arg Leu Ser Cys Ala Ala Ser Gly Phe Thr Val Ser Ser Asn
               20                  25                  30
   Tyr Met Thr Trp Val Arg Gln Ala Pro Gly Lys Gly Leu Glu Trp Val
           35                  40                  45
   Ser Val Ile Tyr Ser Asp Asp Asn Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Thr Ser Leu Arg Ala Glu Asp Thr Ala Val Tyr Tyr Cys Ala
                   85                  90                  95
   Ser Gly Ile Asn Tyr Tyr Tyr Tyr Gly Leu Asp Val Trp Gly Gln Gly
               100                 105                 110
   Thr Thr Val Thr Val Ser Ser Ala
           115                 120
<210> 37
<400> 37
   000
<210> 38
   <211> 121
   <212> PRT
   <213> Homo sapiens
<400> 38
   Gln Val Gln Leu Gln Glu Ser Gly Pro Gly Leu Val Lys Pro Ser Glu
    1               5                  10                  15
   Thr Leu Ser Leu Thr Cys Ile Val Ser Gly Asp Ser Ile Ser Gly Tyr
               20                  25                  30
   Tyr Trp Ser Trp Ile Arg Gln Pro Ala Gly Lys Gly Leu Glu Trp Ile
           35                  40                  45
   Gly Arg Ile Tyr Thr Ser Gly Asn Thr Ile Tyr Asn Pro Ser Leu Lys
       50                  55                  60
   Ser Arg Val Thr Met Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Arg Glu Arg Pro Asp Tyr Gly Gly Asn Phe Phe Asp Tyr Trp Gly Gln
               100                 105                 110
   Gly Thr Leu Val Thr Val Ser Ser Ala
           115                 120
<210> 39
<400> 39
   000
<210> 40
   <211> 121
   <212> PRT
   <213> Homo sapiens
<400> 40
   Glu Val Gln Leu Val Glu Ser Gly Gly Gly Leu Ile Gln Pro Gly Gly
   1               5                   10                  15
   Ser Leu Arg Leu Ser Cys Ala Ala Ser Gly Phe Thr Val Ser Ser Asn
               20                  25                  30
   Tyr Met Ser Trp Val Arg Gln Ala Pro Gly Lys Gly Leu Glu Trp Val
           35                  40                  45
   Ser Val Ile Tyr Ser Gly Gly His Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Gly Thr Gly Thr Thr Asp Tyr Tyr Tyr Gly Met Asp Val Trp Gly Gln
               100                 105                 110
   Gly Thr Thr Val Thr Val Ser Ser Ala
           115                 120
<210> 41
   <211> 121
   <212> PRT
   <213> Homo sapiens
<400> 41
   Glu Val Gln Leu Val Glu Ser Gly Gly Gly Leu Ile Gln Pro Gly Gly
   1               5                   10                  15
   Ser Leu Arg Leu Ser Cys Ala Ala Ser Gly Phe Thr Val Ser Thr Asn
               20                  25                  30
   Tyr Met Ser Trp Val Arg Gln Ala Pro Gly Lys Gly Leu Asp Trp Val
           35                  40                  45
   Ser Ile Ile Tyr Ser Asp Asp Asn Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Ser Ala Val Tyr Tyr Cys Ala
                   85                  90                  95
   Gly Thr Gly Thr Thr Asp Tyr Tyr Tyr Gly Leu Asp Val Trp Gly Leu
               100                 105                 110
   Gly Thr Thr Val Thr Val Ser Ser Ala
           115                 120
<210> 42
   <211> 121
   <212> PRT
   <213> Homo sapiens
<400> 42
   Glu Val Gln Leu Val Glu Ser Gly Gly Gly Leu Ile Gln Pro Gly Gly
   1               5                   10                  15
   Ser Leu Arg Leu Ser Cys Ala Ala Ser Gly Phe Thr Val Ser Ser Asn
               20                  25                  30
   Tyr Met Ser Trp Val Arg Gln Ala Pro Gly Lys Gly Leu Glu Trp Val
           35                  40                  45
   Ser Val Ile Tyr Ser Gly Asp Asn Thr Tyr Tyr Ala Asp Ser Val Lys
       50                  55                  60
   Gly Arg Phe Thr Ile Ser Arg Asp Asn Ser Lys Asn Thr Leu Phe Leu
   65                  70                  75                  80
   Gln Met Asn Ser Leu Arg Ala Glu Asp Thr Ala Val Tyr Tyr Cys Ala
                   85                  90                  95
   Gly Thr Gly Thr Thr Asn Tyr Tyr Tyr Gly Leu Asp Val Trp Gly Gln
               100                 105                 110
   Gly Thr Thr Val Thr Val Ser Ser Ala
           115                 120
<210> 43
   <211> 121
   <212> PRT
   <213> Homo sapiens
<400> 43
   Glu Val Gln Leu Val Glu Ser Gly Gly Val Leu Ile Gln Pro Gly Gly
   1               5                   10                  15
   Ser Leu Arg Leu Ser Cys Ala Ala Ser Gly Phe Thr Val Ser Ser Asn
               20                  25                  30
   Tyr Met Ser Trp Val Arg Gln Ala Pro Gly Lys Gly Leu Glu Trp Val
           35                  40                  45
   Ser Ile Ile Tyr Ser Asp Gly Asn Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Gly Thr Gly Thr Thr Asp Tyr Tyr Tyr Gly Met Asp Val Trp Gly Gln
               100                 105                 110
   Gly Thr Thr Val Thr Val Ser Ser Ala
                       115                 120
<210> 44
   <211> 121
   <212> PRT
   <213> Homo sapiens
<400> 44
   Glu Val Gln Leu Val Glu Ser Gly Gly Val Leu Ile Gln Pro Gly Gly
   1               5                   10                  15
   Ser Leu Arg Leu Ser Cys Ala Ala Ser Gly Phe Thr Val Ser Ser Asn
               20                  25                  30
   Tyr Met Ser Trp Val Arg Gln Ala Pro Gly Lys Gly Leu Glu Trp Val
           35                  40                  45
   Ser Ile Ile Tyr Ser Asp Gly Asn Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Gly Thr Gly Thr Thr Asp Tyr Tyr Tyr Gly Met Asp Val Trp Gly Gln
               100                 105                 110
   Gly Thr Thr Val Thr Val Ser Ser Ala
           115                 120
<210> 45
<400> 45
   000
<210> 46
   <211> 117
   <212> PRT
   <213> Homo sapiens
<400> 46
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Ser Tyr
               20                  25                  30
   Ser Met Asn Trp Val Arg Gln Ala Pro Gly Lys Gly Leu Glu Trp Val
           35                  40                  45
   Ser Ser Ile Ser Ser Ser Ser Ser Tyr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Gly Ala Ile Phe Gly Val Ala Asn Trp Tyr Phe Asp Leu
   100 105 110
   Trp Gly Arg Gly Thr
   115
<210> 47
<400> 47
   000
<210> 48
   <211> 117
   <212> PRT
   <213> Homo sapiens
<400> 48
   Glu Val Gln Leu Val Glu Ser Gly Gly Gly Leu Val Lys Pro Gly Gly
   1 5 10 15
   Ser Leu Arg Leu Ser Cys Ala Ala Ser Gly Phe Thr Phe Ser Ser Tyr
   20 25 30
   Ser Met Asn Trp Val Arg Gln Ala Pro Gly Lys Gly Leu Glu Trp Val
   35 40 45
   Ser Ser Ile Ser Ser Ser Ser Ser Tyr Ile Tyr Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Asp Gly Ala Ile Phe Gly Val Val Asn Trp Tyr Phe Asp Leu
   100 105 110
   Trp Gly Arg Gly Thr
   115
<210> 49
   <211> 117
   <212> PRT
   <213> Homo sapiens
<400> 49
   Glu Val Gln Leu Val Glu Ser Gly Gly Gly Leu Val Lys Pro Gly Gly
   1 5 10 15
   Ser Leu Arg Leu Ser Cys Ala Ala Ser Gly Phe Thr Phe Ser Ser Tyr
   20 25 30
   Ser Met Asn Trp Val Arg Gln Ala Pro Gly Lys Gly Leu Glu Trp Val
   35 40 45
   Ser Ser Ile Ser Ser Ser Ser Thr Tyr Ile Tyr Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Asp Gly Ala Ile Phe Gly Val Val Asn Trp Tyr Phe Asp Leu
   100 105 110
   Trp Gly Arg Gly Thr
   115
<210> 50
<400> 50
   000
<210> 51
   <211> 126
   <212> PRT
   <213> Homo sapiens
<400> 51
   Gln Val Gln Leu Val Gln Ser Gly Ala Glu Val Lys Lys Pro Gly Ala
   1 5 10 15
   Ser Val Lys Asp Ser Cys Lys Ala Ser Gly Phe Thr Phe Thr Ser Tyr
   20 25 30
   Gly Ile Ser Trp Val Arg Gln Ala Pro Gly Gln Gly Leu Glu Trp Met
   35 40 45
   Gly Trp Ile Ser Ala Tyr Asn Gly Asn Lys Asn Tyr Ala Gln Lys Leu
   50 55 60
   Gln Gly Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Asp Asp Cys Ser Arg Ala Ser Cys Ser Tyr Tyr Tyr Gly Met
   100 105 110
   Asp Val Trp Gly Gln Gly Thr Ala Val Thr Val Ser Ser Ala
   115 120 125
<210> .52
<400> 52
   000
<210> 53
   <211> 122
   <212> PRT
   <213> Homo sapiens
<400> 53
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ser Met Asn Trp Val Arg Gln Ala Pro Gly Lys Gly Leu Glu Trp Val
           35                  40                  45
   Ser Tyr Ile Ser Ser Ser Ser Ser His Ile Tyr Tyr Ala Asp Ser Val
       50                  55                  60
   Lys Gly Arg Phe Thr Ile Ser Arg Asp Asn Ala Lys Lys Ser Leu Tyr
   65                  70                  75                  80
   Leu Gln Met Asn Ser Leu Arg Asp Glu Asp Thr Ala Val Tyr Tyr Cys
                   85                  90                  95
   Ala Arg Asp Gln Asp Asp Tyr Tyr Tyr Tyr Gly Leu Asp Val Trp Gly
               100                 105                 110
   His Gly Thr Thr Val Thr Val Ser Ser Ala
           115                 120
<210> 54
   <211> 378
   <212> DNA
   <213> Homo sapiens
<400> 54
   caggttcagc tggtgcagtc tggagctgag gtgaagaagc ctggggcctc agtgaaggac  60
   tcctgcaagg cttctggttt cacctttacc agctatggta tcagctgggt gcgacaggcc 120
   cctggacaag ggcttgagtg gatgggatgg atcagcgctt acaatggtaa caaaaactat 180
   gcacagaagc tccagggcag agtcaccatg acgacagaca catccacgag cacagcctac 240
   atggagctga ggagcctgag atctgacgac acggccgtat attactgtgc gagagatgat 300
   tgtagtagag ccagctgctc ttactactac ggtatggacg tctggggcca agggaccgcg 360
   gtcaccgtct cctcagcc                                               378
<210> 55
   <211> 339
   <212> DNA
   <213> Homo sapiens
<400> 55
   gatattgtga tgacccagac tccactctct ctggccgtca cccctggaca gccggcctcc  60
   atctcctgca agtctagtca gagcctcctg catagtgata gaaagaccta tttgtattgg 120
   tacctgcaga agccaggcca gcctccacag ctcctgatct atgaagtttc caaccggttc 180
   tctggagtgc cagataggtt cagtggcagc gggtcaggga cagatttcac actgaaaatc 240
   agccgggtgg aggctgagga tgttggggtt tattactgca tgcaaagtat acagcttccg 300
   tggacgttcg gccaagggac caaggtggaa atcaaacga                        339
<210> 56
   <211> 366
   <212> DNA
   <213> Homo sapiens
<400> 56
   gaggtgcagc tggtggagtc tgggggaggc ttggtacagc ctggggggtc cctgagactc  60
   tcctgtgcag cctctggatt caccttcagt agctatagca tgaactgggt ccgccaggct 120
   ccagggaagg ggctggagtg ggtttcatac attagcagta gtagtagtca catatactac 180
   gcagactctg tgaagggccg attcaccatc tccagagaca atgccaagaa atcactgtat 240
   ctgcaaatga acagcctgag agacgaggac acggctgtgt attactgtgc gagagatcag 300
   gacgactact actactacgg tttggacgtc tggggccatg ggaccacggt caccgtctcc 360
   tcagcc                                                            366
<210> 57
   <211> 336
   <212> DNA
   <213> Homo sapiens
<400> 57
   gatattgtga tgactcagtc tccactctcc ctgcccgtca cccctggaga gccggcctcc  60
   atctcctgca ggtctagtca gagcctcctg catcgtaatg gatacaacta tttggattgg 120
   tacctgcaga agccagggca gtctccacag ctcctgatct atctgggttc tcatcgggcc 180
   tccggggtcc ctgacaggtt cagtggcagt ggatcaggca cagattttac actgaaaatc 240
   agcagagtgg aggctgagga tgttggggtt tattattgca tgcaagctct acaaactctc 300
   actttcggcg gagggaccaa ggtggagatc aaacga                           336
<210> 58
   <211> 351
   <212> DNA
   <213> Homo sapiens
<400> 58
   gaggtgcagc tggtggagtc tgggggaggc ctggtcaagc ctggggggtc cctgagactc  60
   tcctgtgcag cctctggatt caccttcagt agctatagca tgaactgggt ccgccaggct 120
   ccagggaagg ggctggagtg ggtctcatcc attagtagta gtagtagtta catatactac 180
   gcagactcag tgaagggccg attcaccatc tccagagaca acgccaagaa ctcactgtat 240
   ctgcaaatga acagcctgag agccgaggac acggctgtgt attactgtgc gagagatggg 300
   gcgatttttg gagtggctaa ctggtacttc gatctctggg gccgtggcac c          351
<210> 59
   <211> 366
   <212> DNA
   <213> Homo sapiens
<400> 59
   gaggtgcagc tggtggagtc tggaggaggc ttgatccagc ctggggggtc cctgagactc 60
   tcctgtgcag cctctgggtt caccgtcagt agcaactaca tgagctgggt ccgccaggct 120
   ccagggaagg ggctggagtg ggtctcagtt atttatagcg gtggtagcac gtactacgca 180
   gactccgtga agggccgatt caccatctcc agagacagtt ccaagaacac gctgtatctt 240
   caaatgaaca gcctgagagc cgaggacacg gccgtgtatt actgtgcggg ttatagtggc 300
   tacgattact actactacgg tttggacgtc tggggccaag ggaccacggt caccgtctcc 360
   tcagcc 366
<210> 60
   <211> 336
   <212> DNA
   <213> Homo sapiens
<400> 60
   gatattgtga tgactcagtc tccactctcc ctgcccgtca cccctggaga gccggcctcc 60
   atctcctgca ggtctagtca gagcctcctg caaagtaatg gatacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct gcaaacgctc 300
   actttcggcg gagggaccaa ggtggagatc aaacga 336
<210> 61
   <211> 363
   <212> DNA
   <213> Homo sapiens
<400> 61
   gaggtgcagc tggtggagtc tggaggaggc ttgatccagc ctggggggtc cctgagactc 60
   tcctgtgcag cctctggatt caccgtcagt accaactaca tgagctgggt ccgccaggct 120
   ccagggaagg gactggactg ggtctcaatt atttatagcg atgataacac atactacgca 180
   gactccgtga agggccgatt caccatctcc agagacaatt ccaagaacac gctgtatctt 240
   caaatgaaca gcctgagagc cgaggactcg gccgtgtatt actgtgcggg aactggaact 300
   acggactact actacggttt ggacgtctgg ggcctaggga ccacggtcac cgtctcctca 360
   gcc 363
<210> 62
   <211> 360
   <212> DNA
   <213> Homo sapiens
<400> 62
   gaggtgcagc tggtggagtc tggaggaaac ttgatccagc ctggggggtc cctgagactc 60
   tcctgtgcag cctctggatt caccgtcagt agtaactaca tgacctgggt ccgccaggct 120
   ccagggaagg ggctggagtg ggtctcagtt atttatagcg atgataacac atactacgca 180
   gactccgtga agggccgatt caccatctcc agagacaatt ccaagaacac gctgtatctt 240
   caaatgacca gcctgagagc cgaggacacg gccgtgtatt actgtgcgag tgggatcaac 300
   tactactact acggtttgga cgtctggggc caagggacca cggtcaccgt ctcctcagcc 360
<210> 63
   <211> 336
   <212> DNA
   <213> Homo sapiens
<400> 63
   gatattgtga tgactcagtc tccactctcc ctgcccgtca cccctggaga gccggcctcc 60
   atctcctgca ggtctagtca gagcctcctg caaagtaatg gatacaatta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactctc 300
   actttcggcg gagggaccaa ggtggagatc aaacga 336
<210> 64
   <211> 372
   <212> DNA
   <213> Homo sapiens
<400> 64
   gaggtgcagc tggtggagtc tggaggaggc ttgatccagc ctggggggtc cctgagactc 60
   tcctgtgcag cctctgggtt caccgtcagt agtacctaca tgacctgggt ccgccaggct 120
   ccagggaagg ggctggagtg ggtctcagtt atttatagtg atggtaacac atactacgca 180
   gactccgtta agggccgatt caccatctcc agagacaatt ccaagaacac gctgtatctt 240
   caaatgaaca gcctgagagc cgaggactcg gccgtgtatt actgtgcggg acgtggatac 300
   aacaatggga acgactacta ctacggtttg gacgtctggg gccaagggac cacggtcacc 360
   gtctcctcag cc 372
<210> 65
   <211> 336
   <212> DNA
   <213> Homo sapiens
<400> 65
   gatattgtga tgactcagtc tccactctcc ctgcccgtca cccctggaga gccggcctcc 60
   atctcctgca ggtctagtca gagcctcctg caaagtaatg gatacaatta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactctc 300
   actttcggcg gagggaccaa ggtggagatc aaacga 336
<210> 66
   <211> 384
   <212> DNA
   <213> Homo sapiens
<400> 66
   caggtgcagc tggtggagtc tgggggaggc gtggtccagc ctgggaggtc cctgagactc 60
   tcctgtgcag cgtctggatt caccttcagt agctatggca tgcactgggt ccgccaggct 120
   ccaggcaagg ggctggagtg ggtggcagtt gtatggtatg atggaagtaa taaatactat 180
   gcagactccg tgaagggccg attcaccatc tccagagaca attccaagaa cactctgaat 240
   ctgcaaatga acagcctgag agccgaggac acggctgtgt attattgtgc gagagatggg 300
   attactctgg ttcggggagt tatctactac tactacggca tggacgtctg gggccaaggg 360
   accacggtca ccgtctcctc agcc 384
<210> 67
   <211> 324
   <212> DNA
   <213> Homo sapiens
<400> 67
   gacatccaga tgacccagtc tccatcctcc ctgtctgcat ctgtaggaga cagagtcacc  60
   atcacttgcc gggcgagtca ggacattagc aactatttag cctggtatca gcagaaacca 120
   gggaaagttc cgaagctcct gatctatgct gcatcctctt tgcaatcagg ggtcccatct 180
   cggttcagtg gcagtggatc tgggacagat ttcactctca ccatcagcag cctgcagcct 240
   gaagatgttg caacttatta ctgtcaagag tttaacagtg ccccgctcac tttcggcgga 300
   gggaccaagg tggagatcaa acga                                         324
<210> 68
   <211> 384
   <212> DNA
   <213> Homo sapiens
<400> 68
   caggtgcagc tggtggagtc tgggggaggc gtggtccagc ctgggaggtc cctgagactc  60
   tcctgtgcag cgtctggatt caccttcagt agctatggca tacactggat ccgccaggct 120
   ccaggcaagg ggctggagtg ggtggcagtt atatggtatg atggaagtca taaatactat 180
   gcagactccg tgaagggccg attcaccatc tccagagaca attccaagaa cacgctttat 240
   cttcaaatga acagcctgag agccgaggac acggctgtgt attactgtgc gagagatggg 300
   attactatgg ttcggggagt cctctactat ttttacggta tggacgtctg ggccaagggg 360
   accacggtca ccgtctcctc agcc                                         384
<210> 69
   <211> 324
   <212> DNA
   <213> Homo sapiens
<400> 69
   gacatccaga tgacccagtc tccatcctcc ctgtctgcat ctgtaggaga cagagtcacc  60
   atcacttgcc gggcgagtca gggcattagc aattatttag cctggtatca gcagaaacca 120
   gggaaagttc ctaagctcct gatctatgct gcatccactt tgcaatcagg ggtcccatct 180
   cggttcagtg gcagtggatc tgggacagat ttcactctca ccatcagcag cctgcagcct 240
   gaagattttg caacttatta ctgtcaagag tttaacagtg ccccgctcac tttcggcgga 300
   gggaccaagg tagagatcaa acga                                         324
<210> 70
   <211> 363
   <212> DNA
   <213> Homo sapiens
<400> 70
   gaggtgcagc tggtggagtc tggaggagtc ttgatccagc ctggggggtc cctgagactc  60
   tcctgtgcgg cctctgggtt caccgtcagt agcaactaca tgagctgggt ccgccaggct 120
   ccagggaagg ggctggagtg ggtctcaatt atttatagcg atggtaacac atactacgca 180
   gactccgtga agggccgatt caccatctcc agagacaatt ccaagaacac gctgtatctt 240
   caaatgaaca gcctgagagc cgaggacacg gccgtgtatt actgtgctgg aactggaact 300
   acggactact actacggaat ggacgtctgg ggccaaggga ccacggtcac cgtctcctca 360
   gcc                                                                363
<210> 71
   <211> 336
   <212> DNA
   <213> Homo sapiens
<400> 71
   gatattgtga tgactcagtc tccactgtcc ctgcccgtca cccctggaga gccggcctcc  60
   atctcctgca ggtctagtca gagcctcctg caaactaatg gatacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttgggctt tattactgca tgcaagctct acaaactctc 300
   accttcggcc aagggacacg actggagatt aaacga                           336
<210> 72
   <231> 363
   <212> DNA
   <213> Homo sapiens
<400> 72
<210> 73
   <211> 336
   <212> DNA
   <213> Homo sapiens
<400> 73
   gatattgtga tgactcagtc tccactctcc ctgcccgtca cccctggaga gccggcctcc  60
   atctcctgca ggtctagtca gagcctcctg caaagtaatg gatacaacta tttggattgg 120
   tacctgcaga agccagggca gtctccacaa ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactctc 300
   accttcggcc aagggacacg actggagatt aaacga                           336
<210> 74
   <211> 372
   <212> DNA
   <213> Homo sapiens
<400> 74
   gaggtgcagc tggtggagtc tggaggaggc ttgatccagc ctggggggtc cctgagactc  60
   tcctgtgcag cctctgggtt caccgtcagt agcacctaca tgagctgggt ccgccaggct 120
   ccagggaagg ggctggagtg ggtctcagtt atttatagtg atggtaacac atactacgca 180
   gactccgtga agggccgatt caccatctcc agagacaatt ccaagaacac gctgtatctt 240
   caaatgaaca gcctgagagc cgaggactcg gccgtgtatt actgtgcggg acgtggatac 300
   agctatggga acgactacta ctacggtttg gacgtctggg gccaagggac cacggtcacc 360
   gtctcctcag cc                                                     372
<210> 75
   <211> 336
   <212> DNA
   <213> Homo sapiens
<400> 75
   gatattgtga tgactcagtc tccactctcc ctgcccgtca cccctggaga gccggcctcc 60
   atctcctgca ggtctagtca gagcctcctg caaagtaatg gatacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tattggggtt tattactgca tgcaagctct acaaactctc 300
   actttcggcg gagggaccaa ggtggagatc aaacga 336
<210> 76
   <211> 351
   <212> DNA
   <213> Homo sapiens
<400> 76
   gaggtgcagc tggtggagtc tgggggaggc ctggtcaagc ctggggggtc cctgagactc 60
   tcctgtgcag cctctggatt caccttcagt agctatagca tgaactgggt ccgccaggct 120
   ccagggaagg ggctggagtg ggtctcatcc attagtagta gtagtactta catatactac 180
   gcagactcag tgaagggccg attcaccatc tccagagaca acgccaagaa ctcactgtat 240
   ctgcaaatga acagcctgag agccgaggac acggctgtgt attactgtgc gagagatggg 300
   gcgatttttg gagtggttaa ctggtacttc gatctctggg gccgtggcac c 351
<210> 77
   <211> 324
   <212> DNA
   <213> Homo sapiens
<400> 77
   gaaattgtgc tgactcagtc tccagacttt cagtctgtga ctccaaagga gaaagtcacc 60
   atcacctgcc gggccagtca gagcattggt agtagcttac actggtacca gcagaaacca 120
   gatcagtctc caaagctcct catcaagtat gcttcccagt ccttctcagg ggtcccctcg 180
   aggttcagtg gcagtggatc tgggacagat ttcaccctca ccatcaatag cctggaagct 240
   gaagatgctg caacgtatta ctgtcatcag agtagtaatt tacctcacac tttcggccct 300
   gggaccaaag tggatatcaa acga 324
<210> 78
   <211> 369
   <212> DNA
   <213> Homo sapiens
<400> 78
   caggttcagc tggtgcagtc tggacctgag gtgaagaagc ctggggcctc agtgaaggtc 60
   tcctgcaagg cttctggtta cacctttacc agctatggta tcaactgggt gcgacaggcc 120
   cctggacaag ggcttgagtg gatgggatgg atcggcactt acaatggtaa cagaaactat 180
   gcacagaagc tccagggcag agtcaccatg accacagaca catccacgag cacagcctac 240
   atggagctga ggagcctgag atctgacgac acggccgtgt attactgtgc gagagacgat 300
   ttttggagtg gttattatga ctactttgac tactggggcc agggaaccct ggtcaccgtc 360
   tcctcagcc 369
<210> 79
   <211> 324
   <212> DNA
   <213> Homo sapiens
<400> 79
   gacatccaga tgacccagtc tccatcctcc ctgtctgcat ctgtaggaga cagagtcacc  60
   atcacttgcc gggcgagtca ggacattaac aattatttag cctggtatca gcagaaacca 120
   gggaaagttc ctaaactcct gatctatgct gcatccactt tgcaatcagg ggtcccatct 180
   cggttcagtg gcagtggatc tgggacagat ttcactctct ccatcagcag cctgcagcct 240
   gaagatgttg caacttatta ctgtcaaaag tataacagtg cccctcggac gttcggccaa 300
   gggaccaagg tggaaatcaa acga                                        324
<210> 80
   <211> 363
   <212> DNA
   <213> Homo sapiens
<400> 80
   caggtgcagc tgcaggagtc gggcccagga ctggtgaagc cttcggagac cctgtccctc  60
   acctgcattg tctctggtga ctccatcagt ggttactatt ggagctggat ccggcagccc 120
   gccgggaagg gactggagtg gattgggcgt atctatacca gtgggaacac catctacaac 180
   ccctccctca agagtcgagt caccatgtca gtagacacgt ccaagaacca gttctccctg 240
   aagctgagct ctgtgaccgc cgcggacacg gccgtgtatt actgtgcgag agagaggccc 300
   gactacggtg gtaacttctt tgactactgg ggccagggaa ccctggtcac cgtctcctca 360
   gcc                                                               363
<210> 81
   <211> 324
   <212> DNA
   <213> Homo sapiens
<400> 81
   gaaattgtgc tgactcagtc tccagacttt cagtctgtga ctccaaagga gaaagtcacc  60
   atcacctgcc gggccagtca gagcattggt agtagcttac actggtacca gcagaaacca 120
   gatcagtctc caaagctcct catcaagcat gcttcccagt ccttctcagg ggtcccctcg 180
   aggttcagtg gcagtggatc tgggacagat ttcaccctca ccatcaatag cctggaagct 240
   gaagatgctg caacgtatta ctgtcatcag agtagtagtt tacctcatac tttcggcgga 300
   gggaccaagg tggagatcaa acga                                        324
<210> 82
   <211> 351
   <212> DNA
   <213> Homo sapiens
<400> 82
   gaggtgcagt tggtggagtc tgggggaggc ctggtcaagc ctggggggtc cctgagactc  60
   tcctgtgcag cctctggatt caccttcagt agctatagca tgaactgggt ccgccaggct 120
   ccagggaagg ggctggagtg ggtctcatcc attagtagta gtagtagtta tatatattac 180
   gcagactcag tgaagggccg attcaccatc tccagagaca acgccaagaa ctccctgtat 240
   ctgcaaatga acagcctgag agccgaggac acggctgtgt attactgtgc gagagatggg 300
   gcgatttttg gagtggttaa ctggtacttc gatctctggg gccgtggcac c          351
<210> 83
   <211> 324
   <212> DNA
   <213> Homo sapiens
<400> 83
   gaaattgtgc tgactcagtc tccagacttt cagtctgtga ctccaaagga gaaagtcacc  60
   atcacctgcc gggccagtca gagcattggt agtagcttac actggtacca gctgaaacca 120
   gatcagtctc caaagctcct catcaagtat gtttcccagt ccttctcagg ggtcccctcg 180
   aggttcagtg gcagtggatc tgggacagat ttcaccctca ccatcagtag cctggaagct 240
   gaagatgctg caacgtattg ctgtcatcag agtagtagtt tacctcacac tttcggccct 300
   gggaccaaag tggatatcaa ccga                                         324
<210> 84
   <211> 363
   <212> DNA
   <213> Homo sapiens
<400> 84
   gaggtgcagc tggtggagtc tggaggaggc ttgatccagc ctggggggtc cctgagactc  60
   tcctgtgcag cctctgggtt caccgtcagt agcaactaca tgagctgggt ccgccaggct 120
   ccagggaagg ggctggagtg ggtctcagtt atttatagcg gtgataacac atactacgca 180
   gactccgtga agggccgctt caccatctcc agagacaatt ccaagaacac gctgtttctt 240
   caaatgaaca gcctgagagc cgaggacacg gccgtgtatt actgtgcggg aactggaact 300
   acgaactact actacggttt ggacgtctgg ggccaaggga ccacggtcac cgtctcctca 360
   gcc                                                                363
<210> 85
   <211> 363
   <212> DNA
   <213> Homo sapiens
<400> 85
   gaggtgcagc tggtggagtc tggaggagtc ttgatccagc ctggggggtc cctgagactc  60
   tcctgtgcag cctctgggtt caccgtcagt agcaactaca tgagctgggt ccgccaggct 120
   ccagggaagg ggctggagtg ggtctcaatt atttatagcg atggtaacac atactacgca 180
   gactccgtga agggccgatt caccatctcc agagacaatt ccaagaacac gctgtatctt 240
   caaatgaaca gcctgagagc cgaggacacg gccgtatatt actgtgctgg aactggaact 300
   acggactact actacggtat ggacgtctgg ggccaaggga ccacggtcac cgtctcctca 360
   gcc                                                                363
<210> 86
   <211> 372
   <212> DNA
   <213> Homo sapiens
<400> 86
   gaggtgcagc tggtggagtc tggaggaggc ttgatccagc ctggggggtc cctgagactc  60
   tcctgtgcag cctctgggtt caccgtcagt agcaactaca tgagctgggt ccgccaggct 120
   ccagggaagg ggctggagtg ggtctcaatt atttatagcg atggtaacac atactacgca 180
   gactccgtga agggccgatt caccatctcc agagacaatt ccaagaacac gctgtttctt 240
   caaatggaca gcctgagagc cgaggacacg gccgtgtatt actgtgcggg acgtggatac 300
   aactatggtt acgactacta ctacggtttg gacgtctggg gccaagggac cacggtcacc 360
   gtctcctcag cc                                                      372
<210> 87
   <211> 336
   <212> DNA
   <213> Homo sapiens
<400> 87
   gatattgtga tgactcagtc tccactctcc ctgcccgtca ctcctggaga gccggcctcc 60
   atctcctgca ggtctagtca gagcctcctg caaagtaatg gatacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactctc 300
   actttcggcg gagggaccaa ggtggagatc aaacga 336
<210> 88
   <211> 372
   <212> DNA
   <213> Homo sapiens
<400> 88
   gaggtgcagc tggtggagtc tggaggaggc ttgatccagc cgggggggtc cctgagactc 60
   tcctgtgcag cctctgggtt caccgtcagt agcaactaca tgagttgggt ccgccaggct 120
   ccagggaggg ggctggagtg ggtctcagtt atttatagcg atggtaacac atactacgca 180
   gactccgtga agggccgatt caccatctcc agagacaatt ccaagaacac gctgtatctt 240
   caaatgaaca gcctgagagc cgaggacacg gccgtgtatt actgtgcggg acgtggatac 300
   agctatggtt acgactacta ctacggtttg gacgtctggg gccaagggac cacggtcacc 360
   gtctcctcag cc 372
<210> 89
   <211> 25
   <212> DNA
   <213> misc_feature
<220>
   <221> misc_feature
   <222> (13)..(13)
   <223> wherein n may be g or c
<400> 89
   caccatggac tgnacctgga ggatc 25
<210> 90
   <211> 25
   <212> DNA
   <213> misc_feature
<220>
   <221> misc_feature
   <222> (22)..(22)
   <223> Wherein n may be a or c
<400> 90
   caccatggac tggacctgga gnatc 25
<210> 91
   <211> 25
   <212> DNA
   <213> misc_feature
<400> 91
   caccatggac tggacctgga gggtc 25
<210> 92
   <211> 25
   <212> DNA
   <213> misc_feature
<400> 92
   caccatggac tggatttgga ggatc 25
<210> 93
   <211> 24
   <212> DNA
   <213> misc_feature
<220>
   <221> misc_feature
   <222> (21)..(21)
   <223> Wherein n may be c or a
<400> 93
   caccatggac acactttgct ncac 24
<210> 94
   <211> 25
   <212> DNA
   <213> misc_feature
<220>
   <221> misc_feature
   <222> (10)..(10)
   <223> Wherein n may be a or g
<400> 94
   caccatggan ttggggctga gctgg 25
<210> 95
   <211> 25
   <212> DNA
   <213> misc_feature
<220>
   <221> misc_feature
   <222> (13)..(13)
   <223> Wherein n may be g or t
<400> 95
   caccatggag ttnggactga gctgg 25
<210> 96
   <211> 25
   <212> DNA
   <213> misc_feature
<220>
   <221> misc_feature
   <222> (19)..(19)
   <223> Wherein n may be g or t
<400> 96
   caccatggag tttgggctna gctgg 25
<210> 97
   <211> 25
   <212> DNA
   <213> misc_feature
<400> 97
   caccatggaa ctggggctcc gctgg 25
<210> 98
   <211> 25
   <212> DNA
   <213> misc_feature
<400> 98
   caccatggag ttggggctgt gctgg 25
<210> 99
   <211> 25
   <212> DNA
   <213> misc_feature
<400> 99
   caccatggag ttttggctga gctgg 25
<210> 100
   <211> 25
   <212> DNA
   <213> misc_feature
<400> 100
   caccatgacg gagtttgggc tgagc 25
<210> 101
   <211> 25
   <212> DNA
   <213> misc_feature
<220>
   <221> misc_feature
   <222> (10)..(10)
   <223> Wherein n may be a or g
<400> 101
   caccatgaan cacctgtggt tcttc 25
<210> 102
   <211> 25
   <212> DNA
   <213> misc_feature
<400> 102
   caccatgaaa catctgtggt tcttc 25
<210> 103
   <211> 25
   <212> DNA
   <213> misc_feature
<400> 103
   caccatgggg tcaaccgcca tcctc 25
<210> 104
   <211> 28
   <212> DNA
   <213> misc_feature
<400> 104
   caccatgtct gtctccttcc tcatcttc 28
<210> 105
   <211> 21
   <212> DNA
   <213> misc_feature
<400> 105
   atggggtccc aggttcacct c 21
<210> 106
   <211> 22
   <212> DNA
   <213> misc_feature
<400> 106
   atgttgccat cacaactcat tg 22

## Description

### Field

The invention relates to antibodies directed to Angiopoietin-like protein 4 (ANGPTL4), and uses of such antibodies. More specifically, the present invention relates to fully human monoclonal antibodies against ANGPTL4 and uses of these antibodies. Aspects of the invention also relate to hybridomas or other cell lines expressing such antibodies. The antibodies herein are useful as diagnostics and as treatments for diseases associated with the activity and/or overproduction of ANGPTL4.

### Background

ANGPTL4 is structurally related to other angiopoietins. (Yoon et al., Molec. Cell. Biol. 20: 5343-5349, (2000)). The angiopoietin family of secreted factors is functionally defined by the C-terminal fibrinogen (FBN)-like domain, which mediates binding to the Tie2 receptor and thereby facilitates a cascade of events ultimately regulating blood vessel formation or angiogenesis. Tie2 (also known as "TEK") is a receptor-like tyrosine kinase expressed almost exclusively in endothelial cells and early hematopoietic cells and required for the normal development of vascular structures during embryogenesis (Partanen, J. et al., Molec. Cell. Biol. 12: 1698-1707 (1992)). Davis et al. (Davis et al., Cell 87: 1161-1169, 1996) identified a secreted ligand for Tie2, termed angiopoietin-1. The human gene encodes a 498-amino acid polypeptide with predicted coiled-coil and fibrinogen-like domains. The structure of angiopoietin-1 differs from that of known angiogenic factors or other ligands for receptor tyrosine kinases. Although angiopoietin-1 bound and induced the tyrosine phosphorylation of Tie2, it did not directly promote the growth of cultured endothelial cells. However, its expression and close proximity to developing blood vessels implicated angiopoietin-1 in endothelial developmental processes.

By homology screening, Maisonpierre et al. (Maisonpierre et al., Science 277: 55-60 (1997)) identified a relative of ANG1, termed angiopoietin-2 (ANG2), and showed that it is a naturally occurring antagonist for Tie2. The predicted ANG2 protein is 496 amino acids long and has a secretion signal peptide. Human and mouse ANG2 are 85% identical in amino acid sequence and approximately 60% identical to their ANG1 homologs. Transgenic overexpression of ANG2 in mice disrupted blood vessel formation in the mouse embryo. In adult mice and humans, it was discovered that ANG2 is expressed only at sites of vascular remodeling.

Valenzuela et al. (Valenzuela et al., Proc. Nat. Acad. Sci. 96: 1904-1909, (1999)) identified two new angiopoietins: angiopoietin-3 (ANG3) in mouse, and angiopoietin-4 (ANG4) in human. They also identified several more distantly related sequences that did not seem to be true angiopoietins, in that they did not bind to Tie receptors. Although ANG3 and ANG4 are more structurally diverged from each other than are the mouse and human versions of ANG1 and ANG2, they appear to represent the mouse and human counterparts of the same gene locus, as revealed in chromosomal localization studies of all the angiopoietins in mouse and human.

Kim et al. (Kim et al., FEBS Lett. 443: 353-356, (1999)) isolated an adult human heart cDNA encoding a novel member of the angiopoietin family, angiopoietin-like-1 (ANGPTL1), which they called angiopoietin-3 (ANG3). ANGPTL1 is widely expressed in human adult tissues as a major 3.0-kb transcript and a less abundant 4.0-kb transcript. Its mRNA levels are highest in highly vascularized tissues. The deduced 491-amino acid ANGPTL1 protein is 29% identical to ANG1 and 26% identical to ANG2. ANGPTL1 contains the N-terminal coiled-coil domain and C-terminal fibrinogen-like domain characteristic of angiopoietins, as well as several potential glycosylation sites. ANGPTL1 has a putative signal sequence at its N terminus, and the authors demonstrated that the protein is secreted. Like ANG1, ANGPTL1 is not an endothelial cell mitogen *in vitro.*

The same group of investigators (Kim et al. (1999)) identified a cDNA from human heart tissue that encoded ANGPTL2, which they called ARP2 for 'angiopoietin-related protein-2.' The predicted 493-amino acid ANGPTL2 protein contains the coiled-coil and fibrinogen-like domains that are conserved in angiopoietins. Human ANGPTL2 shares 95% amino acid sequence identity with mouse ANGPTL2, 59% identity with human ANGPTL1, and 34% identity with human ANG1 and ANG2. Recombinant ANGPTL2 protein induced sprouting in vascular endothelial cells but did not bind to the Tiel or Tie2 receptor. ANGPTL2 may exert a function on endothelial cells through autocrine or paracrine action.

Conklin et al. (Conklin et al., Genomics 62: 477-482 (1999)) isolated a full-length cDNA from a human fetal liver/spleen cDNA library, which encodes a 460-amino acid protein sharing the characteristic structure of angiopoietins. This gene was named angiopoietin-like protein 3 (ANGPTL3). Camenisch et al. (Carnenisch et al., J. Biol. Chem. 277: 17281-17290, (2002)) determined that ANGPTL3 binds to human vascular endothelial cells; however, it does not bind to the Tie2 receptor, which is utilized by other members of the angiopoietin family to regulate blood vessel formation. Crystallographic studies and sequence analysis revealed that the fibrinogen-like domain of ANGPTL3 shares significant similarity with the C terminus of the gamma chain of human fibrinogen, suggesting that ANGPTL3 may bind integrins. Using a panel of integrin subunits expressed in 293 cells, they determined that cells expressing alpha-5/beta-3 integrins adhered to ANGPTL3-coated dishes. Binding induced integrin alpha-5/beta-3-dependent haptotactic endothelial cell adhesion and migration, and stimulated signal transduction pathways characteristic for integrin activation. ANGPTL3 also induced angiogenesis in the rat corneal assay. The C-terminal fibrinogen-like domain alone was sufficient to induce endothelial cell adhesion and in vivo angiogenesis.

Mapping of the hypolipidemia locus in the KK/San strain of the obese mouse lead to the identification of ANGPTL3 as a lipoprotein modulator (Koishi et al., Nature Genet. 30: 151-157, (2002)). The KK obese mouse is moderately obese and has abnormally high levels of plasma insulin, glucose, and lipids. This is a multigenic syndrome that resembles type II diabetes in the human. KK/San is a substrain that showed abnormally low plasma lipid levels (hypolipidemia). Overexpression of ANGPTL3 gene or intravenous injection of the purified protein in KK/San mice elicited an increase in circulating plasma lipid levels. This increase was also observed in normal C57BL/6J mice. These data suggested that ANGPTL3 regulates lipid metabolism in animals. Shimizugawa et al. (Shimizugawa et al., J. Biol. Chem. 277: 33742-33748 (2002)) determined that overexpression of ANGPTL3 in KK/San mice results in a marked increase of triglyceride-enriched very low density lipoprotein (VLDL). *In vivo* studies revealed that there is no significant difference between mutant and wildtype KK mice in the hepatic VLDL triglyceride secretion rate. However, turnover studies using radiolabeled VLDL revealed that the clearance of 3H-triglyceride-labeled VLDL was significantly enhanced in KK/San mice, whereas the clearance of 125I-labeled protein from VLDL was only slightly enhanced. In vitro analysis of recombinant protein revealed that ANGPTL3 directly inhibits lipoprotein lipase (LPL) activity. It was concluded that ANGPTL3 regulates VLDL triglyceride levels through the inhibition of LPL activity.

Angiopoietin-like protein 4 (ANGPTL4) is also known as PPARG (peroxisome proliferative-activated receptor-gamma) angiopoietin-related protein, PGAR, fasting-induced adipose factor (FIAF), or hepatic fibrinogen/angiopoietin-related protein (HFARP). The amino acid sequence of ANGPTL4 can be found on Genbank with accession number NP_647475. Using a subtractive cloning strategy to identify downstream targets of ANGPTL4, and by screening cDNA libraries, Yoon et al. (Yoon et al., Molec. Cell. Biol. 20: 5343-5349 (2000)) isolated mouse and human cDNAs encoding ANGPTL4. The 406-amino acid, 60-kD human ANGPTL4 protein, which shares 75% amino acid identity with the mouse protein, is a member of the angiopoietin family of secreted proteins and bears highest similarity to ANG2. ANGPTL4 shares 35% identity to ANGPTL3.

Using recombinant ANGPTL4, it has been shown that the protein significantly stimulates the migration and invasion of endothelial cells, and promotes *in vitro* tube formation of endothelial cells on Matrigel. ANGPTL4 was further shown to stimulate angiogenesis in a subcutaneous Matrigel plug mouse model (Zhu et al, Zhonghua Yi Xue Za Zhi 2002 Jan 25;82(2)). These results clearly indicate that, like ANGPLT3, ANGPLT4 plays a role in angiogenesis via an unknown mechanism.

Angiogenesis, or neovascularisation, is highly regulated, and it is typically short-lived (usually lasting for less than one week). Nevertheless, angiogenesis can also occur under abnormal conditions. Tumor cells, for example, can turn-on angiogenesis. As new blood vessels bring in fresh nutrients and growth factors, the tumor mass can expand. In fact, angiogenesis appears to be one of the crucial steps in a tumor's transition from a small, harmless cluster of mutated cells to a large, malignant cancer, capable of spreading (metastasizing) to other organs throughout the body. Angiogenesis is also involved in numerous other diseases termed angiogenic diseases. These include benign tumors, rheumatoidal arthritis, fibrosis of the liver and of the kidney, and serious eye-diseases in which abnormal vessels proliferate and destroy vision, such as diabetic retinopathy, macular degeneration, neovascular glaucoma, and retrolental fibroplasia. Ocular neovascularisation is, indeed, the most common cause of blindness.

Interestingly, it was discovered recently that an intravenous injection of the ANGPLT4 protein in KK/San mice rapidly increased the circulating plasma lipid levels at a higher rate than ANGPTL3. Furthermore, ANGPTL4 inhibited the lipoprotein lipase activity *in vitro* (Yoshida et al., J. Lipid Res. 43(11): 1770-1772 (2002)). These data strongly support that ANGPTL4 also plays a role as a lipid metabolism modulator, which regulates VLDL triglyceride levels through the inhibition of LPL activity.

It has been shown that antibodies to angiopoietin related protein 4 (ARP4) can inhibit ARP4-medicated angiogenesis and renal cell carcinoma survival *in vitro* by Zhong et al (Zhong et al, Cancer Res. May 1, 2005 65; 706).

Accordingly, there is a need in the art for a novel approach to treat and diagnose hyperlipidemia, diseases related to angiogenesis including cancer, benign tumors, rheumatoidal arthritis, fibrosis of the liver and of the kidney, serious eye-diseases in which abnormal vessels proliferate and destroy vision, such as diabetic retinopathy, macular degeneration, neovascular glaucoma, and retrolental fibroplasia, and other negative effects caused by ANGPTL4.

### Summary

The antibodies described herein are directed to ANGPTL4 and preferably affect ANGPTL4 function. Other embodiments relate to fully human anti-ANGPTL4 antibodies and anti-ANGPTL4 antibody preparations with desirable properties from a therapeutic perspective, including strong binding affinity for ANGPTL4, the ability to neutralize ANGPTL4 *in vitro* and *in vivo*, and the ability to inhibit ANGPTL4 induced angiogenesis.

In a preferred embodiment, antibodies described herein bind to ANGPTL4 with very high affinities (Kd). For example a human, rabbit, mouse, chimeric or humanized antibody that is capable of binding ANGPTL4 with a Kd less than, but not limited to, 10⁻⁷, 10⁻⁸, 10⁻⁹, 10⁻¹⁰, 10⁻¹¹, 10⁻¹², 10⁻¹³ or 10⁻¹⁴ M, or any range or value therein.

The present invention provides an isolated antibody, or antigen-binding fragment thereof that binds to ANGPTL4 and that comprises both a heavy chain amino acid sequence and a light chain amino acid sequence as defined in claim 1, compositions, and conjugates to therapeutic agents thereof and use in an assay kit as defined in claim 15. The present invention also provides for the use of such antibodies as a medicament, or in a method of treating a disease related to angiogenesis in a mammal.

Certain disclosures herein include an isolated antibody, or fragment thereof, that binds to ANGPTL4 and that comprises a heavy chain polypeptide having a sequence selected from the group consisting of SEQ ID NOs: 24, 25, 26, 27, 29, 30, 32, 34, 36, 38, 40, 41, 42, 43, 44, 46, 47, 48, 49, 51, and 53.

Further disclosures include an isolated antibody, or fragment thereof, that binds to ANGPTL4 and that comprises a light chain polypeptide having a sequence selected from the group consisting of SEQ ID NOs: 2, 3, 4, 5, 6, 7, 9, 10, 12, 14, 16, 18, 19, 21, and 22.

Additional disclosures include an isolated antibody, or fragment thereof, that binds to ANGPTL4 and that comprises a heavy chain polypeptide having a sequence selected from the group consisting of SEQ ID NOs: 24, 25, 26, 27, 29, 30, 32, 34, 36, 38, 40, 41, 42, 43, 44, 46, 47, 48, 49, 51, and 53, and that comprises a light chain polypeptide having a sequence selected from the group consisting of SEQ ID NOs: 2, 3, 4, 5, 6, 7, 9, 10, 12, 14, 16, 18, 19, 21, and 22.

In some embodiments, the isolated antibodies described herein are monoclonal antibodies. In other embodiments, the isolated antibodies are chimeric antibodies. In yet another embodiment, the isolated antibodies are fully human antibodies.

Other disclosures include an isolated antibody that binds to ANGPTL4 and that comprises a heavy chain polypeptide having a sequence comprising the following complementarity determining regions (CDRs): (a) a CDR1 region found within SEQ ID NOs: 24, 25, 26, 27, 29, 30, 32, 34, 36, 38, 40, 41, 42, 43, 44, 46, 47, 48, 49, 51, or 53; b) a CDR2 region found within SEQ ID NOs: 24, 25, 26, 27, 29, 30, 32, 34, 36, 38, 40, 41, 42, 43, 44, 46, 47, 48, 49, 51, or 53; and (c) a CDR3 region found within SEQ ID NOs: 24, 25, 26, 27, 29, 30, 32, 34, 36, 38, 40, 41, 42, 43, 44, 46, 47, 48, 49, 51, or 53.

Additional disclosures include an isolated antibody that binds to ANGPTL4 and that comprises a light chain polypeptide having a sequence comprising the following complementarity determining regions (CDRs): (a) a CDR1 region found within SEQ ID NOs: 2, 3, 4, 5, 6, 7, 9, 10, 12, 14, 16, 18, 19, 21, or 22; (b) a CDR2 region found within SEQ ID NOs: 2, 3, 4, 5, 6, 7, 9, 10, 12, 14, 16, 18, 19, 21, or 22; and (c) a CDR3 region found within SEQ ID NOs: 2, 3, 4, 5, 6, 7, 9,10, 12, 14, 16, 18,19, 21, or 22.

It will be appreciated that embodiments described herein are not limited to any particular form of an antibody. For example, the anti-ANGPTL4 antibodies, described herein may be a full length antibody (e.g. having an intact human Fc region) or an antibody fragment (e.g. a Fab, Fab' or F(ab')₂). In addition, the antibodies described herein can be manufactured from a hybridoma that secretes the antibody, or from a recombinantly produced cell that has been transformed or transfected with a gene or genes encoding the antibody. Specifically, the cell can be a Chinese hamster ovary cell.

In other embodiments, the anti-ANGPTL4 antibodies described herein can be associated with a pharmaceutically acceptable carrier or diluent In further embodiments, the antibodies described herein can be conjugated to any therapeutic agent Specifically, an anti-ANGPTL4 antibodies can be linked to a therapeutic agent that is a radioisotope or a toxin. Preferably, such antibodies may be used for the treatment of diseases, such as tumors and cancer.

Further embodiments include methods of inhibiting angiogenesis associated with the expression of ANGPTL4, comprising treating cells expressing ANGPTL4 with an effective amount of an anti-ANGPTL4 antibody or fragment thereof. These methods can be performed *in vivo.* In other embodiments these methods can be performed on a mammal, particularly a human. In still other embodiments these methods can be performed on a mammal that suffers from a cancer involving angiogenesis. Treatable cancers herein include lung, colon, gastric, renal, prostate, ovarian, uterine, and ocular carcinomas, for example.

In still other embodiments, the antibodies described herein can treat and diagnose hyperlipidemia, diseases related to angiogenesis including, rheumatoidal arthritis, fibrosis of the liver and of the kidney, serious eye-diseases in which abnormal vessels proliferate and destroy vision, such as diabetic retinopathy, macular degeneration, neovascular glaucoma, and retrolental fibroplasia, and other negative effects caused by ANGPTL4. For example, some non-neoplastic conditions that can be treated or diagnosed by the antibodies of the invention include, but are not limited to, undesired or aberrant hypertrophy, arthritis, psoriasis, psoriatic plaques, sarcoidosis, atherosclerosis, atherosclerotic plaques, edema from myocardial infarction, diabetic and other proliferative retinopathies including retinopathy of prematurity, retrolental fibroplasia, neovascular glaucoma, age-related macular degeneration, diabetic macular edema, corneal neovascularization, corneal graft neovascularization, corneal graft rejection, retinal/choroidal neovascularization, neovascularization of the angle (rubeosis), ocular neovascular disease, vascular restenosis, arteriovenous malformations (AVM), meningioma, hemangioma, angiofibroma, thyroid hyperplasias (including Grave's disease), corneal and other tissue transplantation, chronic inflammation, lung inflammation, acute lung injury/ARDS, sepsis, primary pulmonary hypertension, malignant pulmonary effusions, cerebral edema (e.g., associated with acute stroke/closed head injury/trauma), synovial inflammation, pannus formation in RA, myositis ossificans, hypertropic bone formation, osteoarthritis (OA), refractory ascites, polycystic ovarian disease, endometriosis, 3rd spacing of fluid diseases (pancreatitis, compartment syndrome, burns, bowel disease), uterine fibroids, premature labor, chronic inflammation such as IBD (Crohn's disease and ulcerative colitis), renal allograft rejection, inflammatory bowel disease, nephrotic syndrome, undesired or aberrant tissue mass growth (non-cancer), obesity, adipose tissue mass growth, hemophilic joints, hypertrophic scars, inhibition of hair growth, Osler-Weber syndrome, pyogenic granuloma retrolental fibroplasias, scleroderma, trachoma, vascular adhesions, synovitis, dermatitis, preeclampsia, ascites, pericardial effusion (such as that associated with pericarditis), and pleural effusion.

Different embodiments include an assay kit for the detection of ANGPTL4 in mammalian tissues or cells to screen for lung, colon, gastric, renal, prostate, ovarian, uterine, or ocular carcinomas, comprising an antibody that binds to ANGPTL4 and means for indicating the reaction of the antibody with the antigen, if present Many different types of antibodies can be used in the assay kits described herein. For example, the antibody can be a monoclonal antibody. In certain embodiments the antibody that binds ANGPTL4 can be a labeled antibody. Specifically, this antibody can be labeled with a marker selected from the group consisting of a fluorochrome, an enzyme, a radionuclide and a radiopaque material. In other embodiments, the antibody that binds to ANGPTL4 is unlabeled and the means for indicating the reaction of the antibody with the antigen includes a labeled second antibody that is an immunoglobulin. Specifically, the second antibody can be labeled with a marker selected from the group consisting of a fluorochrome, an enzyme, a radionuclide and a radiopaque material.

In other aspects, the invention provides an article of manufacture including a container, which includes a composition containing an anti-ANGPTL4 antibody, and a package insert or label indicating that the composition can be used to treat cancer characterized by the overexpression of ANGPTL4. Treatable cancers include lung, colon, gastric, renal, prostate, ovarian, uterine, and ocular carcinomas, for example.

In certain embodiments, the antibodies described herein can treat and diagnose hyperlipidemia, diseases related to angiogenesis including cancer, benign tumors, rheumatoidal arthritis, fibrosis of the liver and of the kidney, serious eye-diseases in which abnormal vessels proliferate and destroy vision, such as diabetic retinopathy, macular degeneration, neovascular glaucoma, and retrolental fibroplasia, and other negative effects caused by ANGPTL4.

The present invention further provides isolated polynucleotides encoding the heavy chain amino acid sequence and the light chain amino acid sequence of a human anti-ANGPTL4 antibody as defined in claim 14.

Further disclosures include an isolated polynucleotide molecule having a nucleotide sequence encoding a heavy chain polypeptide of a human anti-ANGPTL4 antibody; wherein the nucleotide sequence is selected from the group consisting of SEQ ID Nos: 54, 56, 58, 59, 61, 62, 64, 66, 68, 70, 72, 74, 76, 78, 80, 82, 84, 85, 86, and 88.

Still other disclosures include an isolated polynucleotide molecule having a nucleotide sequence encoding a light chain polypeptide of a human anti-ANGPTL4 antibody; wherein the nucleotide sequence is selected from the group consisting of SEQ ID NOs: 55, 57, 60, 63, 65, 67, 69, 71, 73, 75, 77, 79, 81, 83, and 87.

### Brief Description of the Drawings

FIGURE 1 illustrates sensograms showing the binding kinetics for six purified antibodies (1.1.2, 1.2.1, 1.3.1, 1.4.1, 1.6.1, and 1.9.1) in a BiaCore® binding assay. The star represents mAbs selected to be studied in medium resolution.
FIGURE 2 illustrates sensograms showing the binding kinetics for six purified antibodies (1.10.2, 1.7.3, 1.5.2, 1.8.2, 2.7.3, and 1.11.1) in a BiaCore® binding assay. The stars represent mAbs selected to be studied in medium resolution.
FIGURE 3 is a line graph illustrating fitting data for ANGPTL4/mAb 1.6.1, as determined using a BiaCore® binding assays.
FIGURE 4 is a line graph illustrating fitting data for ANGPTL4/mAb 1.7.3, as determined using a BiaCore® binding assay.
FIGURE 5 is a line graph illustrating fitting data for ANGPTL4/mAb 1.5.2, as determined using a BiaCore® binding assay.
FIGURES 6 (A) and (B) are bar graphs indicating the effectiveness of various ANGPTL4/mAbs to bind ANGPTL4 and induce cell death in 786-0 cancer cells.
FIGURE 7 is a bar graph demonstrating the effectiveness of ANGPTL4/mAb 1.7.1 and ANGPTL4/mAb 1.6.1 to inhibit ANGPTL4 induced Human Umbilical Vein Endothelial Cell (HUVEC) proliferation.
FIGURE 8 is a bar graph demonstrating the effectiveness of ANGPTL4/mAb 1.7.1 and ANGPTL4/mAb 1.6.1 to inhibit ANGPTL4 induced HUVEC migration.
FIGURE 9 shows photomicrographs of the in vitro tube formation assay: In vitro tube formation by (A) VEGF (10 ng/ml) and (B) ANGPTL4 (0.5 µg/ml). ANGPTL4 neutralizing mAbs (10 µg/ml) inhibited ANGPTL4 mediated tube formation (CR064 mAb 1.7.1 (C) and CR064 mAb 1.6.1 (D)).

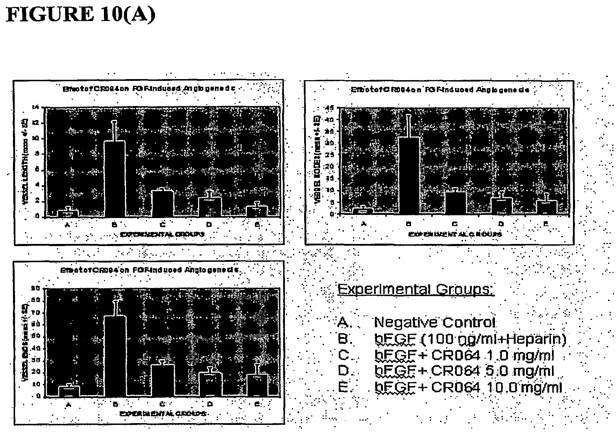

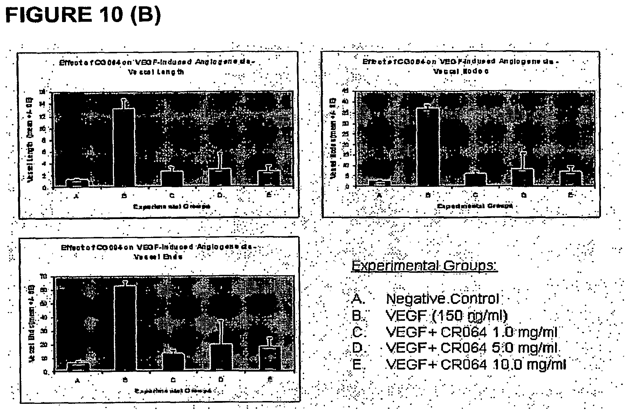

FIGURE 10 shows data from morphometric analyses. (A). Morphometric measurements of the lengths, nodes and ends of the blood vessels in various experimental groups (A-E). Data are presented as mean + STDEV (n=5). Compared to negative control, bFGF increased the vessel length, nodes and ends by 12, 17 and 9 folds respectively. ANGPTL4 mAb designated CR064 inhibited vessel lengths, nodes and ends at all concentrations and the inhibition was statistically significant (p<0.01). (B) Morphometric measurements of the lengths, nodes and ends of the blood vessels in various experimental groups (A-E). Data are presented as mean + STDEV (n=5). Compared to negative control, VEGF increased the vessel length, nodes and ends by 11, 15 and 10 folds respectively. ANGPTL4 mAb designated CR064 inhibited vessel lengths, nodes and ends at all concentrations tested and the inhibition was statistically significant (p<0.01).

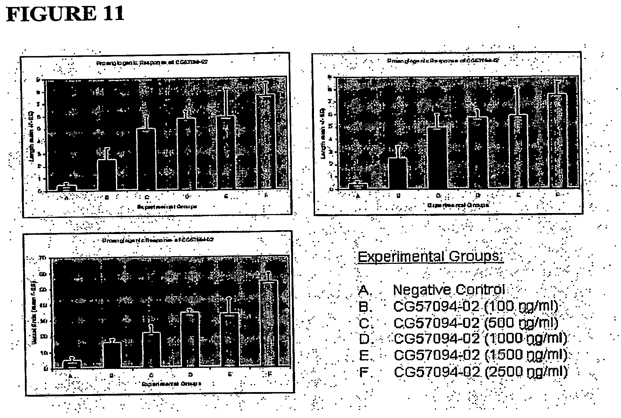

FIGURE 11 shows morphometric measurements of the lengths, nodes and ends of the blood vessels in various experimental groups (A-F). Data are presented as mean + STDEV (n=5). Compared to negative control, ANGPTL4/CG57094 increased the vessel length, nodes and ends in a dose dependent manner. At 500 ng/ml, ANGPTL4 increased vessel lengths, nodes and ends by 12, 12, and 5 fold, respectively, which were comparable with bFGF (100 ng/ml) and VEGF (150 ng/ml) mediated increases in vessel length, nodes and ends. ANGPTL4 mediated increases in vessel lengths, nodes and ends were statistically significant at concentrations 500 ng/ml and higher (P<0.01).

### Detailed Description

### Therapeutics and Diagnostics

The anti-ANGPTL4 antibodies provided herein are useful in the prevention, treatment, and diagnosis of disorders associated with the activity and/or overproduction of ANGPTL4, including hyperlipidemia, and diseases related to angiogenesis including cancer, benign tumors, rheumatoidal arthritis, fibrosis of the liver and of the kidney, serious eye-diseases in which abnormal vessels proliferate and destroy vision, such as diabetic retinopathy, macular degeneration, neovascular glaucoma, and retrolental fibroplasia.

More specifically, patients to be treated with the anti-ANGPTL4 antibodies described herein include patients having, or are suspected of having, tumors, such as esophageal, pancreatic, colorectal tumors, carcinomas, such as renal cell carcinoma (RCC), cervical carcinomas and cervical intraepithelial squamous and glandular neoplasia, and cancers, such as colorectal cancer, breast cancer, lung cancer, and other malignancies. It is desirable to administer anti-ANGPTL4 antibodies as early as possible in the development of the tumor, and to continue treatment for as long as is necessary.

In addition to treating patients having cancer, the antibodies described herein can be used to treat patients having or suspected of having other angiogenesis or ANGPTL4 related disorders including, hyperlipidemia, rheumatoidal arthritis, fibrosis of the liver and of the kidney, serious eye-diseases in which abnormal vessels proliferate and destroy vision, such as diabetic retinopathy, macular degeneration, neovascular glaucoma, and retrolental fibroplasia.

In the treatment and prevention of an ANGPTL4-associated disorder by an anti-ANGPTL4 antibody, the antibody composition can be formulated, dosed, and administered in a fashion consistent with good medical practice. Factors for consideration in this context include the particular disorder being treated, the particular mammal being treated, the clinical condition of the individual patient, the cause of the disorder, the site of delivery of the antibody, the particular type of antibody, the method of administration, the scheduling of administration, and other factors known to medical practitioners. The "therapeutically effective amount" of antibody to be administered will be governed by such considerations, and is the minimum amount necessary to prevent, ameliorate, or treat the disorder, including treating cancer, tumors, hyperlipidemia, rheumatoidal arthritis, fibrosis of the liver and of the kidney, serious eye-diseases in which abnormal vessels proliferate and destroy vision, such as diabetic retinopathy, macular degeneration, neovascular glaucoma, and retrolental fibroplasia. The administered amount of ANGPTL4 is preferably below the amount that is toxic to the host, or renders the host significantly more susceptible to infections.

The initial pharmaceutically effective amount of the antibody administered parenterally is preferably in the range of about 0.1 to 50 mg/kg of patient body weight per day, with the typical initial range of antibody used being 0.3 to 20 mg/kg/day, more preferably 0.3 to 15 mg/kg/day. The desired dosage can be delivered by a single bolus administration, by multiple bolus administrations, or by continuous infusion administration of antibody, depending on the pattern of pharmacokinetic decay that the practitioner wishes to achieve.

Suggested amounts of antibody are subject to a great deal of therapeutic discretion. The key factor in selecting an appropriate dose and scheduling is the result obtained. For example, the antibody may be optionally formulated with one or more agents currently used to prevent or treat tumors such as standard- or high-dose chemotherapy and hematopoietic stem-cell transplantation. The effective amount of such other agents depends on the amount of anti-ANGPTL4 antibody present in the formulation, the type of disorder or treatment, and other relevant factors.

In further embodiments, the anti-ANGPTL4 antibodies described herein can be used for the identification of risk factors, diagnosis, and staging of diseases and disorders associated with ANGPTL4 activity and/or overexpression. These disorders and diseases include, without limitation, cancer, tumors, hyperlipidemia, disorders associated with angiogenesis, including rheumatoidal arthritis, fibrosis of the liver and of the kidney, serious eye-diseases in which abnormal vessels proliferate and destroy vision, such as diabetic retinopathy, macular degeneration, neovascular glaucoma, and retrolental fibroplasia.

Furthermore, biological samples from normal patients and patients suffering from disorders associated with ANGPTL4 can then be compared in order to develop diagnostic kits and screening assays. The concentration of ANGPTL4 in a particular biological sample can be assessed using quantitative sandwich ELISA with 2 fully human anti-ANGPTL4 antibodies. The measured levels of ANGPTL4 in samples from various types of patients can then be used to develop diagnostic kits and screening assays to help practitioners diagnose and track the staging of various types ANGPTL4-related disorders in afflicted patients.

### Definitions

Unless otherwise defined, scientific and technical terms used in connection with the antibodies described herein shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. *See e.g.,* Sambrook et al. Molecular Cloning: A Laboratory Manual (3rd ed., Cold Spring Harbor Laboratory Press, Cold Spring Harbor, N.Y. (2001)), which is incorporated herein by reference. The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

As utilized in accordance with the present disclosure, the following terms, unless otherwise indicated, shall be understood to have the following meanings:

The term "isolated polynucleotide" as used herein shall mean a polynucleotide of genomic, cDNA, or synthetic origin or some combination thereof, which by virtue of its origin the "isolated polynucleotide" (1) is not associated with all or a portion of a polynucleotide in which the "isolated polynucleotide" is found in nature, (2) is operably linked to a polynucleotide which it is not linked to in nature, or (3) does not occur in nature as part of a larger sequence.

The term "isolated protein" referred to herein means a protein of cDNA, recombinant RNA, or synthetic origin or some combination thereof, which by virtue of its origin, or source of derivation, the "isolated protein" (1) is not associated with proteins found in nature, (2) is free of other proteins from the same source, e.g. free of murine proteins, (3) is expressed by a cell from a different species, or (4) does not occur in nature.

The term "polypeptide" is used herein as a generic term to refer to native protein, fragments, or analogs of a polypeptide sequence. Hence, native protein, fragments, and analogs are species of the polypeptide genus. Preferred polypeptides described herein comprise the human heavy chain immunoglobulin molecules and the human light chain immunoglobulin molecules, as well as antibody molecules formed by combinations comprising the heavy chain immunoglobulin molecules with light chain immunoglobulin molecules, such as the light chain immunoglobulin molecules, and vice versa, as well as fragments and analogs thereof.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory or otherwise is naturally-occurring.

The term "operably linked" as used herein refers to positions of components so described that are in a relationship permitting them to function in their intended manner. For example, a control sequence "operably linked" to a coding sequence is ligated in such a way that expression of the coding sequence is achieved under conditions compatible with the control sequences.

The term "control sequence" as used herein refers to polynucleotide sequences which are necessary to effect the expression and processing of coding sequences to which they are ligated. The nature of such control sequences differs depending upon the host organism; in prokaryotes, such control sequences generally include promoter, ribosomal binding site, and transcription termination sequence; in eukaryotes, generally, such control sequences include promoters and transcription termination sequence. The term "control sequences" is intended to include, at a minimum, all components whose presence is essential for expression and processing, and can also include additional components whose presence is advantageous, for example, leader sequences and fusion partner sequences.

The term "polynucleotide" as referred to herein means a polymeric form of nucleotides of at least 10 bases in length, either ribonucleotides or deoxynucleotides or a modified form of either type of nucleotide. The term includes single and double stranded forms of DNA.

The term "oligonucleotide" referred to herein includes naturally occurring, and modified nucleotides linked together by naturally occurring, and non-naturally occurring oligonucleotide linkages. Oligonucleotides are a polynucleotide subset generally comprising a length of 200 bases or fewer. Preferably, oligonucleotides are 10 to 60 bases in length and most preferably 12, 13, 14, 15, 16, 17, 18, 19, or 20 to 40 bases in length. Oligonucleotides are usually single stranded, e.g. for probes; although oligonucleotides may be double stranded, e.g. for use in the construction of a gene mutant. Oligonucleotides described herein can be either sense or antisense oligonucleotides.

The term "naturally occurring nucleotides" referred to herein includes deoxyribonucleotides and ribonucleotides. The term "modified nucleotides" referred to herein includes nucleotides with modified or substituted sugar groups and the like. The term "oligonucleotide linkages" referred to herein includes oligonucleotides linkages such as phosphorothioate, phosphorodithioate, phosphoroselenoate, phosphorodiselenoate, phosphoroanilothioate, phoshoraniladate, phosphoroamidate, and the like. *See e.g.,* LaPlanche et al. Nucl. Acids Res. 14:9081 (1986); Stec et al. J. Am. Chem. Soc. 106:6077 (1984); Stein et al. Nucl. Acids Res. 16:3209 (1988); Zon et al. Anti-Cancer Drug Design 6:539 (1991); Zon et al. Oligonucleotides and Analogues: A Practical Approach, pp. 87-108 (F. Eckstein, Ed., Oxford University Press, Oxford England (1991)); Stec et al. U.S. Patent No. 5,151,510; Uhlmann and Peyman Chemical Reviews 90:543 (1990), the disclosures of which are hereby incorporated by reference. An oligonucleotide can include a label for detection, if desired.

The term "selectively hybridize" referred to herein means to detectably and specifically bind. Polynucleotides, oligonucleotides and fragments thereof, as described herein, selectively hybridize to nucleic acid strands under hybridization and wash conditions that minimize appreciable amounts of detectable binding to nonspecific nucleic acids. High stringency conditions can be used to achieve selective hybridization conditions as known in the art and discussed herein. Generally, the nucleic acid sequence homology between the polynucleotides, oligonucleotides, and fragments described herein and a nucleic acid sequence of interest will be at least 80%, and more typically with preferably increasing homologies of at least 85%, 90%, 95%, 99%, and 100%. Two amino acid sequences are homologous if there is a partial or complete identity between their sequences. For example, 85% homology means that 85% of the amino acids are identical when the two sequences are aligned for maximum matching. Gaps (in either of the two sequences being matched) are allowed in maximizing matching; gap lengths of 5 or less are preferred with 2 or less being more preferred. Alternatively and preferably, two protein sequences (or polypeptide sequences derived from them of at least 30 amino acids in length) are homologous, as this term is used herein, if they have an alignment score of at more than 5 (in standard deviation units) using the program ALIGN with the mutation data matrix and a gap penalty of 6 or greater. *See* Dayhoff, M.O., in Atlas of Protein Sequence and Structure, pp. 101-110 (Volume 5, National Biomedical Research Foundation (1972)) and Supplement 2 to this volume, pp. 1-10. The two sequences or parts thereof are more preferably homologous if their amino acids are greater than or equal to 50% identical when optimally aligned using the ALIGN program. The term "corresponds to" is used herein to mean that a polynucleotide sequence is homologous (i.e., is identical, not strictly evolutionarily related) to all or a portion of a reference polynucleotide sequence, or that a polypeptide sequence is identical to a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

The following terms are used to describe the sequence relationships between two or more polynucleotide or amino acid sequences: "reference sequence", "comparison window", "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA or gene sequence given in a sequence listing or may comprise a complete cDNA or gene sequence. Generally, a reference sequence is at least 18 nucleotides or 6 amino acids in length, frequently at least 24 nucleotides or 8 amino acids in length, and often at least 48 nucleotides or 16 amino acids in length. Since two polynucleotides or amino acid sequences may each (1) comprise a sequence (i.e., a portion of the complete polynucleotide or amino acid sequence) that is similar between the two molecules, and (2) may further comprise a sequence that is divergent between the two polynucleotides or amino acid sequences, sequence comparisons between two (or more) molecules are typically performed by comparing sequences of the two molecules over a "comparison window" to identify and compare local regions of sequence similarity. A "comparison window", as used herein, refers to a conceptual segment of at least 18 contiguous nucleotide positions or 6 amino acids wherein a polynucleotide sequence or amino acid sequence may be compared to a reference sequence of at least 18 contiguous nucleotides or 6 amino acid sequences and wherein the portion of the polynucleotide sequence in the comparison window may comprise additions, deletions, substitutions, and the like (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith and Waterman Adv. Appl. Math. 2:482 (1981), by the homology alignment algorithm of Needleman and Wunsch J. Mol. Biol. 48:443 (1970), by the search for similarity method of Pearson and Lipman Proc. Natl. Acad. Sci. (U.S.A.) 85:2444 (1988), by computerized implementations of these algorithms (GAP, BESTFIT, FASTA, and TFASTA in the Wisconsin Genetics Software Package Release 7.0, (Genetics Computer Group, 575 Science Dr., Madison, Wis.), Geneworks™, or MacVector® software packages), or by inspection, and the best alignment (i.e., resulting in the highest percentage of homology over the comparison window) generated by the various methods is selected.

The term "sequence identity" means that two polynucleotide or amino acid sequences are identical (i.e., on a nucleotide-by-nucleotide or residue-by-residue basis) over the comparison window. The term "percentage of sequence identity" is calculated by comparing two optimally aligned sequences over the window of comparison, determining the number of positions at which the identical nucleic acid base (e.g., A, T, C, G, U, or I) or residue occurs in both sequences to yield the number of matched positions, dividing the number of matched positions by the total number of positions in the comparison window (i.e., the window size), and multiplying the result by 100 to yield the percentage of sequence identity. The terms "substantial identity" as used herein denotes a characteristic of a polynucleotide or amino acid sequence, wherein the polynucleotide or amino acid comprises a sequence that has at least 85 percent sequence identity, preferably at least 90 to 95 percent sequence identity, more usually at least 99 percent sequence identity, as compared to a reference sequence over a comparison window of at least 18 nucleotide (6 amino acid) positions, frequently over a window of at least 24-48 nucleotide (8-16 amino acid) positions, wherein the percentage of sequence identity is calculated by comparing the reference sequence to the sequence which may include deletions or additions which total 20 percent or less of the reference sequence over the comparison window. The reference sequence may be a subset of a larger sequence.

As used herein, the twenty conventional amino acids and their abbreviations follow conventional usage. *See* Immunology - A Synthesis (2nd Edition, E.S. Golub and D.R. Gren, Eds., Sinauer Associates, Sunderland, Mass. (1991)), which is incorporated herein by reference. Stereoisomers (e.g., D-amino acids) of the twenty conventional amino acids, unnatural amino acids such as α-, α-disubstituted amino acids, N-alkyl amino acids, lactic acid, and other unconventional amino acids may also be suitable components for polypeptides described herein. Examples of unconventional amino acids include: 4-hydroxyproline, γ-carboxyglutamate, ε-N,N,N-trimethyllysine, ε-N3-acetyllysine, O-phosphoserine, N-acetylserine, N-formylmethionine, 3-methylhistidine, 5-hydroxylysine, σ-N-methylarginine, and other similar amino acids and imino acids (e.g., 4-hydroxyproline). In the polypeptide notation used herein, the lefthand direction is the amino terminal direction and the right-hand direction is the carboxy-terminal direction, in accordance with standard usage and convention.

Similarly, unless specified otherwise, the lefthand end of single-stranded polynucleotide sequences is the 5' end; the lefthand direction of double-stranded polynucleotide sequences is referred to as the 5' direction. The direction of 5' to 3' addition of nascent RNA transcripts is referred to as the transcription direction; sequence regions on the DNA strand having the same sequence as the RNA and which are 5' to the 5' end of the RNA transcript are referred to as "upstream sequences"; sequence regions on the DNA strand having the same sequence as the RNA and which are 3' to the 3' end of the RNA transcript are referred to as "downstream sequences".

As applied to polypeptides, the term "substantial identity" means that two peptide sequences, when optimally aligned, such as by the programs GAP or BESTFIT using default gap weights, share at least 80 percent sequence identity, preferably at least 90 percent sequence identity, more preferably at least 95 percent sequence identity, and most preferably at least 99 percent sequence identity. Preferably, residue positions which are not identical differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, glutamic-aspartic, and asparagine-glutamine.

As discussed herein, minor variations in the amino acid sequences of antibodies or immunoglobulin molecules are contemplated as being encompassed by the antibodies described herein, provided that the variations in the amino acid sequence maintain at least 75%, more preferably at least 80%, 90%, 95%, and most preferably 99% homology to the antibodies or immunoglobulin molecules described herein. In particular, conservative amino acid replacements are contemplated. Conservative replacements are those that take place within a family of amino acids that are related in their side chains. Genetically encoded amino acids are generally divided into families: (1) acidic=aspartate, glutamate; (2) basic=lysine, arginine, histidine; (3) non-polar=alanine, valine, leucine, isoleucine, proline, phenylalanine, methionine, tryptophan; and (4) uncharged polar=glycine, asparagine, glutamine, cysteine, serine, threonine, tyrosine. More preferred families are: serine and threonine are an aliphatic-hydroxy family; asparagine and glutamine are an amide-containing family; alanine, valine, leucine and isoleucine are an aliphatic family; and phenylalanine, tryptophan, and tyrosine are an aromatic family. For example, it is reasonable to expect that an isolated replacement of a leucine with an isoleucine or valine, an aspartate with a glutamate, a threonine with a serine, or a similar replacement of an amino acid with a structurally related amino acid will not have a major effect on the binding or properties of the resulting molecule, especially if the replacement does not involve an amino acid within a framework site. Whether an amino acid change results in a functional peptide can readily be determined by assaying the specific activity of the polypeptide derivative. Assays are described in detail herein. Fragments or analogs of antibodies or immunoglobulin molecules can be readily prepared by those of ordinary skill in the art. Preferred amino- and carboxy-termini of fragments or analogs occur near boundaries of functional domains. Structural and functional domains can be identified by comparison of the nucleotide and/or amino acid sequence data to public or proprietary sequence databases. Preferably, computerized comparison methods are used to identify sequence motifs or predicted protein conformation domains that occur in other proteins of known structure and/or function. Methods to identify protein sequences that fold into a known three-dimensional structure are known. Bowie et al. Science 253:164 (1991). Thus, the foregoing examples demonstrate that those of skill in the art can recognize sequence motifs and structural conformations that may be used to define structural and functional domains in accordance with the antibodies described herein.

Preferred amino acid substitutions are those which: (1) reduce susceptibility to proteolysis, (2) reduce susceptibility to oxidation, (3) alter binding affinity for forming protein complexes, (4) alter binding affinities, and (4) confer or modify other physicochemical or functional properties of such analogs. Analogs can include various muteins of a sequence other than the naturally-occurring peptide sequence. For example, single or multiple amino acid substitutions (preferably conservative amino acid substitutions) may be made in the naturally-occurring sequence (preferably in the portion of the polypeptide outside the domain(s) forming intermolecular contacts. A conservative amino acid substitution should not substantially change the structural characteristics of the parent sequence (e.g., a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecutar Principles (Creighton, Ed., W. H. Freeman and Company, New York (1984)); Introduction to Protein Structure (C. Branden and J. Tooze, eds., Garland Publishing, New York, N.Y. (1991)); and Thornton et at. Nature 354:105 (1991), which are each incorporated herein by reference.

The term "polypeptide fragment" as used herein refers to a polypeptide that has an amino-terminal and/or carboxy-terminal deletion, but where the remaining amino acid sequence is identical to the corresponding positions in the naturally-occurring sequence deduced, for example, from a full-length cDNA sequence. Fragments typically are at least 5, 6, 8 or 10 amino acids long, preferably at least 14 amino acids long, more preferably at least 20 amino acids long, usually at least 50 amino acids long, and even more preferably at least 70 amino acids long. The term "analog" as used herein refers to polypeptides which are comprised of a segment of at least 25 amino acids that has substantial identity to a portion of a deduced amino acid sequence and which has at least one of the following properties: (1) specific binding to ANGPTL4, under suitable binding conditions, (2) ability to block appropriate ANGPTL4 binding, or (3) ability to inhibit ANGPTL4 expressing cell growth *in vitro* or *in vivo.* Typically, polypeptide analogs comprise a conservative amino acid substitution (or addition or deletion) with respect to the naturally-occurring sequence. Analogs typically are at least 20 amino acids long, preferably at least 50 amino acids long or longer, and can often be as long as a full-length naturally-occurring polypeptide.

Peptide analogs are commonly used in the pharmaceutical industry as non-peptide drugs with properties analogous to those of the template peptide. These types of non-peptide compound are termed "peptide mimetics" or "peptidomimetics". Fauchere, J. Adv. Drug Res. 15:29 (1986); Veber and Freidinger TINS p.392 (1985); and Evans et al. J. Med. Chem. 30:1229 (1987), which are incorporated herein by reference. Such compounds are often developed with the aid of computerized molecular modeling. Peptide mimetics that are structurally similar to therapeutically useful peptides may be used to produce an equivalent therapeutic or prophylactic effect. Generally, peptidomimetics are structurally similar to a paradigm polypeptide (i.e., a polypeptide that has a biochemical property or pharmacological activity), such as human antibody, but have one or more peptide linkages optionally replaced by a linkage selected from the group consisting of:-CH₂NH--, --CH₂S--, --CH₂-CH₂--, --CH=CH--(cis and trans), --COCH₂--, --CH(OH)CH₂--, and -CH₂SO--, by methods well known in the art. Systematic substitution of one or more amino acids of a consensus sequence with a D-amino acid of the same type (e.g., D-lysine in place of L-lysine) may be used to generate more stable peptides. In addition, constrained peptides comprising a consensus sequence or a substantially identical consensus sequence variation may be generated by methods known in the art (Rizo and Gierasch Ann. Rev. Biochem. 61:387 (1992), incorporated herein by reference); for example, by adding internal cysteine residues capable of forming intramolecular disulfide bridges which cyclize the peptide.

The terms "antibody" or "antibody peptide(s)" refer to an intact antibody, or a binding fragment thereof, that competes with the intact antibody for specific binding. Binding fragments are produced by recombinant DNA techniques, or by enzymatic or chemical cleavage of intact antibodies. Binding fragments include Fab, Fab', F(ab')2, Fv, and single-chain antibodies. An antibody other than a "bispecific" or "bifunctional" antibody is understood to have each of its binding sites identical. An antibody substantially inhibits adhesion of a receptor to a counterreceptor when an excess of antibody reduces the quantity of receptor bound to counterreceptor by at least about 20%, 40%, 60% or 80%, and more usually greater than about 85% (as measured in an in vitro competitive binding assay).

The term "epitope" includes any protein determinant capable of specific binding to an immunoglobulin or T-cell receptor. Epitopic determinants usually consist of chemically active surface groupings of molecules such as amino acids or sugar side chains and usually have specific three dimensional structural characteristics, as well as specific charge characteristics. An antibody is said to specifically bind an antigen when the dissociation constant is ≤ 1 µM, preferably ≤ 100 nM and most preferably ≤ 10 nM.

The term "agent" is used herein to denote a chemical compound, a mixture of chemical compounds, a biological macromolecule, or an extract made from biological materials.

The terms "active" or "activity" in regard to an ANGPTL4 polypeptide refer to a portion of ANGPTL4 polypeptide which has a biological or an immunological activity of a native ANGPTL4 polypeptide. "Biological" when used herein refers to a biological function that results from the activity of the native ANGPTL4 polypeptide. Thus, a biological activity can include angiogenesis, for example.

The term "mammal" when used herein refers to any animal that is considered a mammal. Preferably, the mammal is human.

Digestion of antibodies with the enzyme, papain, results in two identical antigen-binding fragments, known also as "Fab" fragments, and a "Fc" fragment, having no antigen-binding activity but having the ability to crystallize. Digestion of antibodies with the enzyme, pepsin, results in the a F(ab')₂ fragment in which the two arms of the antibody molecule remain linked and comprise two-antigen binding sites. The F(ab')₂ fragment has the ability to crosslink antigen.

"Fv" when used herein refers to the minimum fragment of an antibody that retains both antigen-recognition and antigen-binding sites.

"Fab" when used herein refers to a fragment of an antibody which comprises the constant domain of the light chain and the CH1 domain of the heavy chain.

"Liposome" when used herein refers to a small vesicle that may be useful for delivery of drugs that may include the ANGPTL24 polypeptide described herein or antibodies to such a ANGPTL4 polypeptide to a mammal.

"Label" or "labeled" as used herein refers to the addition of a detectable moiety to a polypeptide, for example, a radiolabel, fluorescent label, enzymatic label chemiluminescent label or a biotinyl group. Radioisotopes or radionuclides may include ³H, 14_{C}, ¹⁵N, ³⁵S, ⁹⁰Y, ⁹⁹Tc, ¹¹¹In, ¹²⁵I, ¹³¹I, fluorescent labels may include rhodamine, lanthanide phosphors or FITC and enzymatic labels may include horseradish peroxidase, β-galactosidase, luciferase, alkaline phosphatase.

The term "pharmaceutical agent or drug" as used herein refers to a chemical compound or composition capable of inducing a desired therapeutic effect when properly administered to a patient. Other chemistry terms herein are used according to conventional usage in the art, as exemplified by The McGraw-Hill Dictionary of Chemical Terms (Parker, S., Ed., McGraw-Hill, San Francisco (1985)), incorporated herein by reference).

The term "antineoplastic agent" is used herein to refer to agents that have the functional property of inhibiting a development or progression of a neoplasm in a human, particularly a malignant (cancerous) lesion, such as a carcinoma, sarcoma, lymphoma, or leukemia. Inhibition of metastasis is frequently a property of antineoplastic agents.

As used herein, "substantially pure" means an object species is the predominant species present (i.e., on a molar basis it is more abundant than any other individual species in the composition), and preferably a substantially purified fraction is a composition wherein the object species comprises at least about 50 percent (on a molar basis) of all macromolecular species present. Generally, a substantially pure composition will comprise more than about 80 percent of all macromolecular species present in the composition, more preferably more than about 85%, 90%, 95%, and 99%. Most preferably, the object species is purified to essential homogeneity (contaminant species cannot be detected in the composition by conventional detection methods) wherein the composition consists essentially of a single macromolecular species.

The term "patient" includes human and veterinary subjects.

### Antibody Structure

The basic antibody structural unit is known to comprise a tetramer. Each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Human light chains are classified as κ and λ light chains. Heavy chains are classified as mu, delta, gamma, alpha, or epsilon, and define the antibody's isotype as IgM, IgD, IgA, and IgE, respectively. Within light and heavy chains, the variable and constant regions are joined by a "J" region of about 12 or more amino acids, with the heavy chain also including a "D" region of about 10 more amino acids. *See generally,* Fundamental Immunology Ch. 7 (Paul, W., ed., 2nd ed. Raven Press, N.Y. (1989)) (incorporated by reference in its entirety for all purposes). The variable regions of each light/heavy chain pair form the antibody binding site.

Thus, an intact antibody has two binding sites. Except in bifunctional or bispecific antibodies, the two binding sites are the same.

The chains all exhibit the same general structure of relatively conserved framework regions (FR) joined by three hyper variable regions, also called complementarity determining regions or CDRs. The CDRs from the two chains of each pair are aligned by the framework regions, enabling binding to a specific epitope. From N-terminal to C-terminal, both light and heavy chains comprise the domains FR1, CDR1, FR2, CDR2, FR3, CDR3 and FR4. The assignment of amino acids to each domain is in accordance with the definitions of Kabat Sequences of Proteins of Immunological Interest (National Institutes of Health, Bethesda, Md. (1987 and 1991)), or Chothia & Lesk J. Mol. Biol. 196: 901-917 (1987); Chothia et al. Nature 342: 878-883 (1989).

A bispecific or bifunctional antibody is an artificial hybrid antibody having two different heavy/light chain pairs and two different binding sites. Bispecific antibodies can be produced by a variety of methods including fusion of hybridomas or linking of Fab' fragments. *See, e.g.,* Songsivilai & Lachmann Clin. Exp. Immunol. 79: 315-321 (1990), Kostelny et al. J. Immunol. 148:1547-1553 (1992). Production of bispecific antibodies can be a relatively labor intensive process compared with production of conventional antibodies and yields and degree of purity are generally lower for bispecific antibodies. Bispecific antibodies do not exist in the form of fragments having a single binding site (e.g., Fab, Fab', and Fv).

### Human Antibodies and Humanization of Antibodies

Human antibodies avoid some of the problems associated with antibodies that possess murine or rat variable and/or constant regions. The presence of such murine or rat derived proteins can lead to the rapid clearance of the antibodies or can lead to the generation of an immune response against the antibody by a patient. In order to avoid the utilization of murine or rat derived antibodies, fully human antibodies can be generated through the introduction of human antibody function into a rodent so that the rodent produces fully human antibodies.

### Human Antibodies

One method for generating fully human antibodies is through the use of XenoMouse® trains of mice which have been engineered to contain 245 kb and 190 kb-sized germline configuration fragments of the human heavy chain locus and κ light chain locus. *See* Green et al. Nature Genetics 7: 13-21 (1994). The XenoMouse® trains are available from Abgenix, Inc. (Fremont, CA).

The production of the XenoMouse® is further discussed and delineated in U.S. Patent Application Serial Nos. 07/466,008, filed January 12, 1990, 07/610,515, filed November 8, 1990, 07/919,297, filed July 24, 1992, 07/922,649, filed July 30, 1992, filed 08/031,801, filed March 15,1993, 08/112,848, filed August 27, 1993, 08/234,145, filed April 28, 1994, 08/376,279, filed January 20, 1995, 08/430, 938, April 27, 1995, 08/464,584, filed June 5, 1995, 08/464,582, filed June 5, 1995, 08/463,191, filed June 5, 1995, 08/462,837, filed June 5, 1995, 08/486,853, filed June 5, 1995, 08/486,857, filed June 5, 1995, 08/486,859, filed June 5, 1995, 08/462,513, filed June 5, 1995, 08/724,752, filed October 2, 1996, and 08/759,620, filed December 3, 1996 and U.S. Patent Nos. 6,162,963, 6,150,584, 6,114,598, 6,075,181, and 5,939,598 and Japanese Patent Nos. 3 068 180 B2, 3 068 506 B2, and 3 068 507 B2. *See also* Mendez et al. Nature Genetics 15:146-156 (1997) and Green and Jakobovits J. Exp. Med. 188:483-495 (1998). *See also* European Patent No., EP 0 463 151 B1, grant published June 12, 1996, International Patent Application No., WO 94/02602, published February 3, 1994, International Patent Application No., WO 96/34096, published October 31, 1996, WO 98/24893, published June 11, 1998, WO 00/76310, published December 21, 2000. The disclosures of each of the above-cited patents, applications, and references are hereby incorporated by reference in their entirety.

In an alternative approach, others, including GenPharm International, Inc., have utilized a "minilocus" approach. In the minilocus approach, an exogenous Ig locus is mimicked through the inclusion of pieces (individual genes) from the Ig locus. Thus, one or more V_{H} genes, one or more D_{H} genes, one or more J_{H} genes, a mu constant region, and a second constant region (preferably a gamma constant region) are formed into a construct for insertion into an animal. This approach is described in U.S. Patent No. 5,545,807 to Surani et al. and U.S. Patent Nos. 5,545,806, 5,625,825, 5,625,126, 5,633,425, 5,661,016, 5,770,429, 5,789,650, 5,814,318, 5,877,397, 5,874,299, and 6,255,458 each to Lonberg and Kay, U.S. Patent No. 5,591,669 and 6,023.010 to Krimpenfort and Berns, U.S. Patent Nos. 5,612,205, 5,721,367, and 5,789,215 to Berns et al., and U.S. Patent No. 5,643,763 to Choi and Dunn, and GenPharm International U.S. Patent Application Serial Nos. 07/574,748, filed August 29, 1990, 07/575,962, filed August 31, 1990, 07/810,279, filed December 17, 1991, 07/853,408, filed March 18, 1992, 07/904,068, filed June 23, 1992, 07/990,860, filed December 16, 1992, 081053,131, filed April 26, 1993, 08/096,762, filed July 22, 1993, 08/155,301, filed November 18, 1993, 08/161,739, filed December 3, 1993, 08/165,699, filed December 10, 1993, 08/209,741, filed March 9, 1994, the disclosures of which are hereby incorporated by reference. *See also* European Patent No. 0 546 073 B1, International Patent Application Nos. WO 92/03918, WO 92/22645, WO 92/22647, WO 92/22670, WO 93/12227, WO 94/00569, WO 94/25585, WO 96/14436, WO 97/13852, and WO 98/24884 and U.S. Patent No. 5,981,175, the disclosures of which are hereby incorporated by reference in their entirety. *See further* Taylor et al., 1992, Chen et al., 1993, Tuaillon et al., 1993, Choi et al., 1993, Lonberg et al., (1994), Taylor et al., (1994), and Tuaillon et al., (1995), Fishwild et al., (1996), the disclosures of which are hereby incorporated by reference in their entirety.

Kirin has also demonstrated the generation of human antibodies from mice in which, through microcell fusion, large pieces of chromosomes, or entire chromosomes, have been introduced. *See* European Patent Application Nos. 773 288 and 843 961, the disclosures of which are hereby incorporated by reference.

Human anti-mouse antibody (HAMA) responses have led the industry to prepare chimeric or otherwise humanized antibodies. While chimeric antibodies have a human constant region and a murine variable region, it is expected that certain human anti-chimeric antibody (HACA) responses will be observed, particularly in chronic or multi-dose utilizations of the antibody. Thus, it would be desirable to provide fully human antibodies against ANGPTL4 in order to vitiate concerns and/or effects of HAMA or HACA response.

### Additional Criteria for Antibody Therapeutics

As discussed herein, the function of the ANGPTL4 antibody appears important to at least a portion of its mode of operation. By function, is meant, by way of example, the activity of the ANGPTL4 antibody in operation with ANGPTL4. Accordingly, in certain respects, it may be desirable in connection with the generation of antibodies as therapeutic candidates against ANGPTL4 that the antibodies be capable of fixing complement and participating in complement dependent cytotoxicity "CDC." There are a number of isotypes of antibodies that are capable of the same, including, without limitation, the following: murine IgM, murine IgG2a, murine IgG2b, murine IgG3, human IgM, human IgG1, and human IgG3. It will be appreciated that antibodies that are generated need not initially possess such an isotype but, rather, the antibody as generated can possess any isotype and the antibody can be isotype switched thereafter using conventional techniques that are well known in the art. Such techniques include the use of direct recombinant techniques (*see e*.*g*., U.S. Patent No. 4,816,397), cell-cell fusion techniques (*see e.g.,* U.S. Patent Nos. 5,916,771 and 6,207,418), among others.

In the cell-cell fusion technique, a myeloma or other cell line is prepared that possesses a heavy chain with any desired isotype and another myeloma or other cell line is prepared that possesses the light chain. Such cells can, thereafter, be fused and a cell line expressing an intact antibody can be isolated.

By way of example, ANGPTL4 antibodies discussed herein are human anti-ANGPTL4 IgG2 antibodies. If such antibodies possessed desired binding to the ANGPTL4 molecule, they could be readily isotype switched to generate a human IgM, human IgGl, or human IgG3 isotype, while still possessing the same variable region (which defines the antibody's specificity and some of its affinity). Such molecule would then be capable of fixing complement and participating in CDC.

Accordingly, as antibody candidates are generated that meet desired "structural" attributes as discussed above, they can generally be provided with at least certain of the desired "functional" attributes through isotype switching.

### Design and Generation of Other Therapeutics

Based on the activity of the antibodies that are produced and characterized herein with respect to ANGPTL4, the design of other therapeutic modalities beyond antibody moieties is facilitated. Such modalities include, without limitation, advanced antibody therapeutics, such as bispecific antibodies, immunotoxins, and radiolabeled therapeutics, generation of peptide therapeutics, gene therapies, particularly intrabodies, antisense therapeutics, and small molecules.

In connection with the generation of advanced antibody therapeutics, where complement fixation is a desirable attribute, it may be possible to sidestep the dependence on complement for cell killing through the use of bispecifics, immunotoxins, or radiolabels, for example.

For example, in connection with bispecific antibodies, bispecific antibodies can be generated that comprise (i) two antibodies one with a specificity to ANGPTL4 and another to a second molecule that are conjugated together, (ii) a single antibody that has one chain specific to ANGPTL4 and a second chain specific to a second molecule, or (iii) a single chain antibody that has specificity to ANGPTL4 and the other molecule. Such bispecific antibodies can be generated using techniques that are well known; for example, in connection with (i) and (ii) *see e.g.,* Fanger et al. Immunol Methods 4:72-81 (1994) and Wright and Harris, *supra.* and in connection with (iii) *see e.g.,* Traunecker et al. Int. J. Cancer (Suppl.) 7:51-52 (1992). In each case, the second specificity can be made to the heavy chain activation receptors, including, without limitation, CD16 or CD64 (*see e.g.,* Deo et al. 18:127 (1997)) or CD89 (*see e.g.,* Valerius et al. Blood 90:4485-4492 (1997)). Bispecific antibodies prepared in accordance with the foregoing would be likely to kill cells expressing ANGPTL4.

In connection with immunotoxins, antibodies can be modified to act as immunotoxins utilizing techniques that are well known in the art. *See e.g.,* Vitetta Immunol Today 14:252 (1993). *See also* U.S. Patent No. 5,194,594. In connection with the preparation of radiolabeled antibodies, such modified antibodies can also be readily prepared utilizing techniques that are well known in the art. *See e.g.,* Junghans et al. in Cancer Chemotherapy and Biotherapy 655-686 (2d edition, Chafner and Longo, eds., Lippincott Raven (1996)). *See also* U.S. Patent Nos. 4,681,581, 4,735,210, 5,101,827, 5,102,990 (RE 35,500), 5,648,471, and 5,697,902. Each of immunotoxins and radiolabeled molecules would be likely to kill cells expressing ANGPTL4.

### Therapeutic Administration and Formulations

It will be appreciated that administration of therapeutic entities in accordance with the methods described herein will be administered with suitable carriers, excipients, and other agents that are incorporated into formulations to provide improved transfer, delivery, tolerance, and the like. A multitude of appropriate formulations can be found in the formulary known to all pharmaceutical chemists: Remington's Pharmaceutical Sciences (18th ed, Mack Publishing Company, Easton, PA (1990)), particularly Chapter 87 by Block, Lawrence, therein. These formulations include, for example, powders, pastes, ointments, jellies, waxes, oils, lipids, lipid (cationic or anionic) containing vesicles (such as Lipofectin™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. Any of the foregoing mixtures may be appropriate in treatments and therapies in accordance with the antibodies and methods described herein, provided that the active ingredient in the formulation is not inactivated by the formulation and the formulation is physiologically compatible and tolerable with the route of administration. *See also* Baldrick P. "Pharmaceutical excipient development: the need for preclinical guidance." Regul. Toxicol. Pharmacol. 32(2):210-8 (2000), Wang W. "Lyophilization and development of solid protein pharmaceuticals." Int. J. Pharm. 203(1-2):1-60 (2000), Charman WN "Lipids, lipophilic drugs, and oral drug delivery-some emerging concepts." J Pharm Sci .89(8):967-78 (2000), Powell et al. "Compendium of excipients for parenteral formulations" PDA J Pharm Sci Technol. 52:238-311 (1998) and the citations therein for additional information related to formulations, excipients and carriers well known to pharmaceutical chemists.

### Preparation of Antibodies

Antibodies described herein were prepared through the utilization of the XenoMouse® technology, as described below. Such mice, then, are capable of producing human immunoglobulin molecules and antibodies and are deficient in the production of murine immunoglobulin molecules and antibodies. Technologies utilized for achieving the same are disclosed in the patents, applications, and references disclosed in the background section, herein. In particular, however, a preferred embodiment of transgenic production of mice and antibodies therefrom is disclosed in U.S. Patent Application Serial No. 08/759,620, filed December 3, 1996 and International Patent Application Nos. WO 98/24893, published June 11, 1998 and WO 00/76310, published December 21, 2000, the disclosures of which are hereby incorporated by reference. *See also* Mendez et al. Nature Genetics 15:146-156 (1997), the disclosure of which is hereby incorporated by reference.

Through use of such technology, fully human monoclonal antibodies to a variety of antigens have been produced. Essentially, XenoMouse® lines of mice are immunized with an antigen of interest, lymphatic cells (such as B-cells) are recovered from the mice that expressed antibodies, the recovered cell lines are fused with a myeloid-type cell line to prepare immortal hybridoma cell lines. These hybridoma cell lines are screened and selected to identify hybridoma cell lines that produced antibodies specific to the antigen of interest. Herein, is described the production of multiple hybridoma cell lines that produce antibodies specific to ANGPTL4. Further, a characterization of the antibodies produced by such cell lines are provided, including nucleotide and amino acid sequence analyses of the heavy and light chains of such antibodies.

Alternatively, instead of being fused to myeloma cells to generate hybridomas, the recovered cells, isolated from immunized XenoMouse® lines of mice, are screened further for reactivity against the initial antigen, preferably ANGPTL4 protein. Such screening includes ELISA with ANGPTL4-His protein, a competition assay with known antibodies that bind the antigen of interest, and *in vitro* binding to transiently transfected CHO cells expressing full length ANGPTL4. Single B cells secreting antibodies of interest are then isolated using a ANGPTL4-specific hemolytic plaque assay (Babcook et al., Proc. Natl. Acad. Sci. USA, i93:7843-7848 (1996)). Cells targeted for lysis are preferably sheep red blood cells (SRBCs) coated with the ANGPTL4 antigen. In the presence of a B cell culture secreting the immunoglobulin of interest and complement, the formation of a plaque indicates specific ANGPTL4-mediated lysis of the target cells. The single antigen-specific plasma cell in the center of the plaque can be isolated and the genetic information that encodes the specificity of the antibody is isolated from the single plasma cell. Using reverse-transcriptase PCR, the DNA encoding the variable region of the antibody secreted can be cloned. Such cloned DNA can then be further inserted into a suitable expression vector, preferably a vector cassette such as a pcDNA, more preferably such a pcDNA vector containing the constant domains of immunoglobulin heavy and light chain. The generated vector can then be transfected into host cells, preferably CHO cells, and cultured in conventional nutrient media modified as appropriate for inducing promoters, selecting transformants, or amplifying the genes encoding the desired sequences. Herein, is described the isolation of multiple single plasma cells that produce antibodies specific to ANGPTL4. Further, the genetic material that encodes the specificity of the anti-ANGPTL4 antibody is isolated, and introduced into a suitable expression vector which is then transfected into host cells.

In general, antibodies produced by the above-mentioned cell lines possessed fully human IgG2 heavy chains with human kappa light chains. The antibodies possessed high affinities, typically possessing Kd's of from about 10⁻⁶ through about 10⁻¹¹ M, when measured by either solid phase and solution phase.

As will be appreciated, antibodies as described herein can be expressed in cell lines other than hybridoma cell lines. Sequences encoding particular antibodies can be used for transformation of a suitable mammalian host cell. Transformation can be by any known method for introducing polynucleotides into a host cell, including, for example packaging the polynucleotide in a virus (or into a viral vector) and transducing a host cell with the virus (or vector) or by transfection procedures known in the art, as exemplified by U.S. Patent Nos. 4,399,216, 4,912,040, 4,740,461, and 4,959,455 (which patents are hereby incorporated herein by reference). The transformation procedure used depends upon the host to be transformed. Methods for introduction of heterologous polynucleotides into mammalian cells are well known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are well known in the art and include many immortalized cell lines available from the American Type Culture Collection (ATCC), including but not limited to Chinese hamster ovary (CHO) cells, HeLa cells, baby hamster kidney (BHK) cells, monkey kidney cells (COS), human hepatocellular carcinoma cells (e.g., Hep G2), and a number of other cell lines. Cell lines of particular preference are selected through determining which cell lines have high expression levels and produce antibodies with constitutive ANGPTL4 binding properties.

Antibodies as described herein are capable of binding to ANGPTL4. The antibodies described herein are also useful in the detection of ANGPTL4 in patient samples and accordingly are useful as diagnostics as described herein. In addition, based on the ability to neutralize lipoprotein lipase inhibition by ANGPTL4, it is expected that the antibodies herein will have therapeutic effect in the treatment of hyperlipidemia.

The following examples, including the experiments conducted and results achieved are provided for illustrative purposes only and are not to be construed as limiting upon the teachings herein.

### EXAMPLE 1

### ANGPTL4 antigen preparation

Recombinant ANGPTL4 proteins were prepared according to the following protocol. ANGPTL4 cDNA was cloned by Reverse Transcriptase-PCR (RT-PCR) with primers based on the sequence of ANGPTL4. *See* Yoon et al., Molec. Cell. Biol. 20: 5343-5349 (2000). The cDNA was amplified through PCR, and cloned into a plasmid (Plasmid #1614). The plasmid was digested and ligated into a vector (pEE14.4/Sec), transfected into HEK 293 cells, and expressed.

The resulting ANGPTL4 immunogen was a 34 KD V5-his tagged secreted product. Metal chelate affinity chromatography was used to purify the secreted proteins. Samples were washed with 20mM, 50mM, and 100mM imidazole and eluted with 500mM imidazole. This was followed by cation exchange chromatography, where samples were eluted against a 0-1 M NaCl gradient. Dialysis took place at 4°C through a 3,500 MW cutoff membrane against 20 mM Tris HCl, pH 7.4 and 150 mM NaCl.

A typical SDS PAGE and Western Blot was run to characterize the purity and quantity of synthesized ANGPTL4 antigen. The results of these assays are provided in Table 1.

**Table 1:**

| **ANGPTL4 Purity and Quantity** | |
|---|---|
| **GEL TYPE** | 4-20% Tris-Glycine 1.0mm |
| **STAINING** | Coomassie |
| **ESTIMATED PURITY** | >75% |
| **ESTIMATED SIZE** | 36 kDa |
| **PROTEIN CONCENTRATION:** | |
| **By Bradford Method** | 0.55 mg/mL |
| **By A₂₈₀ Absorbance** | ND |
| **ENDOTOXIN LEVEL** | 50.6 EU/mg by LAL |
| **WESTERN BLOT & ANALYSIS DATA** | (+) Anti-V5 HRP, 1:500 |
| **TOTAL PROTEIN BATCH QUANTITY** | 4.3 mg |
| **PROTEIN BUFFER COMPOSITION** | 20mM Tris-HCL, pH 7.4/ 150mM NaCl |
| **STERILE FILTERED** | Yes |

### EXAMPLE 2

### Immunization and selection of animals for harvesting by ELISA.

This example illustrates the preparation of fully human monoclonal antibodies which specifically bind to ANGPTL4. Monoclonal antibodies against ANGPTL4 were developed by immunizing XenoMouse® mice (XenoMouse® strains XMG2, XMG4 (3C-1 strain), Abgenix, Inc. Fremont, CA) according to the schedule shown in Table 2. The initial immunization was with 10 µg antigen admixed 1:1 v/v with TiterMax Gold (CytRX Corporation, Los Angeles, CA). Subsequent boosts were made with 10 µg antigen admixed 1:1 v/v with 100 µg alum gel in pyrogen-free D-PBS and sometimes with 50% TiterMax Gold, followed by injections with 10 µg antigen admixed 1:1 v/v with 10 µg ANGPTL4 antigen in alum gel, and then a final boost of 10 µg antigen in PBS. In particular, each mouse was immunized in the footpad by subcutaneous injection. The animals were bled to obtain sera for harvest selection as described below in Table 2.

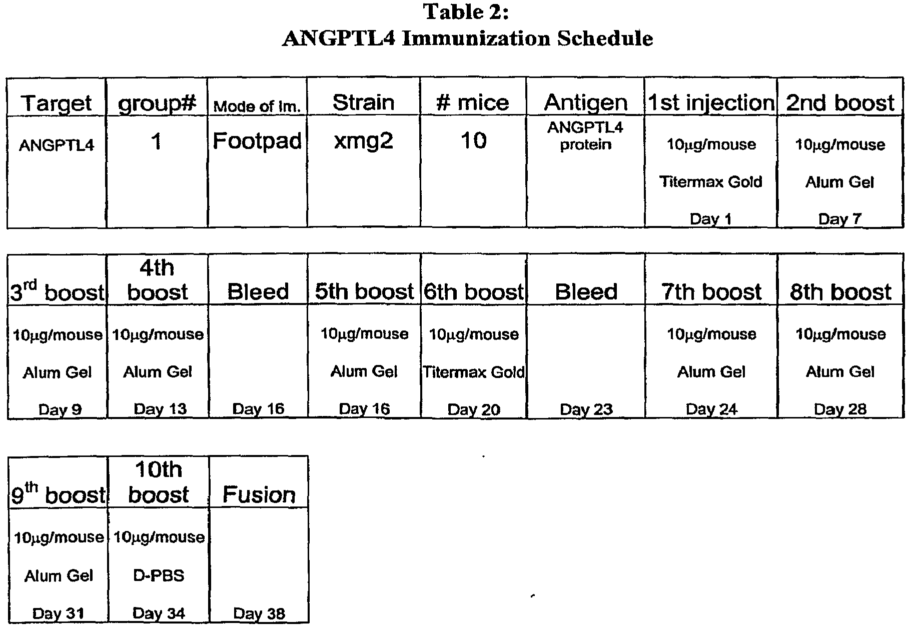

Antibody titers were determined by indirect ELISA. The titer value is the reciprocal of the greatest dilution of sera with an OD reading two-fold that of background. Briefly, ANGPTL4 (1 µg/mL) was coated onto Costar Labcoat Universal Binding Polystyrene 96 well plates overnight at 4°C. The solution containing unbound ANGPTL4 was removed and the plates were treated with UV light (365nm) for 4 minutes (4000 microjoules). The plates were washed five times with dH₂O

XenoMouse® sera from the ANGPTL4 immunized animals, or naïve XenoMouse® animals, were titrated in 2% milk/PBS at 1:2 dilutions in duplicate from a 1:100 initial dilution. The last well was left blank as a control. The plates were washed five times with dH₂O. A goat anti-human IgG Fc-specific HRP-conjugated antibody was added at a final concentration of 1 µg/mL for 1 hour at room temperature. The plates were washed five times with dH₂O. The plates were developed with the addition of TMB for 30 minutes and the ELISA was stopped by the addition of 1 M phosphoric acid. The specific titer of individual XenoMouse® animals was determined from the optical density at 450 nm and are shown in Table 3. The titer represents the reciprocal dilution of the serum and therefore the higher the number the greater the humoral immune response to ANGPTL4. Lymph nodes from all immunized XenoMouse® animals were harvested for fusion.

**Table 3:**

| **ANGPTL4 Serum Titers** | | |
|---|---|---|
| **Group 1, fp, xmg2, 10 mice, +10µg/mL V5 Peptide** | | |
| | | |
| | **Bleed after 4 inj.** | **Bleed after 6 inj.** |
| | | |
| Mouse ID | Reactivity to ANGPTL4 | |
| | Titers via hlgG | |
| O480-1 | 62,000 | 119,000 |
| O480-2 | 29,000 | 103,000 |
| O480-3 | 33,000 | 238,000 |
| O480-4 | 35,000 | 204,000 |
| O480-5 | 22,000 | 173,000 |
| O480-6 | 15,000 | 138,000 |
| O480-7 | 57,000 | 196,000 |
| O480-8 | 7,300 | 76,000 |
| O480-9 | 11,000 | 70,000 |
| O480-10 | 17,000 | 101,000 |
| NC | 200 | 25 |

### EXAMPLE 3

### Recovery of lymphocytes. B-cell isolations, fusions and generation of hybridomas

The following protocol was used to generate hybridomas that produce antibodies directed against ANGPTL4. Immunized mice prepared according to Example 2 were sacrificed by cervical dislocation, and the lymph nodes harvested and pooled from each cohort. The lymphoid cells were dissociated by grinding in DMEM to release the cells from the tissues and the cells were suspended in DMEM. The cells were counted, and 0.9 mL DMEM per 100 million lymphocytes added to the cell pellet to resuspend the cells gently but completely. Using 100 µL. of CD90+ magnetic beads per 100 million cells, the cells were labeled by incubating the cells with the magnetic beads at 4°C for 15 minutes. The magnetically labeled cell suspension containing up to 108 positive cells (or up to 2x109 total cells) was loaded onto a LS+ column and the column washed with DMEM. The total effluent was collected as the CD90-negative fraction (most of these cells are B cells).

P3 myeloma cells and B cell-enriched lymph node cells were combined in a ratio of 1:1 (myeloma:lymph nodes) into a 50 mL conical tube in DMEM. The combined cells were centrifuged at 800xg (2000 rpm) for 5-7 minutes and the supernatant immediately removed from the resulting pellet. Two to four mL of Pronase solution (CalBiochem, Cat. #53702; 0.5 mg/mL in PBS) were added to the cells to resuspend the cell pellet gently. The enzyme treatment was allowed to proceed for no more than two minutes and the reaction stopped by the addition of 3-5 mL of FBS. Enough ECF solution was added to bring the total volume to 40 mL and the mixture was centrifuged at 800xg (2000 rpm) for 5-7 minutes. The supernatant was removed and the cell pellet gently resuspended with a small volume of ECF solution, followed by enough ECF solution to make a total volume of 40 mL. The cells were mixed well and counted, then centrifuged at 800xg (2000 rpm) for 5-7 minutes. The supernatant was removed and the cells resuspended in a small volume of ECF solution. Enough additional ECF solution was added to adjust the concentration to 2 x 10⁶ cells/mL.

The cells were then placed in an Electro-Cell-Fusion (ECF) generator (Model ECM2001, Genetronic, Inc., San Diego, CA) and fused according to the manufacturer's instructions. After ECF, the cell suspensions were carefully removed from the fusion chamber under sterile conditions and transferred into a sterile tube containing the same volume of Hybridoma Medium in DMEM. The cells were incubated for 15-30 minutes at 37°C, and then centrifuged at 400xg (1000 rpm) for five minutes. The cells were gently resuspended in a small volume of ½ HA medium (1 bottle of 50X HA from Sigma, Cat. #A9666 and 1 liter of Hybridoma Medium) and the volume adjusted appropriately with more ½ HA medium (based on 5x10⁶ B cells per 96-well plate and 200 µl per well). The cells were mixed well and pipetted into 96-well plates and allowed to grow. On day 7 or 10, one-half the medium was removed, and the cells were re-fed with ½ HA medium.

### EXAMPLE 4

### Selection of candidate antibodies for ELISA

The following protocol was followed to select candidate antibodies for ELISA. After 14 days of culture, the hybridoma supernatants that were prepared in Example 3 were screened for ANGPTL4-specific monoclonal antibodies using ELISA. The ELISA plates (Fisher, Cat. No. 12-565-136) were coated with 50 µl/well of ANGPTL4 (2 µg/mL) in Coating Buffer (0.1 M Carbonate Buffer, pH 9.6, NaHCO3 8.4 g/L), then incubated at 4°C overnight. After incubation, the plates were washed with Washing Buffer (0.05% Tween 20 in PBS) three times. 200 µl/well Blocking Buffer (0.5% BSA, 0.1% Tween 20, 0.01% Thimerosal in 1x PBS) were added and the plates incubated at room temperature for 1 hour.

After incubation, the plates were washed with Washing Buffer three times. 50 µl/well of hybridoma supernatants, and positive and negative controls were added and the plates incubated at room temperature for two hours. After incubation, the plates were washed three times with Washing Buffer. 100 µl/well of detection antibody goat anti-huIgGfc-HRP (Caltag, Cat. #H10507), goat anti-hIgκ-HRP (Southern Biotechnology, Cat. # 2060-05), and goat anti-hIgλ, (Southern Biotechnology, Cat. # 2070-05) were added and the plates incubated at room temperature for one hour. In the secondary screen, three sets of samples (positives in first screening) were screened, one set for hIgG detection, one set for hκ detection and one set for hλ. detection. After incubation, the plates were washed three times with Washing Buffer. 100 µl/well of TMB (BioFX Lab. Cat. #TMSK-0100-01) were added and the plates were allowed to develop for about 10 minutes (until negative control wells barely started to show color). Then 50 µL/well stop solution (TMB Stop Solution (BioFX Lab. Cat. #STPR-0100-01) was added and the plates were read on an ELISA plate reader at wavelength 450 nm.

Positive hits from the ELISA assay were verified by FACS analysis, using the following procedure. For suspension cells, 50 µL of culture was mixed with an equal volume of 0.4% trypan blue. 10 µL of the mixture was then counted in a hemacytometer. For each experiment, 0.2-lx 10⁶ cells were used. Cells were transferred and filtered through a 40 µm cell strainer on top of a 15 mL Falcon tube, followed by centrifugation at 1200 rpm (Beckman Coulter, Allegra™ 6, swing bucket) for 5 minutes.

For adherent cells, 5 mL EDTA was added and the cultures were incubated at 37°C for 3-5 minutes to detach the cells. 0.8 mL of 2% FBS PBS was added to dilute the EDTA. 50 jim of culture was used for counting cell numbers. Cells were then transferred to a Falcon tube and centrifuged as described earlier.

After discarding the supernatant, cells were washed with FACS buffer (PBS containing 2% FBS). The cell pellet was then resuspended in FACS buffer resulting in a final concentration of 2 x 10⁷ cells/mL. 50 µL of the cell suspension was aliquoted into each well of a 96 well microtiter plate. Diluted antibody (∼0.5 µg/million cells/well in 100 µL 2% FBS PBS) was added, and samples were incubated at 4°C for 30 minutes in the dark. Cells were then centrifuged at 1200 rpm for 5 minutes and the supernatant was discarded. Following two washes with FACS buffer, the cells were centrifuged. 100 µL of secondary detection antibody was added, and samples were mixed. Samples were then incubated, washed, and centrifuged as described above. After resuspension in 0.5- 1 mL FACS buffer or 1% paraformaldehyde, samples were analyzed by flow. Results are summarized in Tables 4 and 5. As Tables 4 and 5 indicate, all antibodies sampled had positive ELISA binding results.

**Table 4:**

| **Summary of ELISA Analyses of Antibody** | | | | | | | |
|---|---|---|---|---|---|---|---|
| **Group # 1** | | **Fusion #1** | | | | | |
| **ID** | **Line/Clone** | **Freeze/Exhaust** | **Volume** | **Concentration** | **Specificity** | | |
| | **(L) or (C)** | **sups (F) or (E)** | **(mL)** | **hIgG/K(µg/mL)** | **Isotype** | **ELISA 1 hIgG** | **ELISA 2 hK** |
| 1.1.1 | C | E | 5.5 | 38.66 | xmg2 | Positive | Positive |
| 1.1.2 | C | E | 5.5 | 48.00 | xmg2 | Positive | Positive |
| 1.1.3 | C | E | 5.5 | 33.33 | xmg2 | Positive | Positive |
| 1.2.1 | C | E | 5.5 | 375.33 | xmg2 | Positive | Positive |
| 1.2.2 | C | E | 5.5 | 301.33 | xmg2 | Positive | Positive |
| 1.2.3 | C | E | 2 | 375.33 | xmg2 | Positive | Positive |
| 1.3.1 | C | E | 9.5 | 91.47 | xmg2 | Positive | Positive |
| 1.3.2 | C | E | 9 | 48.33 | xmg2 | Positive | Positive |
| 1.3.3 | C | E | 10 | 46.35 | amp2 | Positive | Positive |
| 1.4.1 | C | E | 4 | 346.7 | xmg2 | Positive | Positive |
| 1.4.2 | C | E | 5 | 39.8 | xmg2 | Positive | Positive |
| 1.4.3 | C | E | 9 | 43.61 | xmg2 | Positive | Positive |
| 1.5.1 | C | E | 10 | 75.09 | xmg2 | Positive | Positive |
| 1.5.2 | C | E | 5.5 | 107.64 | xmg2 | Positive | Positive |
| 1.5.3 | C | E | 9.5 | 90.26 | xmg2 | Positive | Positive |
| 1.6.1 | C | E | 8 | 69.53 | xmg2 | Positive | Positive |
| 1.6.2 | C | E | 8 | 68.47 | xmg2 | Positive | Positive |
| 1.6.3 | C | E | 8.5 | 49.18 | xmg2 | Positive | Positive |
| 1.7.1 | C | E | 6 | 37.2 | xmg2 | Positive | Positive |
| 1.7.2 | C | E | 4.5 | 306.3 | xmg2 | Positive | Positive |
| 1.7.3 | C | E | 6.5 | 401.26 | xmg2 | Positive | Positive |
| 1.8.1 | C | E | 10.5 | 9.03 | xmg2 | Positive | Positive |
| 1.8.2 | C | E | 5 | 18.31 | xmg2 | Positive | Positive |
| 1.8.3 | C | E | 5 | 8.46 | xmg2 | Positive | Positive |
| 1.9.1 | C | E | 5.5 | 88.08 | xmg2 | Positive | Positive |
| 1.9.2 | C | E | 7.5 | 21.95 | xmg2 | Positive | Positive |
| 1.9.3 | C | E | 10 | 15.10 | xmg2 | Positive | Positive |
| 1.10.1 | C | E | 9 | 0.64 | xmg2 | Positive | Positive |
| 1.10.2 | C | E | 3.5 | 103.91 | xmg2 | Positive | Positive |
| 1.10.3 | C | E | 3.5 | 0.587 | xmg2 | Positive | Positive |
| 1.11.1 | C | E | 2 | 104.25 | xmg2 | Positive | Positive |
| 1.11.2 | C | E | 9.5 | 91.46 | xmg2 | Positive | Positive |
| 1.11.3 | C | E | 7 | 78.89 | xmg2 | Positive | Positive |

**Table 5:**

| **Summary of ELISA Analyses of Antibody** | | | | | | | |
|---|---|---|---|---|---|---|---|
| **Group #1** | **Fusion #2** | | | | | | |
| **ID** | **Line/Clone** | **Freeze/Exhaust** | **Volume** | **Concentration** | **Specificity** | | |
| | **(L) or (C)** | **sups (F) or (E)** | **(mL)** | **hIgG/K(µg/mL)** | **Isotype** | **ELISA 1 hIgG** | **ELISA 2 hK** |
| 2.2.1 | C | E | 3.5 | 28.95 | xmg2 | Positive | Positive |
| 2.2.2 | C | E | 3.5 | 28.42 | xmg2 | Positive | Positive |
| 2.2.3 | C | E | 4 | 23.55 | xmg2 | Positive | Positive |
| 2.3.1 | C | E | 11 | 16.12 | xmg2 | Positive | Positive |
| 2.3.2 | C | E | 10.5 | 18.82 | xmg2 | Positive | Positive |
| 2.3.3 | C | E | 7 | 40.77 | xmg2 | Positive | Positive |
| 2.4.1 | C | E | 5 | 23.8 | xmg2 | Positive | Positive |
| 2.4.2 | C | E | 10.5 | 12.15 | xmg2 | Positive | Positive |
| 2.4.3 | C | E | 11 | 9.12 | xmg2 | Positive | Positive |
| 2.5.1 | C | E | 7.5 | 57.39 | xmg2 | Positive | Positive |
| 2.5.2 | C | E | 7.5 | 47.77 | xmg2 | Positive | Positive |
| 2.5.3 | C | E | 5.5 | 59.0 | xmg2 | Positive | Positive |
| 2.6.1 | C | E | 11 | 28.00 | xmg2 | Positive | Positive |
| 2.6.2 | C | E | 9 | 44.00 | xmg2 | Positive | Positive |
| 2.6.3 | C | E | 7.5 | 56.00 | xmg2 | Positive | Positive |
| 2.7.1 | C | E | 11 | 37.59 | xmg2 | Positive | Positive |
| 2.7.2 | C | E | 5.5 | 20.41 | xmg2 | Positive | Positive |
| 2.7.3 | C | E | 5.5 | 184.24 | xmg2 | Positive | Positive |
| 2.8.1 | C | E | 9 | 64.93 | xmg2 | Positive | Positive |
| 2.8.2 | C | E | 9 | 40.83 | xmg2 | Positive | Positive |
| 2.8.3 | C | E | 3 | 119.20 | xmg2 | Positive | Positive |
| 2.9.1 | C | E | 9.5 | 66.82 | xmg2 | Positive | Positive |
| 2.9.2 | C | E | 11 | 79.23 | xmg2 | Positive | Positive |
| 2.9.3 | C | E | 10 | 98.36 | xmg2 | Positive | Positive |
| 2.10.1 | C | E | 4.5 | 102.45 | xmg2 | Positive | Positive |
| 2.10.2 | C | E | 6 | 105.69 | xmg2 | Positive | Positive |
| 2.10.3 | C | E | 5.5 | 159.24 | xmg2 | Positive | Positive |

### EXAMPLE 5

### Binning of Antibodies.

Beads were prepared for coupling to primary unknown antibodies using the following procedure. Individual tubes were used for each unknown supernatant. The volume of supernatant needed was calculated using the following formula: (n+10) x 50 µL (where n = total number of samples on plate). If the concentration was known, a. concentration of 0.5 µg/mL was used. While protecting the beads from light, the bead stock was gently vortexed, and diluted in supernatant to a concentration of 2500 of each bead per well or 0.5X10⁵ /mL. Samples were incubated on a shaker in the dark at room temperature overnight, or for 2 hours if at a known concentration of 0.5 µg/mL.

The filter plate was prewetted by adding 200 µL wash buffer per well, which was then aspirated. 50 µL of each bead was added to each well of the filter plate. Samples were washed once by adding 100 µL/well wash buffer and aspirating. Antigen and controls were added to the filter plate at 50 µl/well. The plate was covered, incubated in the dark for 1 hour on a shaker, and then samples were washed 3 times. A secondary unknown antibody was then added at 50 µL/well. The same dilution (or concentration if known) as used for the primary antibody was employed. The plate was incubated in the dark for two hours at room temperature on a shaker, and the samples were washed three times. 50 µL/well biotinylated mxhIgG diluted 1:500 was added, and samples were incubated in the dark for 1 hour with shaking at room temperature.

After starting the Luminex 100™, samples were washed 3 times. 50 uL/well Streptavidin-PE at a 1:1000 dilution was added, and samples were incubated in the dark for 15 minutes with shaking at room temperature. After running 2 wash cycles on the Luminex 100™, samples were washed 3 times. Contents in each well were resuspended in 80 µL blocking buffer. Samples were carefully mixed with pipetting several times to resuspend the beads. Samples were then analyzed on the Luminex 100™. Results are presented in Table 6 below. As shown, the antibodies in each bin were found to compete with one another.

**Table 6:**

| **Binning of ANGPTL4 Antibodies** | | | | | |
|---|---|---|---|---|---|
| **bin 1** | **bin 2** | | **Bin 3** | | **bin 4** |
| | **2a** | **2b** | **3a** | **3b** | |
| x1.2.1 | x1.1.2 | x1.6.1 | x1.10.1 | x2.3.1 | x2.4.1 |
| x1.2.3 | x1.1.3 | x1.6.2 | x1.10.2 | x2.3.2 | |
| x1.3.1 | x2.2.2 | x1.6.3 | x1.10.3 | x2.3.3 | |
| x1.3.2 | x2.2.3 | x1.7.1 | x1.11.1 | | |
| x1.3.3 | x2.5.1 | x1.7.2 | x1.11.2 | | |
| x1.4.1 | x2.5.2 | x1.7.3 | x1.11.3 | | |
| x1.4.2 | x2.5.3 | | | | |
| x1.5.2 | | | | | |
| x1.5.3 | | | | | |
| x1.8.1 | | | | | |
| x1.8.2 | | | | | |
| x1.8.3 | | | | | |
| x1.9.1 | | | | | |
| x1.9.2 | | | | | |
| x1.9.3 | | | | | |
| x2.6.1 | | | | | |
| x2.6.2 | | | | | |
| x2.6.3 | | | | | |
| x2.7.1 | | | | | |
| x2.7.2 | | | | | |
| x2.7.3 | | | | | |
| x2.8.2 | | | | | |
| x2.8.3 | | | | | |
| x2.9.1 | | | | | |
| x2.9.2 | | | | | |
| x2.9.3 | | | | | |
| x2.10.1 | | | | | |
| x2.10.2 | | | | | |
| x2.10.3 | | | | | |

### EXAMPLE 6

### Determination of anti-ANGPTL4 antibody affinity using BiaCore® analysis--ANGPTL4

### Low Resolution Screen of 12 Purified Monoclonal Antibodies

The following protocol was used to determine anti-ANGPTL4 antibody affinities using a BiaCore® analysis. A high-density goat anti-human antibody surface over a CM5 BiaCore® chip was prepared using routine amine coupling. All purified mAbs were diluted to ∼ 5 µg/mL in HBS-P running buffer containing 100 µg/mL BSA and 10 mg/mL carboxymethyldextran. Each mAb was captured on a separate surface using a 2 minute contact time, and a 5 minute wash for stabilization of mAb baseline.

ANGPTL4 was kinjected at 292 nM over all surfaces for 75 seconds, followed by a 3-minute dissociation. Double-referenced binding data were obtained by subtracting the signal from a control flow cell and subtracting the baseline drift of a buffer kinject just prior to the ANGPTL4 injection. ANGPTL4 binding data for each mAb were normalized for the amount of mAb captured on each surface. The normalized, drift-corrected responses for 12 mAbs were also measured.

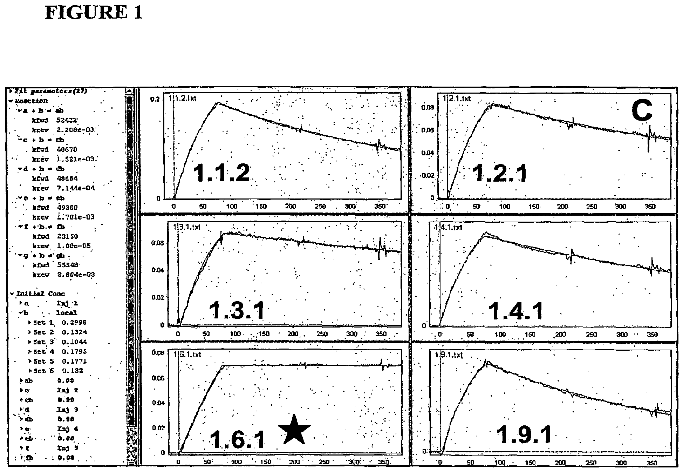

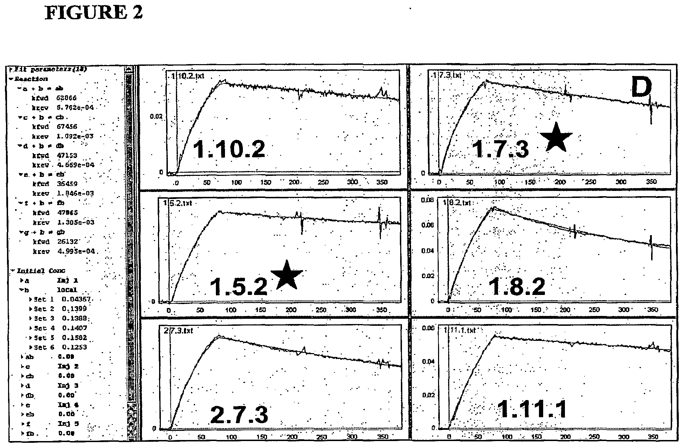

Data were globally fit to a 1:1 interaction model to determine the binding kinetics. Fits for the 12 tested antibodies are shown in **Figure 1** and **Figure 2****.** The sensorgrams are shown with their respective fits and are identified by sample number. Stars indicate mAbs selected to be studied in medium resolution. The kinetic analysis results of ANGPTL4 binding at 25°C are listed in Table 7 below. The mAbs are ranked from high affinity to low affinity.

**Table 7:**

| **ANGPTL4 Low Resolution BiaCore® Screen of 12 Purified Monoclonal Antibodies** | | | | |
|---|---|---|---|---|
| Sample | Amt. Captured (RU) | kₐ (M⁻¹s⁻¹) | k_{d} (S⁻¹) | K_{d} (nM) |
| **1.6.1** | **855** | **2.3 X 10⁴** | **1.0 X 10⁻⁵** * | **0.43** |
| **1.5.2** | **346** | **4.7 X 10⁴** | **4.7 X 10⁻⁴** | **10** |
| 1.10.2 | 475 | 6.3 X 10⁴ | 6.8 X 10⁻⁴ | 11 |
| 1.3.1 | 432 | 4.9 X 10⁴ | 7.1 X 10⁻⁴ | 15 |
| **1.7.3** | **243** | **6.7 X 10⁴** | **1.1 X 10⁻³** | **16** |
| 1.11.1 | **646** | 2.6 X 10⁴ | 5.0 X 10⁻⁴ | **19** |
| 2.7.3 | 324 | 4.8 X 10⁴ | 1.3 X 10⁻³ | 27 |
| 1.2.1 | 335 | 4.9 X 10⁴ | 1.5 X 10⁻³ | 31 |
| 1.4.1 | 257 | 4.9 X 10⁴ | 1.7 X 10⁻³ | 35 |
| 1.1.2 | 327 | 5.2 X 10⁴ | 2.2 X 10⁻³ | 42 |
| 1.8.2 | 706 | 3.6 X 10⁴ | 1.8 X 10⁻³ | 51 |
| 1.9.1 | 431 | 5.6 X 10⁴ | 2.8 X 10⁻³ | 51 |

With reference to Table 7, the samples shown in bold were run in a medium resolution mode (5-7 additional ANGPTL4 concentrations) to examine more closely the binding kinetics of these mAbs. The "*" indicates that this k_{d} was held constant as a best estimate for the order of magnitude characteristic of slow off-rate data. The fitting model for 1.6.1 detected no measurable decay in signal over the relatively brief dissociation time and therefore required the k_{d} to be constant in order to fit the on-rate data. Sample 1.7.3 was chosen over samples 1.3.1 and 1.10.2 because mAb 1.7.3 showed a higher normalized Rmax (0.1399) with less immobilized mAb (243 RU) as compared to mAbs 1.3.1 (Rmax = 0.1044 with 432 RU mAb) and 1.10.2 (Rmax = 0.04367 with 475 RU mAb).

### EXAMPLE 7

### Determination of anti-ANGPTL4 antibody affinity using BiaCore® analysis --ANGPTL4

### Medium Resolution Screen with Three Purified Monoclonal Antibodies

The following is another protocol that was used to determine anti-ANGPTL4 antibody affinity using another BiaCore® analysis. A high-density goat antihuman antibody surface was prepared over a CM5 BiaCore® chip using routine amine coupling. mAbs 1.5.2 and 1.7.3 were diluted to ∼ 5 µg/mL and mAb 1.6.1 was diluted to ∼ 2.5 µg/mL. All three mAbs were diluted with HBS-P running buffer containing 100 µg/mL BSA. Each purified mAb was captured for two minutes on a different flow cell surface for every ANGPTL4 injection cycle using a BiaCore® 2000 instrument.

ANGPTL4 was kinjected at 593, 198, 99, 49, 25, 12, and 6 nM over all surfaces for 1.5 minutes, followed by a 20 minute dissociation. The samples were prepared in HBS-P running buffer containing 100 µg/mL BSA. All samples were randomly injected in duplicate with several mAb capture/buffer kinject cycles interspersed for double referencing. The high-density goat α human antibody surfaces were regenerated with a 12-second pulse of 1/100 diluted concentrated phosphoric acid (146 mM, pH 1.5) after each cycle.

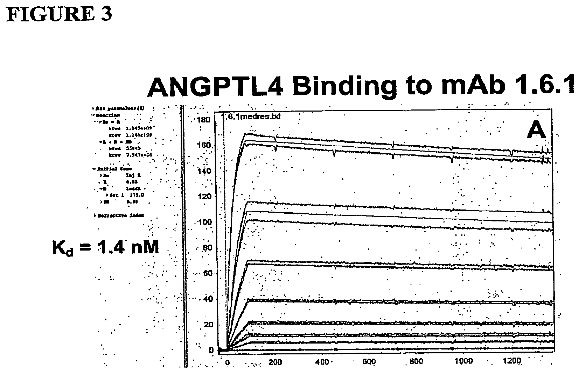

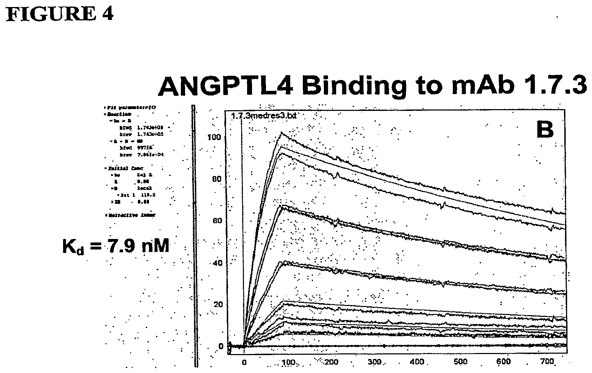

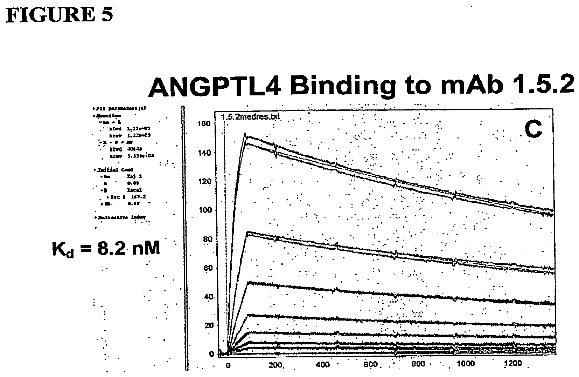

The data were fit to a 1:1 interaction model with mass transport using CLAMP software (University of Utah, Salt Lake City, UT). The data from the 593 nM injections over mAb 1.7.3 were not included in the fitting software because it displayed heterogeneity. Table 8 provides the resulting binding constants. The fits to the data are shown in Figures 3,4 and 5.

**Table 8:**

| **ANGPTL4 Low Resolution BiaCore® Screen of 3 Purified Monoclonal Antibodies** | | | | |
|---|---|---|---|---|
| **Sample** | **Rₘₐₓ** | **kₐ (M⁻¹s⁻¹)** | **k_{d} (s⁻¹)** | **K_{d} (nM)** |
| 1.6.1 | 173 | 5.58 X 10⁴ | 7.95 X 10⁻⁵ | 1.4 |
| 1.7.3 | 119 | 9.98 X 10⁴ | 7.86 X 10⁻⁴ | 7.9 |
| 1.5.2 | 167 | 4.06 X 10⁴ | 3.34 X 10⁻⁴ | 8.2 |

### EXAMPLE 8

### Structural Analysis of ANGPTL4 Antibodies

The variable heavy chain genes and the variable light chain genes of the antibodies were sequenced to determine their DNA sequences. The VH and VL transcripts were amplified from individual hybridoma clones in 96 well plates using reverse transcriptase polymerase chain reaction (RT-PCR). Reverse transcription-PCR (RT-PCR) was carried out by rapidly freezing cells in the presence of ribonuclease inhibitor plus DTT and by using extracts of 250 or fewer cells directly in the RT-PCR assay. (Klebe R.J., et al (1996) RT-PCR without RNA isolation. BioTechniques 21: 1094-1100). Hybridoma clones in 96 well plates were spun briefly to collect cells in the bottom of wells, and lysed in freezings solution (0.15M NaCl, 10 mM Tris, pH 8.0). 10µL of resuspended cells were transferred to a 96 well plate with 10 µl of 2X RNase inhibitor stock (0.15M NaCl, 10 mM Tris, pH 8.0, 100U Rnasin, 5mM DTT). Cells were rapidly frozen by placing the plates into ethanol prechilled to -70°C.

Two PCR reactions were run for each hybridoma: one for light chain (kappa (κ) or lambda (λ)), and one for gamma heavy chain (γ). A QIAGEN® OneStep RT-PCR kit was used for amplification, (Qiagen® Catalog No.210212). In the coupled RT-PCR reactions, cDNA was synthesized with a unique blend of RT enzymes (Omniscript™ and Sensiscript®) using antisense sequence specific primers that corresponded to C-κ, C-λ or to a consensus of the CH1 regions of Cγ genes. RT was preformed at 50°C for 1 hour followed by PCR amplification of the cDNA by HotStarTaq® DNA Polymerase for high specificity and sensitivity. Each PCR reaction used a mixture of 5' sense primers which are provided in Table 13 below. Primer sequences were designed based on leader sequences of VH, VK and Vλ. PCR reactions were run at 94°C for 15 minutes, initial hot start followed by 40 cycles of 94°C for 30 seconds (denaturation), 60°C for 30 seconds (annealing) and 72°C for 1 minute (elongation).

An aliquot of 5 µL of each PCR product was analyzed by agarose gel electrophoresis, stained with ethidium bromide and photographed under UV transillumination. PCR products were purified and directly sequenced using forward and reverse PCR primers using the ABI PRISM BigDye® terminator cycle sequencing ready reaction Kit (Perkin Elmer).

Analysis of IgH and IgL sequences was accomplished using sequence analysis software tools, and by alignment of VH genes to a proprietary antibody germ line database. The sequences were then translated to determine their primary amino acid sequence and compared to the germline VH, D and J-region sequences to assess somatic hypermutations.

Table 9 below shows the alignment of the amino acid sequences of the light chain variable domain regions of 15 anti-ANGPTL4 antibodies with their respective germline sequences. The # sign indicates that no equivalent amino acid was present. The identification of the CDRs (CDR1, CDR2, CDR3) and framework regions (FR1, FR2, and FR3) of each antibody are shown under the respective column headings.

Table 10 shows the alignment of the amino acid sequences of the heavy chain variable domain regions of 22 anti-ANGPTL4 antibodies with their respective germline sequences. The # sign indicates that no equivalent amino acid was present.

**Table 9**

| **Light Chain of ANGPTL4 Antibodies** | | | | | | |
|---|---|---|---|---|---|---|
| **Seq ID No.** | **Chain Name** | **V** | **J** | **FR1** | **CDR1** | **FR2** |
| 1 | | | Germline | DIVMTQSPLSLPVTPGEPASISC | RSSQSLLHSNGYNYLD | WYLQKPGQSPQLLIY |
| 2 | 2.8.1 | A3 | JK4 | DIVMTQSPLSLPVTPGEPASISC | RSSQSLLQSNGYNYLD | WYLQKPGQSPQLLIY |
| 3 | 2.10.1 | " | " | DIVMTQSPLSLPVTPGEPASISC | RSSQSLLQSNGYNYLD | WYLQKPGQSPQLLIY |
| 4 | 1.4.2 | " | " | DIVMTQSPLSLPVTPGEPASISC | RSSQSLLQSNGYNYLD | WYLQKPGQSPQLLIY |
| 5 | 1.11 | " | " | DIVMTQSPLSLPVTPGEPASISC | RSSQSLLHRNGYNYLD | WYLQKPGQSPQLLIY |
| 6 | 1.2 | " | " | DIVMTQSPLSLPVTPGEPASISC | RSSQSLLQSNGYNYLD | WYLQKPGQSPQLLIY |
| 7 | 1.5 | " | " | DIVMTQSPLSLPVTPGEPASISC | RSSQSLLQSNGYNYLD | WYLQKPGQSPQLLIY |
| 8 | | | Germline | DIQMTQSPSSLSASVGDRVTITC | RASQGISNYLA | WYQQKPGKVPKLLIY |
| 9 | 1.7 | A20 | JK4 | DIQMTQSPSSLSASVGDRVTITC | RASQGISNYLA | WYQQKPGKVPKLLIY |
| 10 | 1.6 | " | " | DIQMTQSPSSLSASVGDRVTITC | RASQDISNYLA | WYQQKPGKVPKLLIY |
| 11 | | | Germline | DIVMTQTPLSLSVTPGQPASISC | KSSQSLLHSDGKTYLY | WYLQKPGQPPQLLIY |
| 12 | 1. 10 | A2 | JK1 | DIVMTQTPLSLAVTPGQPASISC | KSSQSLLHSDRKTYLY | WYLQKPGQPPQLLIY |
| 13 | | | Germline | DIQMTQSPSSLSASVGDRVTITC | RASQGISNYLA | WYQQKPGKVPKLLIY |
| 14 | 2.3.1 | A20 | JK1 | DIQMTQSPSSLSASVGDRVTITC | RASQDINNYLA | WYQQKPGKVPKLLIY |
| 15 | | | Germline | EIVLTQSPDFQSVTPKEKVTITC | RASQSIGSSLH | WYQQKPDQSPKLLIK |
| 16 | 2.4.1 | A26 | JK4 | EIVLTQSPDFQSVTPKEKVTITC | RASQSIGSSLH | WYQQKPDQSPKLLIK |
| 17 | | Germline | Germline | DIVMTQSPLSLPVTPGEPASISC | RSSQSLLHSNGYNYLD | WYLQKPGQSPQLLIY |
| 18 | 1.9 | A3 | JK5 | DIVMTQSPLSLPVTPGEPASISC | RSSQSLLQSNGYNYLD | WYLQKPGQSPQLLIY |
| 19 | 1.8 | " | " | DIVMTQSPLSLPVTPGEPASISC | RSSQSLLQTNGYNYLD | WYLQKPGQSPQLLIY |
| 20 | | | Germline | EIVLTQSPDFQSVTPKEKVTITC | RASQSIGSSLH | WYQQKPDQSPKLLIK |
| 21 | 2.2.1.2 | A26 | JK3 | EIVLTQSPDFQSVTPKEKVTITC | RASQSIGSSLH | WYQQKPDQSPKLLIK |
| 22 | 2.5.1 | " | " | EIVLTQSPDFQSVTPKEKVTITC | RASQSIGSSLH | WYQLKPDQSPKLLIK |

| **Seq ID No.** | **Chain Name** | | **CDR2** | **FR3** | **CDR3** | **J** |
|---|---|---|---|---|---|---|
| 1 | Germline | | LGSNRAS | GVPDRFSGSGSGTDFTLKISRVEAEDVGVYYC | MQALQTLT | FGGGTKVEIKR |
| 2 | 2.8.1 | | LGSNRAS | GVPDRFSGSGSGTDFTLKISRVEAEDVGVYYC | MQALQTLT | FGGGTKVEIKR |
| 3 | 2.10.1 | | LGSNRAS | GVPDRFSGSGSGTDFTLKISRVEAEDIGVYYC | MQALQTLT | FGGGTKVEIKR |
| 4 | 1.4.2 | | LGSNRAS | GVPDRFSGSGSGTDFTLKISRVEAEDVGVYYC | MQALQTLT | FGGGTKVEIKR |
| 5 | 1.11 | | LGSHRAS | GVPDRFSGSGSGTDFTLKISRVEAEDVGVYYC | MQALQTLT | FGGGTKVEIKR |
| 6 | 1.2 | | LGSNRAS | GVPDRFSGSGSGTDFTLKISRVEAEDVGVYYC | MQALQTLT | FGGGTKVEIKR |
| 7 | 1.5 | | LGSNRAS | GVPDRFSGSGSGTDFTLKISRVEAEDVGVYYC | MQALQTLT | FGGGTKVEIKR |
| 8 | Germline | | AASTLQS | GVPSRFSGSGSGTDFTLTISSLQPEDVATYYC | QKYNSA##T | FGGGTKVEIKR |
| 9 | 1.7 | | AASTLQS | GVPSRFSGSGSGTDFTLTISSLQPEDFATYYC | QEFNSAPLT | FGGGTKVEIKR |
| 10 | 1.6 | | AASSLQS | GVPSRFSGSGSGTDFTLTISSLQPEDVATYYC | QEFNSAPLT | FGGGTKVEIKR |
| 11 | Germline | | EVSNRFS | GVPDRFSGSGSGTDFTLKISRVEAEDVGVYYC | MQSIQLPWT | FGQGTKVEIKR |
| 12 | 1. 10 | | EVSNRFS | GVPDRFSGSGSGTDFTLKISRVEAEDVGVYYC | MQSIQLPWT | FGQGTKVEIKR |
| 13 | Germline | | AASTLQS | GVPSRFSGSGSGTDFTLTISSLQPEDVATYYC | QKYNSAPRT | FGQGTKVEIKR |
| 14 | 2.3.1 | | AASTLQS | GVPSRFSGSGSGTDFTLSISSLQPEDVATYYC | QKYNSAPRT | FGQGTKVEIKR |
| 15 | Germline | | YASQSFS | GVPSRFSGSGSGTDFTLTINSLEAEDAATYYC | HQSSSLP#T | FGGGTKVEIKR |
| 16 | 2.4.1 | | HASQSFS | GVPSRFSGSGSGTDFTLTINSLEAEDAATYYC | HQSSSLPHT | FGGGTKVEIKR |
| 17 | Germline | | LGSNRAS | GVPDRFSGSGSGTDFTLKISRVEAEDVGVYYC | MQALQTLT | FGQGTRLEIKR |
| 18 | 1.9 | | LGSNRAS | GVPDRFSGSGSGTDFTLKISRVEAEDVGVYYC | MQALQTLT | FGQGTRLEIKR |
| 19 | 1.8 | | LGSNRAS | GVPDRFSGSGSGTDFTLKISRVEAEDVGLYYC | MQALQTLT | FGQGTRLEIKR |
| 20 | Germline | | YASQSFS | GVPSRFSGSGSGTDFTLTINSLEAEDAATYYC | HQSSSLPHT | FGPGTKVDIKR |
| 21 | 2.2.1.2 | | YASQSFS | GVPSRFSGSGSGTDFTLTINSLEAEDAATYYC | HQSSNLPHT | FGPGTKVDIKR |
| 22 | 2.5.1 | | YVSQSFS | GVPSRFSGSGSGTDFTLTISSLEAEDAATYCC | HQSSSLPHT | FGPGTKVDINR |

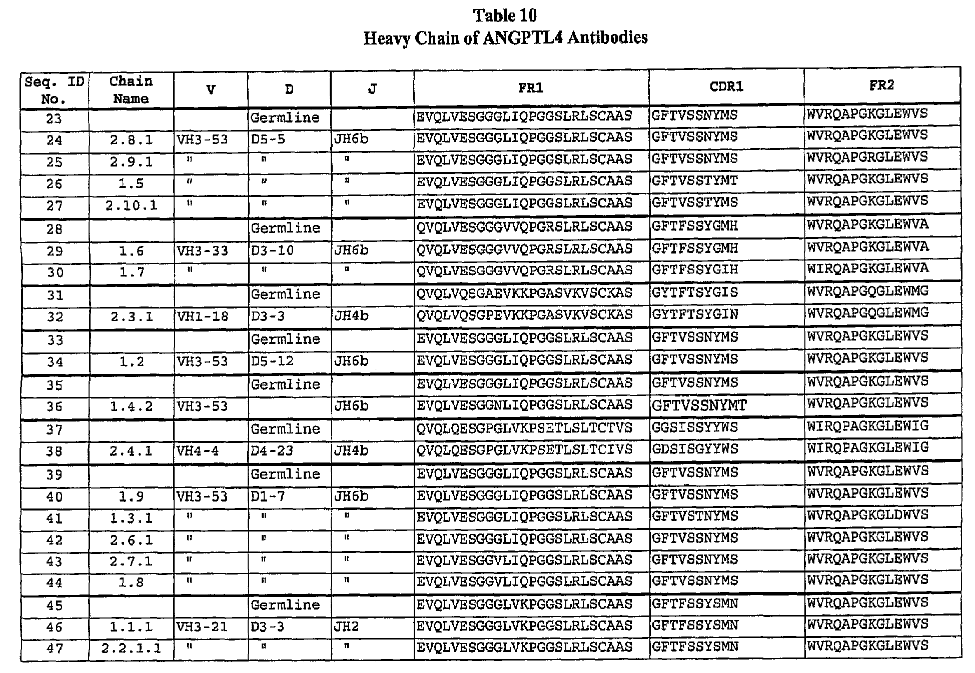

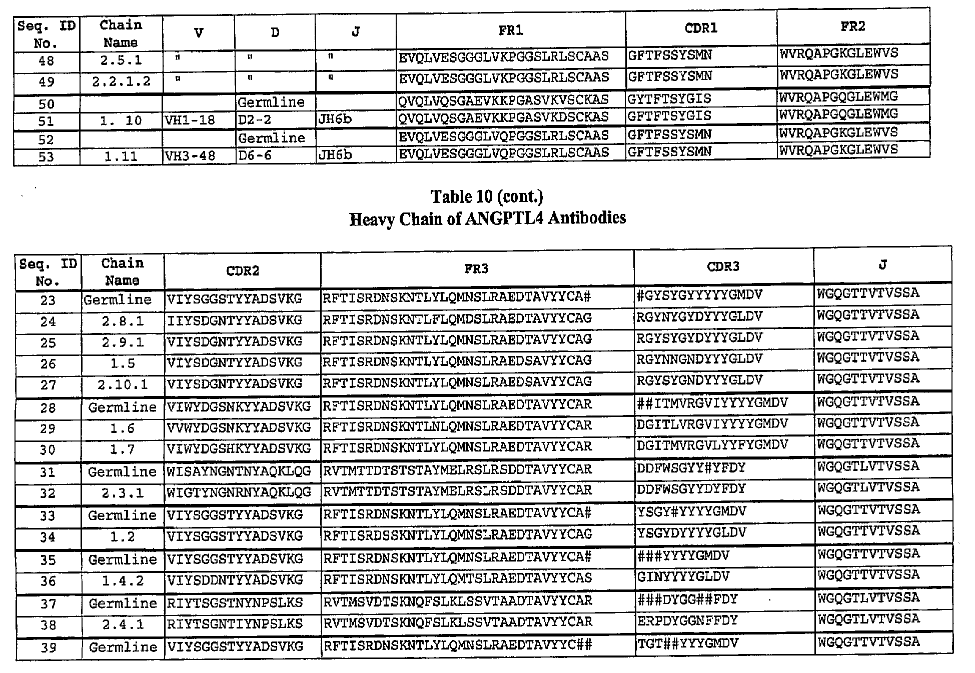

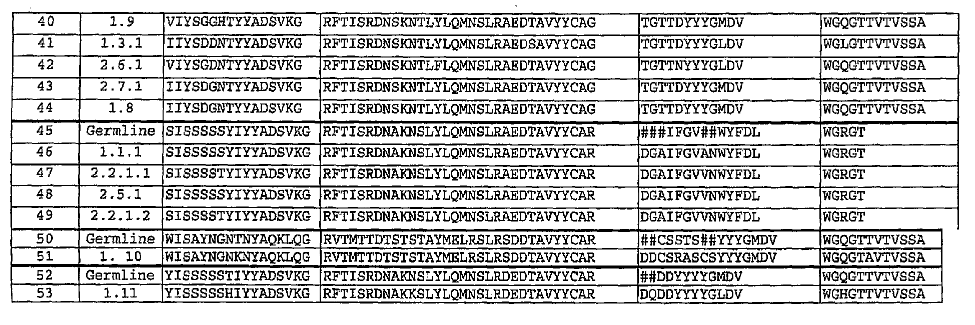

**Table 11**

| **Light Chain of ANGPTL4 Antibodies** | | | | |
|---|---|---|---|---|
| **Seq ID No.** | **Ab** | **CDR1 amino acid positions** | **CDR2 amino acid positions** | **CDR3 amino acid positions** |
| 2 | 2.8.1 | 24-39 | 55-61 | 94-101 |
| 3 | 2.10.1 | 24-39 | 55-61 | 94-101 |
| 4 | 1.4.2 | 24-39 | 55-61 | 94-101 |
| 5 | 1.11 | 24-39 | 55-61 | 94-101 |
| 6 | 1.2 | 24-39 | 55-61 | 94-101 |
| 7 | 1.5 | 24-39 | 55-61 | 94-101 |
| 9 | 1.7 | 24-34 | 50-56 | 89-97 |
| 10 | 1.6 | 24-34 | 50-56 | 89-97 |
| 12 | 1.10 | 24-39 | 55-61 | 94-102 |
| 14 | 2.3.1 | 24-34 | 50-56 | 89-97 |
| 16 | 2.4.1 | 24-34 | 50-56 | 89-97 |
| 18 | 1.9 | 24-39 | 55-61 | 94-101 |
| 19 | 1.8 | 24-39 | 55-61 | 94-101 |
| 21 | 2.2.1.2 | 24-34 | 50-56 | 89-97 |
| 22 | 2.5.1 | 24-34 | 50-56 | 89-97 |

**Table 12**

| **Heavy Chain of ANGPTL4 Antibodies** | | | | |
|---|---|---|---|---|
| **Seq. ID No.** | **Ab** | **CDR1 amino acid positions** | **CDR2 amino acid positions** | **CDR3 amino acid positions** |
| 24 | 2.8.1 | 26-35 | 50-65 | 98-112 |
| 25 | 2.9.1 | 26-35 | 50-65 | 98-112 |
| 26 | 1.5 | 26-35 | 50-65 | 98-112 |
| 27 | 2.10.1 | 26-35 | 50-65 | 98-112 |
| 29 | 1.6 | 26-35 | 50-66 | 99-116 |
| 30 | 1.7 | 26-35 | 50-66 | 99-116 |
| 32 | 2.3.1 | 26-35 | 50-66 | 99-111 |
| 34 | 1.2 | 26-35 | 50-65 | 98-110 |
| 36 | 1.4.2 | 26-35 | 50-65 | 98-108 |
| 38 | 2.4.1 | 26-35 | 50-65 | 98-109 |
| 40 | 1.9 | 26-35 | 50-65 | 98-109 |
| 41 | 1.3.1 | 26-35 | 50-65 | 98-109 |
| 42 | 2.6.1 | 26-35 | 50-65 | 98-109 |
| 43 | 2.7.1 | 26-35 | 50-65 | 98-109 |
| 44 | 1.8 | 26-35 | 50-65 | 98-109 |
| 46 | 1.1.1 | 26-35 | 50-66 | 99-112 |
| 47 | 2.2.1 | 26-35 | 50-66 | 99-112 |
| 48 | 2.5.1 | 26-35 | 50-66 | 99-112 |
| 49 | 2.2.1 | 26-35 | 50-66 | 99-112 |
| 51 | 1.10 | 26-35 | 50-66 | 99-114 |
| 53 | 1.11 | 26-35 | 50-66 | 99-110 |

The complete sequence information for the anti-ANGPTL4 antibodies are provided below with nucleotide and amino acid sequences for each gamma and kappa chain combination.

### ANTIBODY 1.10

**AA Sequence - Heavy Chain**
**Nucleotide Sequence - Heavy Chain**
**AA Sequence - Light Chain**
**Nucleotide Sequence - Light Chain**

### ANTIBODY 1.11

**AA Sequence - Heavy Chain**
**Nucleotide Sequence - Heavy Chain**
AA Sequence - Light Chain
**Nucleotide Sequence - Light Chain**

### ANTIBODY 1.1.1

**AA Sequence - Heavy Chain**
**Nucleotide Sequence - Heavy Chain**

### ANTIBODY 1.2

**AA Sequence - Heavy Chain**
**Nucleotide Sequence - Heavy Chain**
**AA Sequence - Light Chain**
**Nucleotide Sequence - Light Chain**

### ANTIBODY 1.3.1

**AA Sequence - Heavy Chain**
**Nucleotide Sequence - Heavy Chain**

### ANTIBODY 1.4.2

**AA Sequence - Heavy Chain**
**Nucleotide Sequence - Heavy Chain**
**AA Sequence - Light Chain**
**Nucleotide Sequence - Light Chain**

### ANTIBODY 1.5

**AA Sequence - Heavy Chain**
**Nucleotide Sequence - Heavy Chain**
**AA Sequence - Light Chain**
**Nucleotide Sequence - Light Chain**

### ANTIBODY 1.6

**AA Sequence - Heavy Chain**
**Nucleotide Sequence - Heavy Chain**
**AA Sequence - Light Chain** **(SEQ. ID NO.: 10)**
**Nucleotide Sequence - Light Chain**

### ANTIBODY 1.7

**AA Sequence - Heavy Chain**
**Nucleotide Sequence - Heavy Chain**
**AA Sequence - Light Chain**
**Nucleotide Sequence - Light Chain**

### ANTIBODY 1.8

**AA Sequence - Heavy Chain**
**Nucleotide Sequence - Heavy Chain**
**AA Sequence - Light Chain**
**Nucleotide Sequence - Light Chain** **(SEQ. ID NO.: 71)**

### ANTIBODY 1.9

**AA Sequence - Heavy Chain**
**Nucleotide Sequence - Heavy Chain**
**AA Sequence - Light Chain**
**Nucleotide Sequence - Light Chain** **(SEQ. ID NO.: 73)**

### ANTIBODY 2.10.1

**AA Sequence - Heavy Chain**
**Nucleotide Sequence - Heavy Chain**
**AA Sequence - Light Chain**
**Nucleotide Sequence - Light Chain**

### ANTIBODY 2.2.1.2

**AA Sequence - Heavy Chain** **(SEQ. ID NO.: 49)**
**Nucleotide Sequence - Heavy Chain** **(SEQ. ID NO.: 76)**
**AA Sequence - Light Chain** **(SEQ. ID NO.: 21)**
**Nucleotide Sequence - Light Chain** **(SEQ. ID NO.: 77)**

### ANTIBODY 2.3.1

**AA Sequence - Heavy Chain**
**Nucleotide Sequence - Heavy Chain**
**AA Sequence - Light Chain**
**Nucleotide Sequence - Light Chain**

### ANTIBODY 2.4.1.1

**AA Sequence - Heavy Chain**
**Nucleotide Sequence - Heavy Chain**
**AA Sequence - Light Chain**
**Nucleotide Sequence - Light Chain**

### ANTIBODY 2.5.1

**AA Sequence - Heavy Chain**
**Nucleotide Sequence - Heavy Chain**
**AA. Sequence - Light Chain**
**Nucleotide Sequence - Light Chain**

### ANTIBODY 2.6.1

**AA Sequence - Heavy Chain** **(SEQ. ID NO.: 42)**
**Nualeotide Sequence - Heavy Chain** **(SEQ. ID NO.: 84)**

### ANTIBODY 2.7.1

**AA Sequence - Heavy Chain**
**Nucleotide Sequence - Heavy Chain**

### ANTIBODY 2.8.1

**AA Sequence - Heavy Chain**
**Nucleotide Sequence - Heavy Chain**
**AA Sequence - Light Chain**
**Nucleotide Sequence - Light Chain**

### ANTIBODY 2.9.1

**AA Sequence - Heavy Chain**
**Nucleotide Sequence - Heavy Chain**

**TABLE 13 - Primers Sequences Used for Sequencing.**

| | | |
|---|---|---|
| VH | cacc ATG GAC TGG(C) ACC TGG AGG ATC | Seq ID No: 89 |
| VH | cacc ATG GAC TGG ACC TGG AGA(C) ATC | Seq ID No: 90 |
| VH | cacc ATG GAC TGG ACC TGG AGG GTC | Seq ID No: 91 |
| VH | cacc ATG GAC TGG ATT TGG AGG ATC | Seq ID No: 92 |
| VH | cacc ATG GAC ACA CTT TGC TC(A)C AC | Seq ID No: 93 |
| VH | cacc ATG GAA(G) TTG GGG CTG AGC TGG | Seq ID No: 94 |
| VH | cacc ATG GAG TTG(T) GGA CTG AGC TGG | Seq ID No: 95 |
| VH | cacc ATG GAG TTT GGG CTG(T) AGC TGG | Seq ID No: 96 |
| VH | cacc ATG GAA CTG GGG CTC CGC TGG | Seq ID No: 97 |
| VH | cacc ATG GAG TTG GGG CTG TGC TGG | Seq ID No: 98 |
| VH | cacc ATG GAG TTT TGG CTG AGC TGG | Seq ID No: 99 |
| VH | cacc ATG ACG GAG TTT GGG CTG AGC | Seq ID No: 100 |
| VH | cacc ATG AAA(G) CAC CTG TGG TTC TTC | Seq ID No: 101 |
| VH | cacc ATG AAA CAT CTG TGG TTC TTC | Seq ID No: 102 |
| VH | cacc ATG GGG TCA ACC GCC ATC CTC | Seq ID No: 103 |
| VH | cacc ATG TCT GTC TCC TTC CTC ATC TTC | Seq ID No: 104 |
| VK | ATG GGG TCC CAG GTT CAC CTC | Seq ID No: 105 |
| VK | ATG TTG CCA TCA CAA CTC ATT G | Seq ID No: 106 |

### EXAMPLE 9

### Cell Survival Assay 786-0 Cells

786-0 is a human cell line derived from renal carcinoma. This cell line lacks one allele and expresses a truncated protein (AA 1-104) from the second allele of the von Hippel-Lindau tumor suppressor gene (VHL). The inactivation of the VHL gene predisposes affected individuals to the human VHL cancer syndrome and is associated with sporadic renal cell carcinomas (RCC) and brain hemangioblastomas. Accordingly, this cell line represent a suitable *in vitro* model to study tumorogenic mechanisms in renal carcinoma (Pause A, et al., Proc Natl Acad Sci U.S.A., 95(3):993-8 February 3, 1998)

The following experiment demonstrates that treating 786-0 cells with purified ANGPTL4 protein, influences their survival in low serum conditions that would otherwise lead to cell death. More specifically, cells (5000 cells/well) were plated in the inner sixty wells of a 96-well plate in DMEM containing 10% FBS. The following day, the cells were washed twice in SFM and treated with ANGPTL4 protein at 0.6µg/mL in 0.5% FBS/DMEM. Untreated cells (in 0.5% of FBS) served as baseline controls. Cells cultured in 10 % FBS served as positive controls. On the third day following treatment, MTS (20 ul of MTS dye, CellTiter 96 Aqueous Non-Radioactive Cell Proliferation Assay Kit from Promega) was added to the medium, and the cells were incubated for 0.5-4 hours The absorbance of the wells was then determined using a microplate absorbance reader (490 nm). The results showed that ANGPTL4 protein preparation was able to stimulate the survival of 786-0 cells compared to the controls. The EC50 was below 500 ng/mL and above 100 ng/mL.

### Identification of ANGPTL4 neutralizing antibodies

As demonstrated in the Cell Survival Assay for 786-0 Cells, purified ANGPTL4 allows 786-0 cells to survive serum starvation with an EC50 between 0.1 -0.5 µg/mL, depending on the batch of protein used. To determine whether an antibody can neutralize ANGPTL4 activity, various amounts of antibody were added to the Cell Survival Assay for 786-0 cells, as described immediately above. The results were assessed by measuring the MTS activity of the cells after four days of treatment, comparing cells treated with various amount of the antibody, (1) relative treated with non-binding antibody (negative controls) and (2) relative to serum-starved cells (positive control).

Antibodies that can neutralize the ANGPTL4 activity at least with a molar ratio of 10:1 antibody:ANGPTL4 can be useful as a therapeutic. Lower molar ratios are desired.

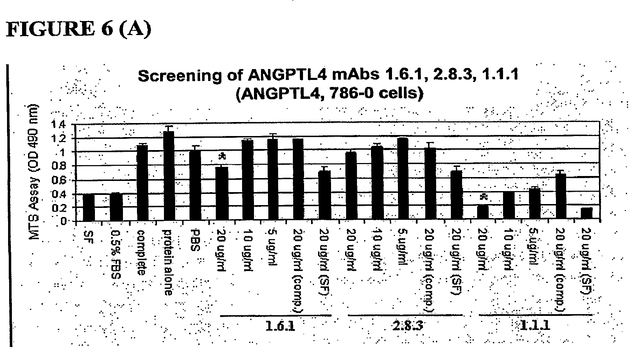

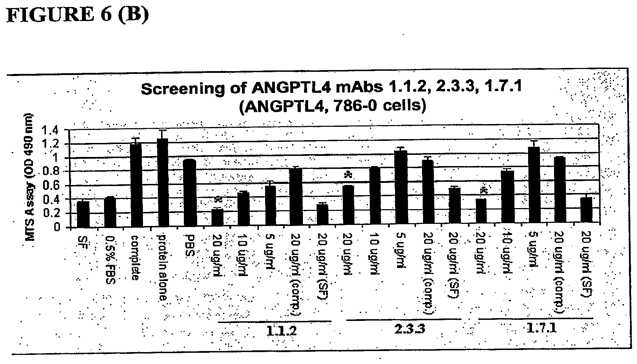

The results, as provided in Figures 6A and 6B, indicate that antibodies 1.1.2, 1.1.1, 1.7.1, 1.6.1, 2.3.3 significantly inhibited ANGPTL4 and induced cell death in 786-0 cells. Specifically, ANGPTL4 (500ng/ml) which increases 786-0 cell survival was inhibited by ANGPTL4 neutralizing antibodies in a dose dependent manner (IC50 of 27nM).

### ANGPTL4 neutralizing antibodies inhibit ANGPTL4-induced HUVEC proliferation.

As discussed previously, ANGPTL4 belongs to the angiopoietin-like family of angiogenic factors that can influence endothelial cell proliferation. More specifically, this protein is able to induce endothelial cell proliferation (Zhu et al). The following experiment was conducted to determine whether the antibodies described herein could neutralize the effect of ANGPTL4 protein on HUVECs and therefore inhibit cell proliferation.

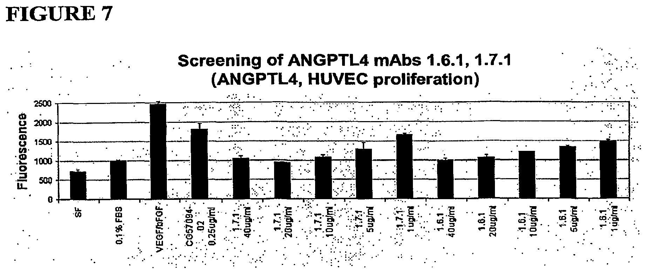

Cells (2000 cells/well) were plated in the inner sixty wells of a 96-well plate in Cascade 200 media containing various growth factors. The following day, the cells were washed twice in SF media and treated with ANGPTL4 protein at 0.25 µg/mL or ANGPTL4 protein mixed with different concentrations of mAbs in 0.1% FBS/Cascade 200 media. Untreated cells (grown in 0.1% FBS) served as baseline controls. Cells treated with VEGF/basic FGF (10ng/mL) served as positive controls. Two days after cells were plated, fresh treatments were added again without changing media. On the fourth day following treatment, Cell-titer blue (CellTiter-Blue Cell Viability Assay kit from Promega) was added to the medium and the cells were incubated for 1-4 hours. The fluorescence of the wells was then determined using a fluorometer (560excitation/590emission). The results, as provided in Figure 7, indicate that antibodies 1.7.1 and 1.6.1 inhibited survival activity of ANGPTL4 in HUVEC cells (P values are 0.0024 and 0.0045). At a concentration of 250 ng/ml, ANGPTL4 caused approximately 2-fold enhancement of HUVEC migration and induced tube formation in matrigel. HUVEC migration was inhibited by ANGPTL24 neutralizing antibodies in a dose dependent manner with an IC50 of 7 nm. HUVEC tube formation was also blocked at an IC50 of 27 nM.

### ANGPTL24 neutralizing antibodies inhibit ANGPTL4-induced HUVEC migration.

Studies have shown that ANGPTL4 is a proangiogenic factor (Zhu et al. Zhonghua Yi Xue Za Zhi 2002 Jan 25;82(2), Le Jan et al. Am J Pathol 2003 May 162(5)). The effects of ANGPTL4 on angiogenesis were determined by an *in vitro* migration assay. The following experiment was conducted to determine whether the antibodies described herein would neutralize the effect of ANGPTL4 protein on angiogenesis.

Eight micron biocoat chambers were coated with type I collagen. 10,000 HUVEC cells were added in Cascade 200 media to the top of each chamber. ANGPLT4 treatment was added to bottom chambers and anti-ANGPLT4 antibodies were added to top chambers and incubated for 4 hours. The medium from the top and bottom chambers was aspirated. Cells were stained with crystal violet in the bottom chamber. Cells were then destained washed with water and air dried. The cells in 3 fields/well under 20x microscope were counted.

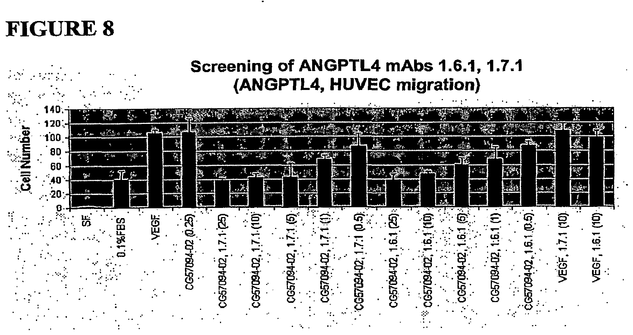

The results, as provided in Figure 8, indicate that ANGPTL4 protein stimulated HUVEC migration in the *in vitro* migration assay. Antibody clones 1.7.1 and 1.6.1 neutralized the activity of the protein and inhibited HUVEC migration in a dose-dependent manner. However these two clones do not appear to effect VEGF induced HUVEC migration. Accordingly, the neutralization activities of clone 1.7.1 and 1.6.1 are specific for the ANGPTL4 protein.

### ANGPTL4 neutralizing antibodies inhibit ANGPTL4-induced tube formation in HUVEC.

To test the effect of ANGPTL4 protein on angiogenesis, an *in vitro* tube formation assay was performed. The following experiment was conducted to determine whether the antibodies described herein could neutralize the effect of ANGPTL4 protein on angiogenesis.

A matrigel (BD Biosciences, cat# 354234) was thawed overnight on ice in a cold room. The following day, the matrigel was diluted to 6mg/mL with cold Cascade 200 media and plated at 100 µL per well in a 96-well plate. HUVEC cells (5000 cell/well) were mixed with protein and mAb in Cascade 200 media containing 0.1% FBS, and the mixture was added to the hardened matrigel. The matrigel was incubated for 18 hours and tube formation was observed under a microscope.

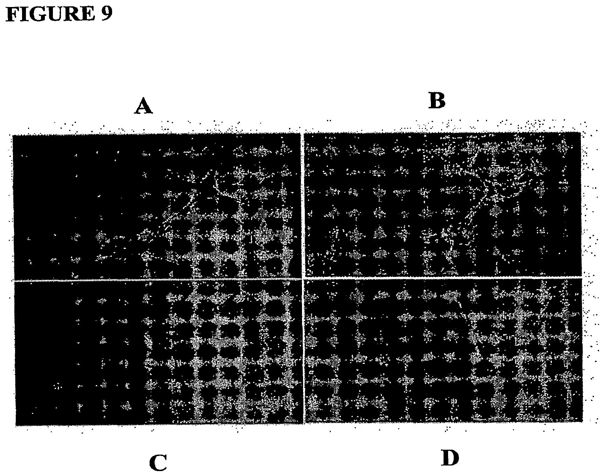

The results indicated that HUVEC cells that were treated with VEGF (10 ng/ml) served as a positive control. Furthermore, ANGPTL4 protein promoted tube formation in HUVEC cells. Antibody clones 1.7.1 and 1.6.1 neutralized the activity of ANGPTL4 protein and inhibited tube formation. Photomicrographs of the in vitro tube formation assay are shown in Figure 9.

### EXAMPLE 10

### Use of ANGPTL4 mAb to Inhibit Angiogenesis in Preclinical In Vivo Model

Previously we have shown that ANGPTI4 mAb designated CR064 inhibited endothelial cell proliferation, migration and tube formation in vitro. Hence, we examined whether CR064 would inhibit angiogenesis in in vivo model. The following experiments were conducted to determine whether the antibodies described herein could neutralize bFGF and VEGF induced angiogenesis using a matrigel plug model in athymic nude mice.

Two studies were carried out to induce angiogenesis in vivo in the matrigel plug model. Female athymic nude mice were injected subcutaneously on the right flank with 0.5 ml of a matrigel mixture on day 0. In one study 100 ng/ml of bFGF + 20 U/ml heparin was mixed with the matrigel. In another study, 150 ng/ml of human VEGF165 was mixed with matrigel. In both studies, negative control groups received matrigel mixed with HBSS. CR064 (0, 1.0, 5.0 and 10.0 mg/kg) was administered intraperitoneally in mice on days 0 and 3. On day 7 mice were anesthetized by Ketamine and Xylazine mixture and matrigel plugs were removed. Gels were photographed under transillumination and morphometric analysis was made.

Data from morphometric analyses are shown in Figures 10A and 10B. In both studies, the mean number of vessel ends, nodes, and lengths were highest in the presence of the growth factors. ANGPTL4 mAb (CR064) inhibited vessel lengths, ends and nodes at all doses (1, 5, 10 mg/kg) and the inhibitions were statistically significant (P<0.01). P values were calculated by Students t-test.

Snap frozen sections of matrigels were also used for immunocytochemical staining with rat monoclonal antibody directed against mouse CD31 antigen conjugated with phycoerythrin. Anti-CD31-PE stained slides were observed under Fluorescence microscope using appropriate filters. In both experiments CR064 caused decrease in CD31 positive cells in a dose dependent manner.

Hence, ANGPTL4 mAb (CR064) inhibited bFGF and VEGF -induced angiogenesis in matrigel plug assay in athymic nude mice. Inhibition of angiogenesis was statistically significant.

### EXAMPLE 11

### Use of ANGPTL4/CG57094 to Induce Angiogenesis in Matrigel Plug Model In Vivo

Previously we have shown that ANGPTL4 induces endothelial cell proliferation, migration and tube formation in vitro in a manner similar to VEGF and bFGF. Here we examined whether ANGPTL4 induces angiogenesis in in vivo matrigel plug model. The following experiment was conducted to determine the effect of ANGPTL4 in inducing angiogenesis in matrigel plug in athymic nude mice in a manner similar to VEGF and bFGF.

ANGPTL4 was mixed with 0.5 ml of matrigel at various concentrations (0, 100, 500, 1000, 1500, and 2500 ng/ml). Matrigels were implanted in nude mice on day 0. On day 7 mice were sacrificed, matrigels were photographed and morphometric analysis of vessel ends, nodes and lengths were carried out. Vessel lengths, ends and nodes increased as a function of ANGPTL4 in a dose dependent manner (Figure 11). P values were calculated by Students t-test. Hence, ANGPTL4 (CG57094-02) induces dose-dependent angiogenesis in matrigel plug assay in nude mice.

### EXAMPLE 12

### Use of Anti-ANGPTL4 Antibodies for Cancer Treatment

A patient suffering from a carcinoma is selected for treatment. The patient is injected daily with a therapeutically effective dose of an anti-ANGPTL4 antibody. At periodic times during the treatment, the human patient is monitored to determine the progress of the carcinoma. It is discovered that the patient treated with the anti-ANGPTL4 antibody has a lower level of cancer growth and spreading in comparison to similar patients that have not been given the antibody treatment.

### EXAMPLE 13

### Use of Anti-ANGPTL4 Antibodies for Tumor Treatment

A human patient suffering from a tumor is selected for treatment. The patient is given a daily injection of a therapeutically effective dose of an anti-ANGPTL4 antibody. At periodic times during the treatment, the patient is monitored to determine whether the tumor spreads, remains stable, or shrinks. It is discovered that the patient treated with anti-ANGPTL4 antibodies has a lower level of tumor growth and metastasis in comparison to similar patients that are not given the antibodies.

### EXAMPLE 14

### Use of anti-ANGPTL4 antibodies for the treatment of hyperlipidemia

In order to treat a patient exhibiting indications of hyperlipidemia, the patient is injected daily with an effective amount of an anti-ANGPTL4 antibody. At periodic times during the treatment, the patient is monitored to determine whether the hyperlipidemia is progressing. In particular, the patient is tested to see if there is an increase of lipids and lipoproteins in the bloodstream. These lipids include cholesterol, cholesterol esters, phospholipids and triglycerides, for example. It is discovered that the patient treated with anti-ANGPTL4 antibodies has a lower blood level of in comparison to untreated patients.

### EXAMPLE 15

### Use of anti-ANGPTL4 antibodies for diagnosing and staging cancer in human patients

Serum levels of ANGPTL4 from healthy individuals and from patients having carcinomas are analyzed. The concentration of ANGPTL4 is assessed using quantitative sandwich ELISA with 2 fully human anti-ANGPTL4 antibodies. The results show that ANGPTL24 levels are noticeably higher in the sera of patients having carcinomas compared to the control patients who do not have cancer. By measuring the level of ANGPTL4, the physician can determine the stage of cancer that the patient is in.

### EXAMPLE 16

### Use of anti-ANGPTL4 antibodies for diagnosing and staging hyperlipidemia in human patients

Serum levels of ANGPTL4 from healthy individuals and from patients having hyperlipidemia are analyzed. The concentration of ANGPTL4 is assessed using quantitative sandwich ELISA with 2 fully human anti-ANGPTL4 antibodies. The results show that ANGPTL4 levels are noticeably higher in the sera of patients having hyperlipidemia compared to the control patients who do not have the disease. By measuring the level of ANGPTL4, the physician can determine the stage of hyperlipidemia that the patient is in.

## Claims

1. An isolated antibody, or antigen-binding fragment thereof, that binds to ANGPTL4 and that comprises both a heavy chain amino acid sequence and a light chain amino acid sequence selected from the group consisting of:
(a) the heavy chain amino acid sequence of SEQ ID NO: 24 and the light chain amino acid sequence of SEQ ID NO: 2;
(b) the heavy chain amino acid sequence of SEQ ID NO: 26 and the light chain amino acid sequence of SEQ ID NO: 7;
(c) the heavy chain amino acid sequence of SEQ ID NO: 27 and the light chain amino acid sequence of SEQ ID NO: 3;
(d) the heavy chain amino acid sequence of SEQ ID NO: 29 and the light chain amino acid sequence of SEQ ID NO: 10;
(e) the heavy chain amino acid sequence of SEQ ID NO: 30 and the light chain amino acid sequence of SEQ ID NO: 9;
(f) the heavy chain amino acid sequence of SEQ ID NO: 32 and the light chain amino acid sequence of SEQ ID NO: 14;
(g) the heavy chain amino acid sequence of SEQ ID NO: 34 and the light chain amino acid sequence of SEQ ID NO: 6;
(h) the heavy chain amino acid sequence of SEQ ID NO: 36 and the light chain amino acid sequence of SEQ ID NO: 4;
(i) the heavy chain amino acid sequence of SEQ ID NO: 38 and the light chain amino acid sequence of SEQ ID NO: 16;
(j) the heavy chain amino acid sequence of SEQ ID NO: 40 and the light chain amino acid sequence of SEQ ID NO: 18;
(k) the heavy chain amino acid sequence of SEQ ID NO: 44 and the light chain amino acid sequence of SEQ ID NO: 19;
(l) the heavy chain amino acid sequence of SEQ ID NO: 48 and the light chain amino acid sequence of SEQ ID NO: 22;
(m) the heavy chain amino acid sequence of SEQ ID NO: 49 and the light chain amino acid sequence of SEQ ID NO: 21;
(n) the heavy chain amino acid sequence of SEQ ID NO: 51 and the light chain amino acid sequence of SEQ ID NO: 12; and
(o) the heavy chain amino acid sequence of SEQ ID NO: 53 and the light chain amino acid sequence of SEQ ID NO: 5.

2. The antibody or antigen-binding fragment of claim 1, wherein
(a) the antibody is a monoclonal antibody;
(b) the antibody is a chimeric antibody;
(c) the antibody is a human antibody, or
(d) the antigen-binding fragment is selected from Fab, Fab', F(ab')₂, Fv and single chain.

3. A composition comprising the antibody or antigen-binding fragment of claim 1 or 2, and a pharmaceutically acceptable carrier.

4. The antibody or antigen-binding fragment of claim 1 or 2, wherein the antibody or antigen-binding fragment is conjugated to a therapeutic agent.

5. The antibody or antigen-binding fragment of claim 4, wherein the therapeutic agent is a toxin or a radioisotope.

6. A hybridoma cell line producing the antibody of claim 1 or 2.

7. A transformed cell comprising a gene encoding the antibody or antigen-binding fragment of claim 1 to 2.

8. The antibody or antigen-binding fragment of claim 1 to 2 for use as a medicament.

9. The antibody or antigen-binding fragment of claim 1 or 2 for use in a method of treating a disease related to angiogenesis in a mammal.

10. The antibody or antigen-binding fragment of claim 9, wherein the disease related to angiogenesis is a cancer.

11. The antibody or antigen-binding fragment of claim 10, wherein the cancer is a lung cancer, colon cancer, gastric cancer, renal cancer, prostate cancer, ovarian cancer, uterine cancer, or ocular carcinoma.

12. The antibody or antigen-binding fragment of claim 9, wherein the disease related to angiogenesis is a non-neoplastic condition selected from the group consisting of:
arthritis, rheumatoid arthritis, psoriasis, psoriatic plaques, sarcoidosis, atherosclerosis, atherosclerotic plaques, edema from myocardial infarction, retinopathy of prematurity, retrolental fibroplasia, neovascular glaucoma, macular degeneration, diabetic macular edema, corneal neovascularization, corneal graft neovascularization, corneal graft rejection, retinal or choroidal neovasculariztion, neovasculariztion of the angle, ocular neovascular disease, vascular restenosis, arteriovenous malformation.

13. The antibody or antigen-binding fragment of any of claims 9 to 12, wherein the mammal is a human.

14. An isolated polynucleotide molecule comprising:
(a) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 54 and 55;
(b) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 56 and 57;
(c) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 59 and 60;
(d) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 62 and 63;
(e) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 64 and 65;
(f) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 66 and 67;
(g) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 68 and 69;
(h) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 70 and 71;
(i) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 72 and 73;
(j) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 74 and 75;
(k) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 76 and 77;
(l) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 78 and 79;
(m) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 80 and 81;
(n) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 82 and 83; and
(o) a nucleotide sequence encoding the heavy chain amino acid sequence and the light chain amino acid sequence of a human anti-ANGPTL4 antibody, wherein the nucleotide sequence comprises the nucleotide sequences of SEQ ID NOS: 86 and 87.

15. An assay kit for the detection of ANGPTL4 in mammalian tissues or cells in order to screen for lung, colon, gastric, renal, prostate, ovarian, uterine, or ocular carcinomas, the ANGPTL4 being an antigen expressed by lung, colon, gastric, renal, prostate, ovarian, uterine, or ocular carcinomas, the kit comprising an antibody or antigen-binding fragment of any one of claims 1 to 3 and means for indicating the reaction of the antibody or antigen-binding fragment with the antigen, if present.

## Patentansprüche

1. Isolierter Antikörper, oder antigenbindendes Fragment desselben, der sich an ANGPTL4 bindet und der sowohl eine schwere Aminosäuresequenz als auch eine leichte Aminosäuresequenz umfasst, die aus der Gruppe ausgewählt werden, welche aus Folgendem besteht:
(a) die Sequenz der schweren Aminosäure SEQ ID NO: 24 und die Sequenz der leichten Aminosäure SEQ ID NO: 2;
(b) die Sequenz der schweren Aminosäure SEQ ID NO: 26 und die Sequenz der leichten Aminosäure SEQ ID NO: 7;
(c) die Sequenz der schweren Aminosäure SEQ ID NO: 27 und die Sequenz der leichten Aminosäure SEQ ID NO: 3;
(d) die Sequenz der schweren Aminosäure SEQ ID NO: 29 und die Sequenz der leichten Aminosäure SEQ ID NO: 10;
(e) die Sequenz der schweren Aminosäure SEQ ID NO: 30 und die Sequenz der leichten Aminosäure SEQ ID NO: 9;
(f) die Sequenz der schweren Aminosäure SEQ ID NO: 32 und die Sequenz der leichten Aminosäure SEQ ID NO: 14;
(g) die Sequenz der schweren Aminosäure SEQ ID NO: 34 und die Sequenz der leichten Aminosäure SEQ ID NO: 6;
(h) die Sequenz der schweren Aminosäure SEQ ID NO: 36 und die Sequenz der leichten Aminosäure SEQ ID NO: 4;
(i) die Sequenz der schweren Aminosäure SEQ ID NO: 38 und die Sequenz der leichten Aminosäure SEQ ID NO: 16;
(j) die Sequenz der schweren Aminosäure SEQ ID NO: 40 und die Sequenz der leichten Aminosäure SEQ ID NO: 18;
(k) die Sequenz der schweren Aminosäure SEQ ID NO: 44 und die Sequenz der leichten Aminosäure SEQ ID NO: 19;
(l) die Sequenz der schweren Aminosäure SEQ ID NO: 48 und die Sequenz der leichten Aminosäure SEQ ID NO: 22;
(m) die Sequenz der schweren Aminosäure SEQ ID NO: 49 und die Sequenz der leichten Aminosäure SEQ ID NO: 21;
(n) die Sequenz der schweren Aminosäure SEQ ID NO: 51 und die Sequenz der leichten Aminosäure SEQ ID NO: 12; und
(o) die Sequenz der schweren Aminosäure SEQ ID NO: 53 und die Sequenz der leichten Aminosäure SEQ ID NO: 5.

2. Antikörper oder antigenbindendes Fragment nach Anspruch 1, wobei
(a) der Antikörper ein monoklonaler Antikörper ist;
(b) der Antikörper ein chimärischer Antikörper ist;
(c) der Antikörper ein menschlicher Antikörper ist, oder
(d) das Antigenbindungsfragment aus Fab', F(ab')₂, Fv und einer Einzelkette ausgewählt wird.

3. Zusammensetzung, die den Antikörper oder das Antigenbindungsfragment nach Anspruch 1 oder 2 umfasst und einen pharmazeutisch akzeptablen Träger.

4. Antikörper oder Antigenbindungsfragment nach Anspruch 1 oder 2, wobei der Antikörper oder das Antigenbindungsfragment an ein Therapeutikum konjugiert ist.

5. Antikörper oder Antigenbindungsfragment nach Anspruch 4, wobei das Therapeutikum ein Toxin oder ein Radioisotop ist.

6. Hybridom-Zelllinie, die den Antikörper nach Anspruch 1 oder 2 erzeugt.

7. Transformierte Zelle, die ein Gen umfasst, das den Antikörper oder das Antigenbindungsfragment nach Anspruch 1 oder 2 codiert.

8. Antikörper oder Antigenbindungsfragment nach Anspruch 1 oder 2 zur Verwendung als Medikament.

9. Antikörper oder Antigenbindungsfragment nach Anspruch 1 oder 2 zur Verwendung bei einem Verfahren zum Behandeln einer Krankheit, die sich auf die Angiogenese in einem Säugetier bezieht.

10. Antikörper oder Antigenbindungsfragment nach Anspruch 9, wobei die Krankheit, die sich auf die Angiogenese bezieht, Krebs ist.

11. Antikörper oder Antigenbindungsfragment nach Anspruch 10, wobei der Krebs ein Lungenkrebs, Darmkrebs, Magenkrebs, Nierenkrebs, Prostatakrebs, Eierstockkrebs, Gebärmutterkrebs oder Augenkrebs ist.

12. Antikörper oder Antigenbindungsfragment nach Anspruch 9, wobei die Krankheit, die sich auf die Angiogenese bezieht, ein nicht-neoplastischer Zustand ist, der aus der Gruppe ausgewählt wird, die aus Folgendem besteht:
Arthritis, rheumatoide Arthritis, Psoriasis, psoriatische Plaques, Sarkoidose, Atherosklerose, atherosklerotische Plaques, Ödem von einem Herzinfarkt, Retinopathie von Frühgeburten, retrolentale Fibroplasie, neovaskuläres Glaukom, Makuladegeneration, diabetisches Makulaödem, Hornhaut-Neovaskularisierung, Neovaskularisierung eines Hornhauttransplantats, Abstoßung eines Hornhauttransplantats, Neovaskularisierung der Netzhaut oder des Chorions, Neovaskularisierung des Kammerwinkels, neovaskuläre Augenkrankheit, vaskuläre Restenose, arteriovenöse Fehlbildung.

13. Antikörper oder Antigenbindungsfragment nach einem der Ansprüche 9 bis 12, wobei das Säugetier ein Mensch ist.

14. Isoliertes Polynukleotidmolekül, umfassend:
(a) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NOS: 54 und 55 umfasst;
(b) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NOS: 56 und 57 umfasst;
(c) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NOS: 59 und 60 umfasst;
(d) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NOS: 62 und 63 umfasst;
(e) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NOS: 64 und 65 umfasst;
(f) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NO: 66 und 67 umfasst;
(g) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NOS: 68 und 69 umfasst;
(h) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NOS: 70 und 71 umfasst;
(i) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NOS: 72 und 73 umfasst;
(j) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NOS: 74 und 75 umfasst;
(k) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NOS: 76 und 77 umfasst;
(l) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NOS: 78 und 79 umfasst;
(m) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NOS: 80 und 81 umfasst;
(n) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NOS: 82 und 83 umfasst; und
(o) eine Nukleotidsequenz, die die Sequenz der schweren Aminosäure und die Sequenz der leichten Aminosäure eines menschlichen Anti-ANGPTL4-Antikörpers codiert, wobei die Nukleotidsequenz die Nukleotidsequenzen von SEQ ID NOS: 86 und 87 umfasst.

15. Testkit für den Nachweis von ANGPTL4 in Säugetiergeweben oder -zellen, um auf Lungen-, Darm-, Magen-, Nieren-, Prostata-, Eierstocks-, Uterus- oder Augenkarzinome zu untersuchen, wobei das ANGPTL4 ein Antigen ist, das durch Lungen-, Darm-, Magen-, Nieren-, Prostata-, Eierstocks-, Uterus- oder Augenkarzinome exprimiert wird, wobei das Kit einen Antikörper oder ein Antigenbindungsfragment nach einem der Ansprüche 1 bis 3 umfasst sowie Mittel zum Anzeigen der Reaktion des Antikörpers oder Antigenbindungsfragments mit dem Antigen, falls vorhanden.

## Revendications

1. L'anticorps isolé, ou le fragment de liaison à l'antigène de celui-ci, qui se lie à ANGPTL4 et qui comprend à la fois une séquence d'acides aminés de chaîne lourde et une séquence d'acides aminés de chaîne légère sélectionné du groupe constitué par :
(a) la séquence d'acides aminés de chaîne lourde de SEQ ID NO : 24 et la séquence d'acides aminés de chaîne légère de SEQ ID NO : 2 ;
(b) la séquence d'acides aminés de chaîne lourde de SEQ ID NO : 26 et la séquence d'acides aminés de chaîne légère de SEQ ID NO : 7 ;
(c) la séquence d'acides aminés de chaîne lourde de SEQ ID NO : 27 et la séquence d'acides aminés de chaîne légère de SEQ ID NO : 3 ;
(d) la séquence d'acides aminés de chaîne lourde de SEQ ID NO : 29 et la séquence d'acides aminés de chaîne légère de SEQ ID NO : 10 ;
(e) la séquence d'acides aminés de chaîne lourde de SEQ ID NO : 30 et la séquence d'acides aminés de chaîne légère de SEQ ID NO : 9 ;
(f) la séquence d'acides aminés de chaîne lourde de SEQ ID NO : 32 et la séquence d'acides aminés de chaîne légère de SEQ ID NO : 14 ;
(g) la séquence d'acides aminés de chaîne lourde de SEQ ID NO : 34 et la séquence d'acides aminés de chaîne légère de SEQ ID NO : 6 ;
(h) la séquence d'acides aminés de chaîne lourde de SEQ ID NO : 36 et la séquence d'acides aminés de chaîne légère de SEQ ID NO : 4 ;
(i) la séquence d'acides aminés de chaîne lourde de SEQ ID NO : 38 et la séquence d'acides aminés de chaîne légère de SEQ ID NO : 16 ;
(j) la séquence d'acides aminés de chaîne lourde de SEQ ID NO : 40 et la séquence d'acides aminés de chaîne légère de SEQ ID NO : 18 ;
(k) la séquence d'acides aminés de chaîne lourde de SEQ ID NO : 44 et la séquence d'acides aminés de chaîne légère de SEQ ID NO : 19 ;
(l) la séquence d'acides aminés de chaîne lourde de SEQ ID NO : 48 et la séquence d'acides aminés de chaîne légère de SEQ ID NO : 22 ;
(m) la séquence d'acides aminés de chaîne lourde de SEQ ID NO : 49 et la séquence d'acides aminés de chaîne légère de SEQ ID NO : 21 ;
(n) la séquence d'acides aminés de chaîne lourde de SEQ ID NO : 51 et la séquence d'acides aminés de chaîne légère de SEQ ID NO : 12 ; et
(o) la séquence d'acides aminés de chaîne lourde de SEQ ID NO : 53 et la séquence d'acides aminés de chaîne légère de SEQ ID NO : 5.

2. L'anticorps ou le fragment de liaison à l'antigène selon la revendication 1, dans lequel
(a) l'anticorps est un anticorps monoclonal ;
(b) l'anticorps est un anticorps chimérique ;
(c) l'anticorps est un anticorps humain, ou
(d) le fragment de liaison à l'antigène est choisi parmi Fab, Fab', F(ab')2, Fv et une chaîne simple.

3. La composition comprend l'anticorps ou le fragment de liaison à l'antigène de la revendication 1 ou 2, et un support pharmaceutiquement acceptable.

4. L'anticorps ou le fragment de liaison à l'antigène selon la revendication 1 ou 2, dans lequel l'anticorps ou le fragment de liaison à l'antigène est conjugué à un agent thérapeutique.

5. L'anticorps ou le fragment de liaison à l'antigène selon la revendication 4, dans lequel l'agent thérapeutique est une toxine ou un radio-isotope.

6. La lignée cellulaire d'hybridome produit l'anticorps selon la revendication 1 ou 2.

7. La cellule transformée comprend un gène codant pour l'anticorps ou le fragment de liaison à l'antigène selon les revendications 1 à 2.

8. L'anticorps ou le fragment de liaison à l'antigène selon les revendications 1 à 2, destiné à être utilisé comme médicament.

9. L'anticorps ou le fragment de liaison à l'antigène selon la revendication 1 ou 2, destiné à être utilisé dans un procédé de traitement d'une maladie liée à l'angiogenèse chez un mammifère.

10. L'anticorps ou le fragment de liaison à l'antigène selon la revendication 9, dans lequel la maladie liée à l'angiogenèse est un cancer.

11. L'anticorps ou le fragment de liaison à l'antigène selon la revendication 10, dans laquelle le cancer est un cancer du poumon, un cancer du côlon, un cancer gastrique, un cancer du rein, un cancer de la prostate, un cancer de l'ovaire, un cancer de l'utérus, ou un carcinome oculaire.

12. L'anticorps ou le fragment de liaison à l'antigène de la revendication 9, dans laquelle la maladie liée à l'angiogenèse est une maladie non néoplasique choisie dans le groupe constitué par :
l'arthrite, l'arthrite rhumatoïde, le psoriasis, les plaques de psoriasis, la sarcoïdose, l'athérosclérose, les plaques d'athérosclérose, l'oedème de l'infarctus du myocarde, de la rétinopathie des prématurés, la fibroplasie rétrolentale, le glaucome néovasculaire, la dégénérescence maculaire, l'oedème maculaire diabétique, la néovascularisation de la cornée, la cornée néovascularisation de greffe, le rejet de greffe de cornée, la neovasculariztion rétinienne ou choroïdienne, neovasculariztion de l'angle, la maladie néovasculaire oculaire, la resténose vasculaire, la malformation artérioveineuse.

13. L'anticorps ou le fragment de liaison à l'antigène selon l'une quelconque des revendications 9 à 12, dans lequel le mammifère est un humain.

14. La molécule polynucléotidique isolée comprend :
(a) une séquence nucléotidique codant pour la séquence d'acides aminés de la chaîne lourde et la séquence d'acides aminés de la chaîne légère d'un anticorps anti-ANGPTL4 humain, dans lequel la séquence nucléotidique comprend les séquences de SEQ ID NO nucléotidiques: 54 et 55 ;
(b) une séquence nucléotidique codant pour la séquence d'acides aminés à chaîne lourde et la séquence d'acides aminés de chaîne légère d'un anticorps anti-ANGPTL4 humain, dans laquelle la séquence nucléotidique comprend les séquences nucléotidiques de SEQ ID NOS: 56 et 57 ;
(c) une séquence nucléotidique codant pour la séquence d'acides aminés à chaîne lourde et la séquence d'acides aminés de chaîne légère d'un anticorps humain anti-ANGPTL4, dans laquelle la séquence nucléotidique comprend les séquences nucléotidiques de SEQ ID NOS: 59 et 60 ;
(d) une séquence nucléotidique codant pour la séquence d'acides aminés à chaîne lourde et la séquence d'acides aminés de chaîne légère d'un anticorps anti-ANGPTL4 humain, dans laquelle la séquence nucléotidique comprend les séquences nucléotidiques de SEQ ID NOS: 62 et 63 ;
(e) une séquence nucléotidique codant pour la séquence d'acides aminés à chaîne lourde et la séquence d'acides aminés de chaîne légère d'un anticorps anti-ANGPTL4 humain, dans laquelle la séquence nucléotidique comprend les séquences nucléotidiques de SEQ ID NOS: 64 et 65 ;
(f) une séquence nucléotidique codant pour la séquence d'acides aminés à chaîne lourde et la séquence d'acides aminés de chaîne légère d'un anticorps humain anti-ANGPTL4, dans laquelle la séquence nucléotidique comprend les séquences nucléotidiques de SEQ ID NOS: 66 et 67 ;
(g) une séquence nucléotidique codant pour la séquence d'acides aminés à chaîne lourde et la séquence d'acides aminés de chaîne légère d'un anticorps anti-ANGPTL4 humain, dans laquelle la séquence nucléotidique comprend les séquences nucléotidiques de SEQ ID NOS: 68 et 69 ;
(h) une séquence nucléotidique codant pour la séquence d'acides aminés à chaîne lourde et la séquence d'acides aminés de chaîne légère d'un anticorps humain anti-ANGPTL4, dans laquelle la séquence nucléotidique comprend les séquences nucléotidiques de SEQ ID NOS: 70 et 71 ;
(i) une séquence nucléotidique codant pour la séquence d'acides aminés à chaîne lourde et la séquence d'acides aminés de chaîne légère d'un anticorps humain anti-ANGPTL4, dans laquelle la séquence nucléotidique comprend les séquences nucléotidiques de SEQ ID NOS: 72 et 73 ;
(j) une séquence nucléotidique codant pour la séquence d'acides aminés à chaîne lourde et la séquence d'acides aminés de chaîne légère d'un anticorps humain anti-ANGPTL4, dans laquelle la séquence nucléotidique comprend les séquences nucléotidiques de SEQ ID NOS: 74 et 75 ;
(k) une séquence nucléotidique codant pour la séquence d'acides aminés à chaîne lourde et la séquence d'acides aminés de chaîne légère d'un anticorps anti-ANGPTL4 humain, dans laquelle la séquence nucléotidique comprend les séquences nucléotidiques de SEQ ID NOS: 76 et 77 ;
(l) une séquence nucléotidique codant pour la séquence d'acides aminés à chaîne lourde et la séquence d'acides aminés de chaîne légère d'un anticorps humain anti-ANGPTL4, dans laquelle la séquence nucléotidique comprend les séquences nucléotidiques de SEQ ID NOS: 78 et 79 ;
(m) une séquence nucléotidique codant pour la séquence d'acides aminés à chaîne lourde et la séquence d'acides aminés de chaîne légère d'un anticorps humain anti-ANGPTL4, dans laquelle la séquence nucléotidique comprend les séquences nucléotidiques de SEQ ID NOS: 80 et 81 ;
(n) une séquence nucléotidique codant pour la séquence d'acides aminés à chaîne lourde et la séquence d'acides aminés de chaîne légère d'un anticorps anti-ANGPTL4 humain, dans laquelle la séquence nucléotidique comprend les séquences nucléotidiques de SEQ ID NOS: 82 et 83 ; et
(o) une séquence nucléotidique codant pour la séquence d'acides aminés à chaîne lourde et la séquence d'acides aminés de chaîne légère d'un anticorps anti-ANGPTL4 humain, dans laquelle la séquence nucléotidique comprend les séquences nucléotidiques de SEQ ID NOS: 86 et 87.

15. Le kit d'analyse pour la détection de l'ANGPTL4 dans des tissus ou cellules de mammifères afin de cribler des cancers du poumon, du côlon, de l'estomac, du rein, de la prostate, des ovaires, de l'utérus ou des oculaires, l'ANGPTL4 étant un antigène exprimé par le poumon, le côlon, rénal, la prostate, ovarien, utérin ou oculaire, le kit comprend un anticorps ou un fragment de liaison à l'antigène selon l'une quelconque des revendications 1 à 3 et des moyens pour indiquer la réaction de l'anticorps ou du fragment de liaison à l'antigène avec l'antigène, s'il existe.
